# EUROPEAN PATENT SPECIFICATION

(11) **EP 4 051 181 B1**
(45) Date of publication and mention of the grant of the patent: **16.07.2025**
(21) Application number: 20812172.3
(22) Date of filing: 30.10.2020
(51) Int. Cl.: A61F 2/24

(54) **REAL TIME MEASUREMENTS OF PHYSIOLOGICAL PARAMETERS ASSOCIATED WITH HEART VALVE REPLACEMENT**
ECHTZEITMESSUNGEN VON PHYSIOLOGISCHEN PARAMETERN IM ZUSAMMENHANG MIT EINEM HERZKLAPPENERSATZ
MESURES EN TEMPS RÉEL DE PARAMÈTRES PHYSIOLOGIQUES ASSOCIÉS À UN REMPLACEMENT DE VALVULE CARDIAQUE

(30) Priority: 31.10.2019 US 201962928925 P
(43) Date of publication of application: 07.09.2022
(73) Proprietor: Edwards Lifesciences Corporation, Irvine, CA 92614-5688 (US)
(72) Inventor: GOLDBERT, Eran, 30889 Caesarea (IL); LEVI, Tamir S., 30889 Caesarea (IL); MAIMON, David, 30889 Caesarea (IL); COHEN, Oren, 30889 Caesarea (IL); THOMAS, Martyn Rhys, Irvine, CA 92614 (US); SAAR, Tomer, 30889 Caesarea (IL); SCHWARCZ, Elazar Levi, 30889 Caesarea (IL)
(74) Representative: Eisenführ Speiser
(86) International application number: PCT/US2020/058106
(87) International publication number: WO 2021/087196

(56) References cited:
- WO-A1-2014/186646
- US-A1- 2013 274 618
- US-A1- 2017 258 585

## Description

### FIELD OF THE INVENTION

The present invention relates to devices for measuring physiological parameters, such as flow, pressure, temperature, electric conductivity and/or visual indication of thrombus formation or deposits accumulations, prior to, during and/or after prosthetic heart valve implantation procedures.

### BACKGROUND OF THE INVENTION

Native heart valves, such as the aortic, pulmonary and mitral valves, function to assure adequate directional flow from, and to, the heart, and between the heart's chambers, to supply blood to the whole cardiovascular system. Various valvular diseases can render the valves ineffective and require replacement with artificial valves. Surgical procedures can be performed to repair or replace a heart valve. Since surgeries are prone to an abundance of clinical complications, alternative less invasive techniques of delivering a prosthetic heart valve over a catheter and implanting it over the native malfunctioning valve have been developed over the years.

Different types of prosthetic heart valves are known to date, including balloon expandable valve, self-expandable valves and mechanically-expandable valves. Different methods of delivery and implantation are also known, and may vary according to the site of implantation and the type of prosthetic valve. One exemplary technique includes utilization of a delivery assembly for delivering a prosthetic valve in a crimped state, from an incision which can be located at the patient's femoral or iliac artery, toward the native malfunctioning valve. Once the prosthetic valve is properly positioned at the desired site of implantation, it can be expanded against the surrounding anatomy, such as an annulus of a native valve, and the delivery assembly can be retrieved thereafter.

Various parameters, such as prosthetic valve size and expansion diameter, orientation, interaction with the surrounding tissue and the like, may influence various physiological parameters such as: flow patterns and pressure gradients across and/or in the vicinity of the prosthetic valve, electrical conductivity within the native tissue contacted by the prosthetic valve, and post-implantation physiological reaction to the presence of the prosthetic valve, such as inflammation and/or thrombus formation. Accordingly, a need exists for improvements in devices, systems and methods for accurately measuring physiological parameters associated with prosthetic valves, prior-to, during and/or after the implantation procedure, to ensure proper prosthetic valve functionality, as well as long-term durability.
WO 2014/186646 relates to a method for implanting a stent that includes contracting a self-expanding/forcibly-expanding stent of a shape-memory material set to a given shape to a reduced implantation size with a delivery system having drive wires. The stent has a selectively adjustable assembly with adjustable elements operatively connected to the drive wires such that, when the adjustable elements are adjusted by the drive wires, a configuration change in at least a portion of the self-expanding stent occurs.

### SUMMARY OF THE INVENTION

The present disclosure is directed toward devices and assemblies equipped with sensors for monitoring physiological parameters prior to, during, and after prosthetic valve implantation procedures. The devices and assemblies are primarily intended to monitor in real-time physiological parameters such as pressure, flow, temperature, electrical conductivity and/or visual indication of thrombus formation or accumulation of deposits in critical regions to the functionality of the prosthetic valve. The sensors, providing real-time measurements, can be used with a delivery assembly, to ensure proper implantation of a prosthetic valve within a designated site of implantation, such as the site of malfunctioning native valve.

According to the invention, there is provided a delivery assembly comprising a prosthetic valve and a delivery apparatus as defined in claim 1. The prosthetic valve is movable between a radially compressed configuration and a radially expanded configuration. The delivery apparatus comprises a handle, a delivery shaft extending distally from the handle, a nosecone shaft extending through the delivery shaft, a nosecone, a first sensor, and a first transmission line. The nosecone shaft comprises a nosecone shaft outer surface, a nosecone shaft guidewire lumen, and a nosecone shaft distal portion. The nosecone is attached to the nosecone shaft distal portion, and comprises a nosecone guidewire lumen and a nosecone outer surface. The first sensor is retained within the nosecone. The first transmission line is coupled to the first sensor, and extends proximally therefrom, toward the handle.

According to some embodiments, the first sensor is a first pressure sensor.

According to some embodiments, the first transmission line is a first optic fiber, and the first pressure sensor is a first optic pressure sensor.

The nosecone further comprises a nosecone lateral port terminating at a nosecone port opening, wherein the first pressure sensor is positioned within the nosecone lateral port in alignment with the nosecone port opening.

According to some embodiments, the nosecone port opening is formed at the nosecone outer surface.

According to some embodiments, the nosecone port opening is formed between the nosecone lateral port and the nosecone guidewire lumen.

According to some embodiments, the first pressure sensor is attached to the nosecone shaft outer surface.

According to some embodiments, the nosecone shaft further comprises a nosecone shaft sensor lumen and a nosecone shaft side opening, wherein the first pressure sensor is positioned in alignment with the nosecone shaft side opening, and wherein the first transmission line extends through the nosecone shaft sensor lumen.

According to some embodiments, the delivery apparatus further comprises a first sensor shaft extending through the delivery shaft, and comprises a first sensor shaft lumen, a first sensor shaft distal portion, and a first sensor shaft side opening at the first sensor shaft distal portion. In such embodiments, the nosecone is attached to the first sensor shaft distal portion, the first pressure sensor is positioned in alignment with the first sensor shaft side opening, and the first transmission line extends through the first sensor shaft lumen.

According to some embodiments, the delivery apparatus further comprises a second pressure sensor positioned proximal to the prosthetic valve, and a second transmission line coupled to the second pressure sensor and extending proximally therefrom, toward the handle.

According to some embodiments, the second transmission line is a second optic fiber, and the second pressure sensor is a second optic pressure sensor.

According to some embodiments, the second pressure sensor is attached to the nosecone shaft outer surface.

According to some embodiments, the nosecone shaft further comprises a first nosecone shaft sensor lumen having a first nosecone shaft side opening, and a second nosecone shaft sensor lumen having a second nosecone shaft side opening, wherein the first pressure sensor is positioned in alignment with the first nosecone shaft side opening, and wherein the second pressure sensor is positioned in alignment with the second nosecone shaft side opening.

According to some embodiments, the nosecone shaft further comprises a nosecone shaft sensor lumen comprising a first nosecone shaft side opening and a second nosecone shaft side opening, wherein the first pressure sensor is positioned in alignment with the first nosecone shaft side opening, wherein the second pressure sensor is positioned in alignment with the second nosecone shaft side opening, and wherein both the first transmission line and the second transmission line extend through the nosecone shaft sensor lumen.

According to some embodiments, the delivery apparatus further comprises a plurality of actuator arm assemblies, extending through the delivery shaft and releasably coupled to the prosthetic valve, wherein the second pressure sensor is attached to at least one actuation assembly.

According to some embodiments, the delivery apparatus further comprises a re-compression mechanism configured to compress a mechanically expandable prosthetic valve. The re-compression mechanism comprises a re-compression shaft and a re-compression member, wherein the second pressure sensor is attached to the recompression shaft. The re-compression shaft extends through the delivery shaft, and comprises a re-compression shaft main lumen. The re-compression member extends through the re-compression shaft main lumen, and comprises a distal loop. The second pressure sensor is attached to the recompression shaft.

According to some embodiments, the second pressure sensor is attached to an outer surface of the re-compression shaft outer surface.

According to some embodiments, the re-compression shaft further comprises a re-compression shaft sensor lumen and a re-compression shaft side opening, wherein the second pressure sensor is positioned in alignment with the re-compression shaft side opening, and wherein the second transmission line extends through the re-compression shaft sensor lumen.

According to some embodiments, the second pressure sensor is attached to the delivery shaft.

According to some embodiments, the second pressure sensor is attached to an outer surface of the delivery shaft.

According to some embodiments, the delivery shaft further comprises a delivery shaft sensor lumen and a delivery shaft side opening, wherein the second pressure sensor is positioned in alignment with the delivery shaft side opening, and wherein the second transmission line extends through the delivery shaft sensor lumen.

According to some embodiments, the delivery apparatus further comprises a sensing catheter comprising a sensing head, wherein the sensing catheter is axially movable relative to the delivery shaft, and wherein the sensing head comprises a second pressure sensor.

According to some embodiments, the handle further comprises an internal control unit connected to the first transmission line, and configured to receive signals from, and/or transmit signals to, the first pressure sensor, via the first transmission line.

According to some embodiments, the handle further comprises a proximal communication component operatively coupled to the internal control unit, and configured to receive signals from, and/or transmit signals to, components and/or devices external to the delivery assembly.

According to some embodiments, the handle further comprises a display operatively coupled to the internal control unit.

According to some embodiments, the display comprises a digital screen.

According to some embodiments, the display comprises LED lights.

According to some embodiments, the handle further comprises an internal control unit connected to the first transmission line and to the second transmission line, and configured to receive signals from, and/or transmit signals to, the first pressure sensor via the first transmission line, and the second pressure sensor via the second transmission line.

According to some embodiments, the handle further comprises a proximal communication component operatively coupled to the internal control unit, and configured to receive signals from, and/or transmit signals to, components and/or devices external to the delivery assembly.

According to some embodiments, the handle further comprises a display operatively coupled to the internal control unit.

According to some embodiments, there is provided a system comprising the delivery assembly and a sensing catheter comprising a sensing head, wherein the sensing head comprises the second pressure sensor.

According to some embodiments, the sensing catheter is a pigtail catheter.

According to some embodiments, there is provided a method of acquiring a transvalvular pressure measurement, comprising steps of: (a) providing the delivery assembly; (b) advancing the nosecone over a guidewire to a position distal to a native heart valve; (c) expanding the prosthetic valve against the native heart valve; and (d) simultaneously acquiring measurement signals from the first pressure sensor and the second pressure sensor.

According to some embodiments, there is provided a method of acquiring a transvalvular pressure measurement, comprising steps of: (a) providing the delivery assembly; (b) advancing the nosecone over a guidewire to a position distal to a native heart valve; (c) expanding the prosthetic valve against the native heart valve; (d) positioning the sensing head proximal to the prosthetic valve; and (e) simultaneously acquiring measurement signals from the first pressure sensor and the second pressure sensor.

According to some embodiments, there is provided a method of acquiring a transvalvular pressure measurement, comprising steps of: (a) providing the system; (b) advancing the nosecone over a guidewire to a position distal to a native heart valve; (c) expanding the prosthetic valve against the native heart valve; (d) positioning the sensing head proximal to the prosthetic valve; and (e) simultaneously acquiring measurement signals from the first pressure sensor and the second pressure sensor.

According to some embodiments, the methods of acquiring a transvalvular pressure measurement further comprise a step of retracting the guidewire prior to the step of simultaneously acquiring measurement signals.

According to some embodiments, the prosthetic valve is a non-balloon expandable prosthetic valve.

According to some embodiments, the first sensor is a Doppler sensor.

According to some embodiments, there is provided a method of acquiring a transvalvular pressure measurement, comprising steps of: (a) providing the delivery assembly; (b) advancing the nosecone over a guidewire to a position distal to a native heart valve; (c) expanding the prosthetic valve against the native heart valve; and (d) utilizing the Doppler sensor to acquire measurement signals from at least two diametrically opposing regions.

According to some embodiments, the method further comprises steps of: (e) orienting the Doppler sensor at one direction, toward a first region; (f) utilizing the Doppler sensor to acquire measurement signals from the first region; (g) rotating the nosecone so as to orient the Doppler sensor at a diametrically opposite direction, toward a second region; and (h) utilizing the Doppler sensor to acquire measurement signals from the second region.

According to some embodiments, the first sensor is an ultrasonic distance sensor.

According to some embodiments, there is provided a method of acquiring a transvalvular pressure measurement, comprising steps of: (a) providing the delivery assembly; (b) advancing the nosecone over a guidewire to a position distal to a native heart valve; (c) expanding the prosthetic valve against the native heart valve; (d) orienting the ultrasonic distance sensor toward the heart chamber wall; and (e) utilizing the ultrasonic distance sensor to measure distance to the heart chamber wall.

According to some embodiments, the method further comprises a step of utilizing the ultrasonic distance sensor to measure distance to a sidewall of the prosthetic valve.

According to another aspect of the invention, there is provided a method of acquiring flow measurements from at least two diametrically opposing regions, comprising the steps of: (a) providing the delivery assembly comprising a prosthetic valve movable between a radially compressed configuration and a radially expanded configuration, and a delivery apparatus comprising: a handle, a delivery shaft extending distally from the handle, and an ultrasonic measurement catheter extending through the delivery catheter, wherein the delivery catheter comprises a sensing head, and wherein the sensing head comprises a Doppler sensor; (b) expanding the prosthetic valve against a native heart valve; (c) advancing the ultrasonic measurement catheter distally through the expanded prosthetic valve; and (d) utilizing the Doppler sensor to acquire measurement signals from at least two diametrically opposing regions.

According to some embodiments, the step of utilizing the Doppler sensor to acquire measurement signals comprises steps of: (i) orienting the Doppler sensor at one direction, toward a first region; (ii) utilizing the Doppler sensor to acquire measurement signals from the first region; (iii) rotating the nosecone so as to orient the Doppler sensor at a diametrically opposite direction, toward a second region; and (iv) utilizing the Doppler sensor to acquire measurement signals from the second region.

According to another aspect of the invention, there is provided a method of acquiring flow measurements from at least two diametrically opposing regions, comprising the steps of: (a) providing the delivery assembly comprising a prosthetic valve movable between a radially compressed configuration and a radially expanded configuration, and a delivery apparatus comprising: a handle, a delivery shaft extending distally from the handle, and an ultrasonic measurement catheter extending through the delivery catheter, wherein the delivery catheter comprises a sensing head, and wherein the sensing head comprises an ultrasonic distance sensor; (b) expanding the prosthetic valve against a native heart valve; (c) advancing the ultrasonic measurement catheter distally through the expanded prosthetic valve; (d) orienting the ultrasonic distance sensor toward the heart chamber wall; and (e) utilizing the ultrasonic distance sensor to measure distance to the heart chamber wall.

According to some embodiments, the method further comprises a step of utilizing the ultrasonic distance sensor to measure distance to a sidewall of the prosthetic valve.

According to another aspect of the invention, there is provided a method of measuring flow in a region adjacent a prosthetic valve, comprising the steps of: (a) providing a prosthetic valve movable between a radially compressed configuration and a radially expanded configuration, and a delivery apparatus comprising a handle; (b) providing an ultrasonic measurement catheter comprising a sensing head, the sensing head comprising a Doppler sensor; (c) expanding the prosthetic valve against a first native valve, such that at least a portion of the prosthetic valve extends into a heart chamber; (d) extending the ultrasonic measurement catheter through a second native valve, such that the sensing head is positioned within the heart chamber; (e) orienting the Doppler sensor toward the prosthetic valve; and (f) utilizing the Doppler sensor to acquire measurement signals from at least one region adjacent a prosthetic valve.

According to some embodiments, the step of utilizing the Doppler sensor to acquire measurement signals from at least one region comprises utilizing the Doppler sensor to acquire measurement signals from at least two diametrically opposite regions adjacent the prosthetic valve.

According to another aspect of the invention, there is provided a delivery assembly comprising a prosthetic valve and a delivery apparatus. The prosthetic valve is movable between a radially compressed configuration and a radially expanded configuration. The delivery apparatus comprises a handle, a delivery shaft extending distally from the handle, a nosecone shaft extending through the delivery shaft, a nosecone attached to the nosecone shaft, a valved shaft extending through the delivery shaft, a first pressure sensor and a first transmission line. The valved shaft comprises a valved shaft lumen, a valved shaft proximal portion extending into the handle, a valved shaft distal portion, and a shaft valve coupled to the valved shaft proximal portion. The shaft valve is movable between an opened position and a closed position. The first pressure sensor is attached to the valved shaft distal portion, and is disposed within the valved shaft lumen. The first transmission line coupled to the first pressure sensor, and extends proximally therefrom, toward the handle. The shaft valve is configured to prevent flow through the valved shaft lumen in the closed position, and to allow flow there-through in the opened position. The valved shaft is axially movable relative to the delivery shaft.

According to some embodiments, the shaft valve comprises a leaf valve attached to the valved shaft proximal portion via a hinge, wherein the leaf valve is pivotable about the hinge.

According to some embodiments, the shaft valve comprises a stopcock valve.

According to some embodiments, the first transmission line is a first optic fiber, and wherein the first pressure sensor is a first optic pressure sensor.

According to some embodiments, the delivery apparatus further comprises a second pressure sensor positioned proximal to the prosthetic valve, and a second transmission line coupled to the second pressure sensor, and extending proximally therefrom, toward the handle.

According to some embodiments, the second transmission line is a second optic fiber, and wherein the second pressure sensor is a second optic pressure sensor.

According to some embodiments, the second pressure sensor is attached to the nosecone shaft.

According to some embodiments, the delivery apparatus further comprises a plurality of actuator arm assemblies, extending through the delivery shaft and releasably coupled to the prosthetic valve, wherein the second pressure sensor is attached to at least one actuation assembly.

According to some embodiments, the delivery apparatus further comprises a re-compression mechanism configured to compress a mechanically expandable prosthetic valve. The re-compression mechanism comprises a re-compression shaft and a re-compression member. The re-compression shaft extends through the delivery shaft, and comprises a re-compression shaft main lumen. The re-compression member extends through the re-compression shaft main lumen, and comprises a distal loop. The second pressure sensor is attached to the recompression shaft.

According to some embodiments, the second pressure sensor is attached to an outer surface of the re-compression shaft outer surface.

According to some embodiments, the re-compression shaft further comprises a re-compression shaft sensor lumen and a re-compression shaft side opening, wherein the second pressure sensor is positioned in alignment with the re-compression shaft side opening, and wherein the second transmission line extends through the re-compression shaft sensor lumen.

According to some embodiments, the second pressure sensor is attached to the delivery shaft.

According to some embodiments, the second pressure sensor is attached to an outer surface of the delivery shaft.

According to some embodiments, the delivery shaft further comprises a delivery shaft sensor lumen and a delivery shaft side opening, wherein the second pressure sensor is positioned in alignment with the delivery shaft side opening, and wherein the second transmission line extends through the delivery shaft sensor lumen.

According to some embodiments, the delivery apparatus further comprises a second pressure sensor attached to the valved shaft and disposed within the valved shaft lumen, at a position proximal to the first pressure sensor, and a second transmission line coupled to the second pressure sensor, and extending proximally therefrom toward the handle.

According to some embodiments, the handle further comprises an internal control unit connected to the first transmission line and the second transmission line, and configured to receive signals from, and/or transmit signals to, the first pressure sensor via the first transmission line, and the second pressure sensor via the second transmission line.

According to some embodiments, the handle further comprises a proximal communication component operatively coupled to the internal control unit, and configured to receive signals from, and/or transmit signals to, components and/or devices external to the delivery assembly.

According to some embodiments, the handle further comprises a display operatively coupled to the internal control unit.

According to some embodiments, the display comprises a digital screen.

According to some embodiments, the display comprises LED lights.

According to some embodiments, there is provided a method of acquiring a transvalvular pressure measurement, comprising the steps of: (a) providing the delivery assembly; (b) expanding the prosthetic valve against the native heart valve; (c) advancing the valved shaft through the expanded prosthetic valve, so as to position the first pressure sensor distal to the prosthetic valve; (d) moving the shaft valve to the opened position; and (e) simultaneously acquiring measurement signals from the first sensor and the second sensor.

According to some embodiments, there is provided a method of acquiring a transvalvular pressure measurement, comprising the steps of: (a) providing the delivery assembly; (b) expanding the prosthetic valve against the native heart valve; (c) advancing the valved shaft through the expanded prosthetic valve, so as to position the first pressure sensor distal to the prosthetic valve; (d) moving the shaft valve to the opened position; and (e) simultaneously acquiring measurement signals from the first sensor and the second sensor.

According to some embodiments, there is provided a method of acquiring a transvalvular pressure measurement, comprising the steps of: (a) providing the delivery assembly; (b) expanding the prosthetic valve against the native heart valve; (c) advancing the valved shaft through the expanded prosthetic valve, so as to position the first pressure sensor distal to the prosthetic valve, and the second pressure sensor proximal to the prosthetic valve; (d) moving the shaft valve to the opened position; and (e) simultaneously acquiring measurement signals from the first sensor and the second sensor.

According to some embodiments, the prosthetic valve is a non-balloon expandable prosthetic valve.

According to another aspect of the invention, there is provided a delivery assembly comprising a prosthetic valve and a delivery apparatus. The prosthetic valve is movable between a radially compressed configuration and a radially expanded configuration. The delivery apparatus comprises a handle, a delivery shaft extending distally from the handle, a nosecone shaft extending through the delivery shaft, a nosecone attached to the nosecone shaft, a valved guidewire extending through the nosecone shaft and the nosecone, a first pressure sensor, a second pressure, a first transmission line and a second transmission.

The valved guidewire comprises a guidewire internal lumen, a valved guidewire inner surface, a valved guidewire proximal portion extending into the handle, and a guidewire valve coupled to the valved guidewire proximal portion. The guidewire valve is movable between an opened position and a closed position. The first pressure sensor is attached to the valved guidewire inner surface, at a position distal to the prosthetic valve. The second pressure sensor attached to the valved guidewire inner surface, at a position proximal to the prosthetic valve. The first transmission line is coupled to the first pressure sensor, and extends proximally therefrom toward the handle. The second transmission line is coupled to the second pressure sensor, and extends proximally therefrom toward the handle. The guidewire valve is configured to prevent flow through the guidewire internal lumen in the closed position, and to allow flow there-through in the opened position.

According to some embodiments, the guidewire valve comprises a leaf valve attached to the valved guidewire proximal portion via a hinge, wherein the guidewire valve is pivotable about the hinge.

According to some embodiments, the guidewire valve comprises a stopcock valve..

According to some embodiments, the first transmission line is a first optic fiber, wherein the first pressure sensor is a first optic pressure sensor, wherein the second transmission line is a second optic fiber, and wherein the second pressure sensor is a second optic pressure sensor.

According to some embodiments, the handle further comprises an internal control unit connected to the first transmission line and the second transmission line, and configured to receive signals from, and/or transmit signals to, the first pressure sensor via the first transmission line, and the second pressure sensor via the second transmission line.

According to some embodiments, the handle further comprises a proximal communication component operatively coupled to the internal control unit, and configured to receive signals from, and/or transmit signals to, components and/or devices external to the delivery assembly.

According to some embodiments, the handle further comprises a display operatively coupled to the internal control unit.

According to some embodiments, the display comprises a digital screen.

According to some embodiments, the display comprises LED lights.

According to another aspect of the invention, there is provided a delivery assembly comprising a prosthetic valve, at least one sensor housing coupled to the prosthetic valve, at least one sensor retained within the sensor housing, and a delivery apparatus. The prosthetic valve is movable between a radially compressed configuration and a radially expanded configuration. The prosthetic valve comprises an inflow end portion, an outflow end portion, a frame, and a plurality of leaflets coupled to the frame via a plurality of commissures. The delivery apparatus comprises a handle, a delivery shaft extending distally from the handle, at least one transmission line shaft, extending through the delivery shaft, and at least one transmission line, extending through the at least one transmission line shaft.

The at least one transmission line shaft is releasably coupled to the at least one sensor housing. The at least one transmission line is releasably coupled to the at least one sensor. The transmission line shaft is configured to seal the at least one transmission line and the at least one sensor, when the transmission line shaft is coupled to the sensor housing. The at least one transmission line is axially movable relative to the at least one transmission line shaft, when the at least one transmission line is released from the at least one sensor.

According to some embodiments, the at least one transmission line is released from at least one sensor, upon application of a pull force on the at least one transmission line, wherein the magnitude of the pull force is beyond a predetermined threshold magnitude.

According to some embodiments, the sensor housing comprises a housing threaded bore, and the transmission shaft comprises external threading, configured to engage with the housing threaded bore.

According to some embodiments, the at least one sensor is a pressure sensor.

According to some embodiments, the at least one sensor is a flow sensor.

According to some embodiments, the at least one sensor is a temperature sensor.

According to some embodiments, the at least one sensor is a fiber optic sensor, configured to obtain light data.

According to some embodiments, the at least one sensor is an impedance sensor, configured to obtain electric conductivity data.

According to some embodiments, the at least one sensor is oriented radially outward from the prosthetic valve.

According to some embodiments, the at least one sensor housing comprises a first sensor housing and a second sensor housing, wherein the at least one sensor comprises a first sensor retained within the first sensor housing, and a second sensor retained within a second sensor housing, wherein the at least one transmission line shaft comprises a first transmission line shaft, releasably coupled to the first sensor housing, and a second transmission line shaft, releasably coupled to the second sensor housing, and wherein the at least one transmission line comprises a first transmission line, extending through the first transmission line shaft and releasably coupled to the first sensor, and a second transmission line, extending through the second transmission line shaft and releasably coupled to the second sensor.

According to some embodiments, the first sensor housing is coupled to the inflow end portion, and wherein the second sensor housing is coupled to the outflow end portion.

According to some embodiments, the first sensor housing and the second sensor housing are attached to the outflow end portion.

According to some embodiments, the first sensor housing and the second sensor housing are attached to the outflow end portion at diametrically opposite positions.

According to some embodiments, the first sensor housing and the second sensor housing are axially distanced from each other, and are longitudinally aligned along the same circumferential position of the prosthetic valve.

According to some embodiments, the at least one sensor housing comprises a plurality of sensor housings and the at least one sensor comprises a plurality of sensors, wherein the plurality of sensors housings and the plurality of sensors match the number of the plurality of leaflets, and wherein each of the plurality of sensors housings is positioned between the inflow end portion and the outflow end portion, such that each of the plurality of sensors is oriented radially inward, facing a corresponding leaflet of the plurality of leaflets.

According to some embodiments, the at least one sensor housing is attached to a commissure.

According to another aspect of the invention, there is provided a prosthetic valve comprising an inflow end portion, an outflow end portion, a plurality of leaflets, and at least two sensors coupled to the outflow end portion, wherein the prosthetic valve is movable between a radially compressed configuration and a radially expanded configuration.

According to some embodiments, the plurality of sensor are pressure sensors.

According to some embodiments, the plurality of sensor are flow sensors.

According to some embodiments, the plurality of sensors are temperature sensors.

According to some embodiments, the plurality of sensors are circumferentially distanced from each other.

According to some embodiments, at least two of the plurality of sensors are attached to the outflow end portion at diametrically opposite positions.

According to some embodiments, at least two of the plurality of sensors are axially distanced from each other, and are longitudinally aligned along the same circumferential position of the prosthetic valve.

According to another aspect of the invention, there is provided a method of identifying leaflet thrombosis within a pre-mounted prosthetic valve, comprising the steps of: (a) providing an ultrasound echocardiography catheter comprising a sensing head, the sensing head comprising an ultrasound echocardiography sensor; (b) advancing the ultrasound echocardiography catheter toward a lumen of a pre-mounted prosthetic valve; (c) directing the ultrasound echocardiography sensor toward at least one leaflet of the prosthetic valve; and (d) utilizing the ultrasound echocardiography sensor to acquire an image of a space confined between the at least one leaflet and a frame of the prosthetic valve.

According to some embodiments, the method further comprises steps of: (e) directing the ultrasound echocardiography sensor toward at least one other leaflet of the prosthetic valve; and (f) utilizing the ultrasound echocardiography sensor to acquire an image of a space confined between the at least one other leaflet and the frame.

According to another aspect of the invention, there is provided a method of identifying leaflet thrombosis within a pre-mounted prosthetic valve, comprising the steps of: (a) providing an acoustic viscosity catheter comprising a sensing head, the sensing head comprising an acoustic viscosity sensor; (b) advancing the acoustic viscosity catheter toward a lumen of a pre-mounted prosthetic valve; (c) directing the acoustic viscosity sensor toward at least one leaflet of the prosthetic valve; and (d) utilizing the acoustic viscosity sensor to measure blood viscosity in a space confined between the at least one leaflet and a frame of the prosthetic valve.

According to some embodiments, the method further comprises steps of: (e) directing the acoustic viscosity sensor toward at least one other leaflet of the prosthetic valve; and (f) utilizing the acoustic viscosity sensor to measure blood viscosity in a space confined between the at least one other leaflet and the frame.

Certain embodiments of the present invention may include some, all, or none of the above advantages. Further advantages may be readily apparent to those skilled in the art from the figures, descriptions, and claims included herein. Aspects and embodiments of the invention are further described in the specification herein below and in the appended claims.

Unless otherwise defined, all technical and scientific terms used herein have the same meaning as commonly understood by one of ordinary skill in the art to which this invention pertains. In case of conflict, the patent specification, including definitions, governs. As used herein, the indefinite articles "a" and "an" mean "at least one" or "one or more" unless the context clearly dictates otherwise.

The following embodiments and aspects thereof are described and illustrated in conjunction with systems, tools and methods which are meant to be exemplary and illustrative, but not limiting in scope. In various embodiments, one or more of the above-described problems have been reduced or eliminated, while other embodiments are directed to other advantages or improvements.

### BRIEF DESCRIPTION OF THE FIGURES

Some embodiments of the invention are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the invention. For the sake of clarity, some objects depicted in the figures are not to scale.

In the Figures:
Fig. 1 constitutes a sectional view of the human heart.
Fig. 2 constitutes a view in perspective of a delivery assembly comprising a delivery apparatus carrying a prosthetic valve, according to some embodiments.
Fig. 3A constitutes a view in perspective of a prosthetic valve, according to some embodiments.
Fig. 3B constitutes a view in perspective of a prosthetic mechanical valve, according to some embodiments.
Fig. 4A constitutes a view in perspective of a nosecone, according to some embodiments.
Fig. 4B constitutes a cross-sectional side view of the nosecone shown in Fig. 4A.
Figs. 5A-5C show different stages of prosthetic valve deployment, according to some embodiments.
Fig. 6 constitutes a view in perspective of a nosecone provided with a side opening, according to some embodiments.
Figs. 7A-7G show different exemplary embodiments of a sensor embedded within a nosecone.
Fig. 8 constitutes an enlarged view in perspective of the distal portion of a delivery assembly, provided with a sensor positioned proximal to the prosthetic valve, according to some embodiments.
Figs. 9A-9H show different exemplary embodiments of a sensor attached to a component of a delivery apparatus, proximal to the prosthetic valve.
Fig. 10A constitutes a side view of an optic fiber having an optic pressure sensor retained within a nosecone, according to some embodiments.
Fig. 10B shows a zoomed-in view of the region 10B marked in Fig. 10A.
Fig. 11 constitutes an enlarged view in perspective of the distal portion of a delivery assembly, provided with a sensing catheter, according to some embodiments
Fig. 12 shows an exemplary embodiment of a delivery assembly equipped with a sensor retained within the nosecone, during prosthetic valve deployment within a native aortic annulus.
Fig. 13 constitutes an enlarged view in perspective of the distal portion of a system comprising a delivery assembly and a sensing catheter, according to some embodiments.
Fig. 14 shows an exemplary embodiment of a system comprising a delivery assembly and a sensing catheter, during prosthetic valve deployment within a native aortic annulus.
Figs. 15A-15B show a delivery assembly equipped with a valved shaft, in closed and open states, according to some embodiments.
Figs. 16A-16B show sectional views of the delivery assembly shown in Figs. 15A-15B, respectively.
Fig. 17A-17B show a delivery assembly equipped with a valved shaft having a stopcock, in closed and open states, according to some embodiments
Fig. 18A-18B show sectional views of the delivery assembly shown in Figs. 17A-17B, respectively.
Figs. 19A-19B show an exemplary embodiment of a delivery assembly equipped with a valved shaft, in different stages of prosthetic valve deployment within a native aortic annulus.
Fig. 20A-20B show a delivery assembly equipped with a valved guidewire, in closed and open states, according to some embodiments.
Fig. 21A-21B show sectional views of the delivery assembly shown in Figs. 20A-20B, respectively.
Fig. 22A constitutes a view in perspective of sensors attached to a mechanical prosthetic valve, according to some embodiments.
Fig. 22B constitutes a view in perspective of sensors attached to a prosthetic valve, according to some embodiments
Figs. 23A-23C show different operational states of a detachable coupling mechanism between transmission lines and sensors, according to some embodiments.
Fig. 24 shows an exemplary prosthetic valve implanted within a mitral annulus, in an orientation that urges a native mitral leaflet toward the left ventricular outflow tract.
Figs. 25A-25B show an exemplary valve implanted within the mitral annulus in a favorable valve orientation, at different stages of diastole.
Figs. 26A-26B show an exemplary valve implanted within the mitral annulus in an unfavorable valve orientation, at different stages of diastole.
Fig. 27 shows an exemplary embodiment of a delivery assembly equipped with a nosecone-embedded sensor, during prosthetic valve deployment within a native mitral annulus.
Fig. 28 constitutes an enlarged view in perspective of the distal portion of the delivery assembly provided with a Doppler catheter, according to some embodiments.
Fig. 29 shows an exemplary embodiment of a delivery assembly equipped with a Doppler catheter, during prosthetic valve deployment within a native mitral annulus.
Fig. 30 shows an exemplary embodiment of a transcatheter Doppler regulated system, during prosthetic valve deployment within a native mitral annulus.
Figs. 31A-31E show different exemplary embodiments of sensors attached to a prosthetic valve deployed within a native mitral annulus.
Fig. 32 shows an exemplary embodiment of a Doppler catheter extending toward a prosthetic valve implanted within a native annulus.
Figs. 33-36 show different exemplary embodiments of sensors attached to a prosthetic valve.

### DETAILED DESCRIPTION OF SOME EMBODIMENTS

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure. In order to avoid undue clutter from having too many reference numbers and lead lines on a particular drawing, some components will be introduced via one or more drawings and not explicitly identified in every subsequent drawing that contains that component.

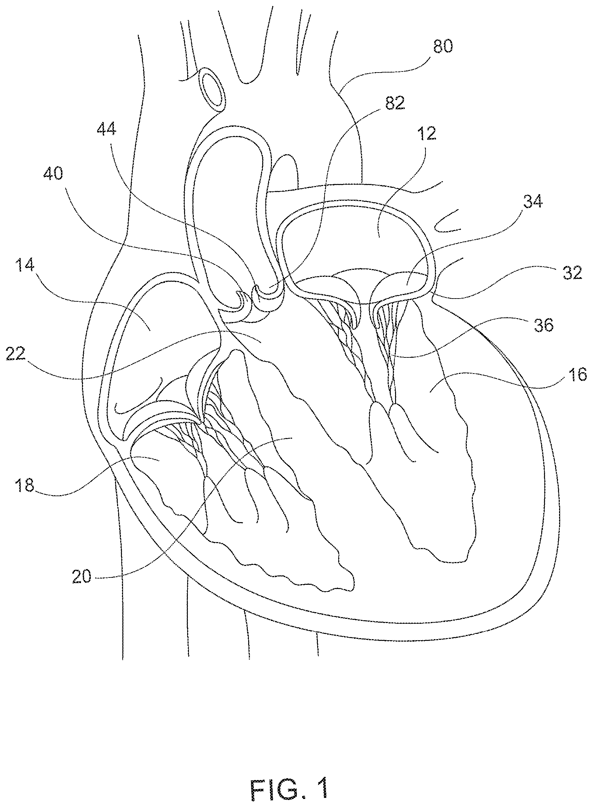

Fig. 1 constitutes a sectional view of a healthy human heart. The heart has a four-chambered conical structure that includes the left atrium 12, the right atrium 14, the left ventricle 16 and the right ventricle 18. The wall separating between the left and right sides of the heart is referred to as the septum 20. The native mitral valve 30 is positioned between the left atrium 12 and the left ventricle 16. The native aortic valve 40 is positioned between the left ventricle 16 and the aorta 80. The initial portion of the aorta 80 extending from the native aortic valve 40 is the aortic root 82, and the adjoining part of the left ventricle 16 is the left ventricular outflow tract (LVOT) 22.

The native mitral valve 30 comprises a mitral annulus 32 and a pair of mitral leaflets 34 extending downward from the annulus 32. When operating properly, the leaflets 34 function together to allow blood flow only from the left atrium 12 to the left ventricle 14. Specifically, during diastole, when the muscles of the left atrium 12 and the left ventricle 16 dilate, oxygenated blood flows from the left atrium 12, through the mitral valve 30, into the left ventricle 16. During systole, when the muscles of the left atrium 12 relax and the left ventricle 16 contacts, the blood pressure within the left ventricle 16 increases so as to urge to two mitral leaflets 34 to coapt, thereby preventing blood flow from the left ventricle 16 back to the left atrium 12. A plurality of fiber cords, referred to as the chordae tendinae 36, tether the mitral leaflets 34 to papillary muscles of the left ventricle 16 to prevent them from prolapsing under pressure and folding back through the mitral annulus 32.

The term "plurality", as used herein, means more than one.

The native aortic valve 40 comprises an aortic annulus 42 and three aortic leaflets 44 extending upward (toward the aortic root 82) from the annulus 42. During systole, blood is expelled from the left ventricle 16, through the aortic valve 40, into the aorta 80. When either the native mitral valve 30 or native aortic valve 40 fails to function properly, a prosthetic replacement valve 140 can help restore functionality.

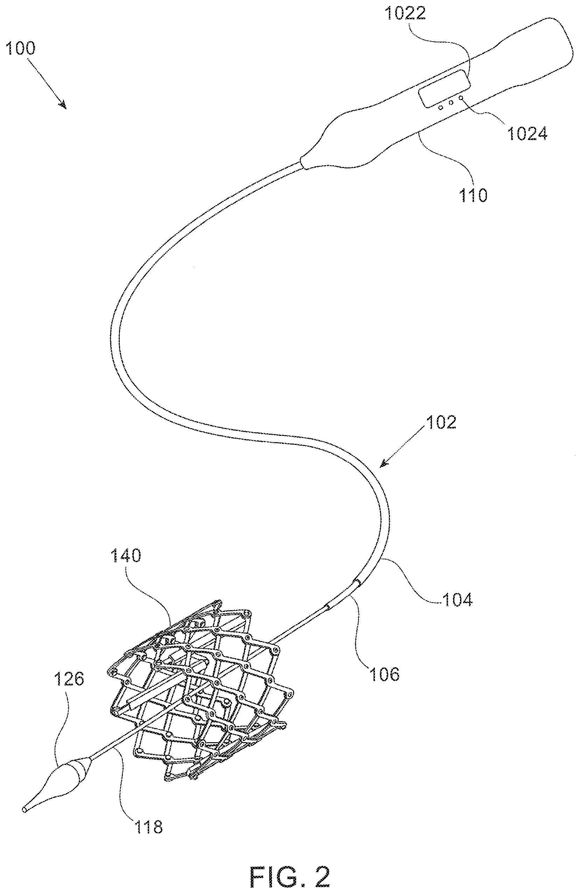

Fig. 2 constitutes a view in perspective of a delivery assembly 100, according to some embodiments. The delivery assembly 100 can include a prosthetic valve 140 and a delivery apparatus 102. The prosthetic valve 140 can be on or releasably coupled to the delivery apparatus 102. The delivery apparatus can include a handle 110 at a proximal end thereof, a nosecone shaft (also termed herein an NC shaft) 118 extending distally from the handle 110, a nosecone (NC) 126 attached to the nosecone shaft distal portion (also termed herein NC shaft distal portion) 120, a delivery shaft 106 extending over the NC shaft 118, and optionally an outer shaft 104 extending over the delivery shaft 106.

The term "proximal", as used herein, generally refers to the side or end of any device or a component of a device, which is closer to the handle 110 or an operator of the handle 110 when in use.

The term "distal", as used herein, generally refers to the side or end of any device or a component of a device, which is farther from the handle 110 or an operator of the handle 110 when in use.

The term "prosthetic valve", as used herein, refers to any type of a prosthetic valve deliverable to a patient's target site over a catheter, which is radially expandable and compressible between a radially compressed, or crimped, state, and a radially expanded state. Thus, a prosthetic valve 140 can be crimped or retained by a delivery apparatus 102 in a compressed state during delivery, and then expanded to the expanded state once the prosthetic valve 140 reaches the implantation site. The expanded state may include a range of diameters to which the valve may expand, between the compressed state and a maximal diameter reached at a fully expanded state. Thus, a plurality of partially expanded states may relate to any expansion diameter between radially compressed or crimped state, and maximally expanded state.

A prosthetic valve 140 of the current disclosure may include any prosthetic valve configured to be mounted within the native aortic valve, the native mitral valve, the native pulmonary valve, and the native tricuspid valve. While a delivery assembly 100 described in the current disclosure, includes a delivery apparatus 102 and a prosthetic valve 140, it should be understood that the delivery apparatus 102 according to any embodiment of the current disclosure can be used for implantation of other prosthetic devices aside from prosthetic valves, such as stents or grafts.

The prosthetic valve 140 can be delivered to the site of implantation via a delivery assembly 100 carrying the valve 140 in a radially compressed or crimped state, toward the target site, to be mounted against the native anatomy, by expanding the valve 140 via various expansion mechanisms. Balloon expandable valves generally involve a procedure of inflating a balloon within a prosthetic valve, thereby expanding the prosthetic valve 140 within the desired implantation site. Once the valve is sufficiently expanded, the balloon is deflated and retrieved along with the delivery apparatus 102. Self-expandable valves include a frame that is shape-set to automatically expand as soon an outer retaining capsule, which may be also defined as the distal portion of an outer shaft 104 or the distal portion of a delivery shaft 106, is withdrawn proximally relative to the prosthetic valve. Mechanically expandable valves are a category of prosthetic valves that rely on a mechanical actuation mechanism for expansion. The mechanical actuation mechanism usually includes a plurality of actuator assemblies, releasably coupled to respective actuation arm assemblies of the delivery apparatus 102, controlled via the handle 110 for actuating the actuator assemblies to expand the prosthetic valve to a desired diameter. The actuator assemblies may optionally lock the valve's position to prevent undesired recompression thereof, and disconnection of the actuation arm assemblies from the actuator assemblies, to enable retrieval of the delivery apparatus 102 once the prosthetic valve is properly positioned at the desired site of implantation.

The delivery assembly 100 can be utilized, for example, to deliver a prosthetic aortic valve for mounting against the aortic annulus 42, to deliver a prosthetic mitral valve for mounting against the mitral annulus 32, or to deliver a prosthetic valve for mounting against any other native annulus.

The outer shaft 104 and the delivery shaft 106 can be configured to be axially movable relative to each other, such that a proximally oriented movement of the outer shaft 104 relative to the delivery shaft 106, or a distally oriented movement of the delivery shaft 106 relative to the outer shaft 104, can expose the prosthetic valve 140 from the outer shaft 104. In alternative embodiments, the prosthetic valve 140 is not housed within the outer shaft 104 during delivery. Thus, according to some embodiments, the delivery apparatus 102 does not include an outer shaft 104.

As mentioned above, the proximal ends of the NC shaft 118, the delivery shaft 106, components of the actuation arm assemblies (in case of mechanically expandable vales), and when present - the outer shaft 104, can be coupled to the handle 110. During delivery of the prosthetic valve 140, the handle 110 can be maneuvered by an operator (e.g., a clinician or a surgeon) to axially advance or retract components of the delivery apparatus 102, such as the nosecone shaft 118, the delivery shaft 106, and/or the outer shaft 104, through the patient's vasculature, as well as to expand or contract a mechanically expandable valve 140', for example by maneuvering the actuation arm assemblies, and to disconnect the prosthetic valve 140 from the delivery apparatus 102, for example - by decoupling the actuation arm assemblies from the actuator assemblies of mechanically expandable valve, in order to retract the delivery apparatus 102 once the prosthetic valve is mounted in the implantation site.

The term "and/or" is inclusive here, meaning "and" as well as "or". For example, "delivery shaft 106 and/or outer shaft 104" encompasses, delivery shaft 106, outer shaft 104, and delivery shaft 106 with outer shaft 104; and, such "delivery shaft 106 and/or outer shaft 104" may include other elements as well.

According to some embodiments, the handle 110 can include one or more operating interfaces, such as steerable or rotatable adjustment knobs, levers, sliders, buttons (not shown) and other actuating mechanisms, which are operatively connected to different components of the delivery apparatus 102 and configured to produce axial movement of the delivery apparatus 102 in the proximal and distal directions, as well as to expand or contract the prosthetic valve 140 via various adjustment and activation mechanisms.

According to some embodiments, the handle further comprises one or more visual or auditory informative elements configured to provide visual or auditory information and/or feedback to a user or operator of the delivery apparatus 102, such as a digital screen 1022 (e.g., an LCD screen), LED lights 1024, speakers (not shown) and the like.

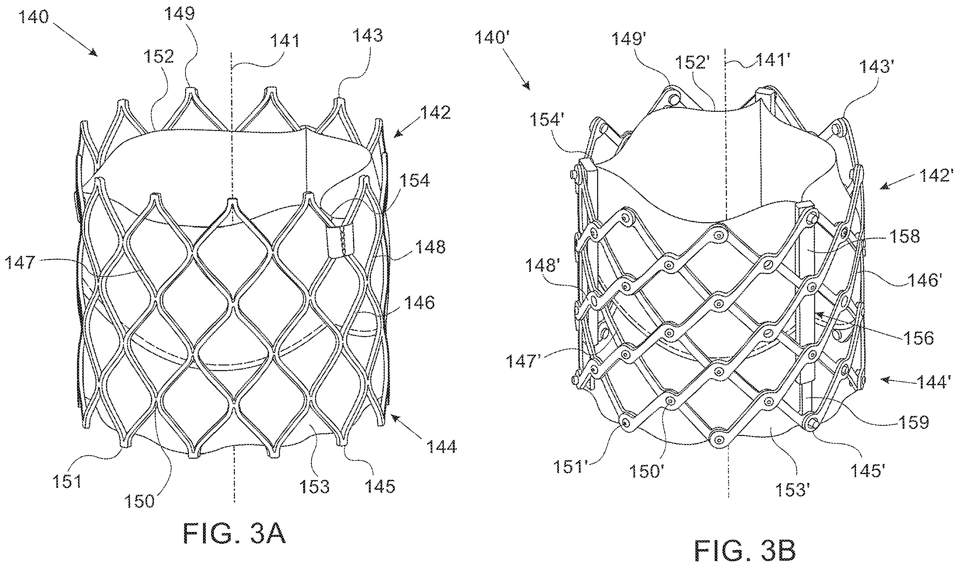

Fig. 3A shows an exemplary prosthetic valve 140 in an expanded state, according to some embodiments. The prosthetic valve 140 can comprise an inflow end portion 144 defining an inflow end 145, and an outflow end portion 142 defining an outflow end 143. The prosthetic valve 140 can define a valve longitudinal axis 141 extending through the inflow end portion 144 and the outflow end portion 142. In some instances, the outflow end 143 is the distal end of the prosthetic valve 140, and the inflow end 145 is the proximal end of the prosthetic valve 140. Alternatively, depending for example on the delivery approach of the valve, the outflow end can be the proximal end of the prosthetic valve, and the inflow end can be the distal end of the prosthetic valve.

The term "outflow", as used herein, refers to a region of the prosthetic valve through which the blood flows through and out of the valve 140, for example between the valve longitudinal axis 141 and the outflow end 143.

The term "inflow", as used herein, refers to a region of the prosthetic valve through which the blood flows into the valve 140, for example between inflow end 145 and the valve longitudinal axis 141.

The valve 140 comprises a frame 146 composed of interconnected struts 148. The frame can be made of various suitable materials, including plastically-expandable materials such as, but not limited to, stainless steel, a nickel based alloy (e.g., a cobalt-chromium or a nickel-cobalt-chromium alloy such as MP35N alloy), polymers, or combinations thereof. When constructed of a plastically-expandable materials, the frame 146 (and thus the prosthetic valve 140) can be crimped to a radially compressed state on a delivery shaft 106, and then expanded inside a patient by an inflatable balloon or equivalent expansion mechanism. Alternatively or additionally, the frame 146 can be made of self-expanding materials such as, but not limited to, nickel titanium alloy (e.g., Nitinol). When constructed of a self-expandable material, the frame 146 (and thus the prosthetic valve 140) can be crimped to a radially compressed state and restrained in the compressed state by insertion into a shaft or equivalent mechanism of a delivery apparatus 102.

In the exemplary embodiment shown in Fig. 3A, the end portions of the struts 148 are forming apices 149 at the outflow end 143 and apices 151 at the inflow end 145. The struts 148 can be interconnected with each other at additional junctions 150 formed between the outflow apices 149 and the inflow apices 151. The junctions 150 can be equally or unequally spaced apart from each other, and/or from the apices 149, 151, between the outflow end 143 and the inflow end 145. The struts 148 collectively define a plurality of open cells 147 of the frame 146. According to some embodiments, as shown in the exemplary embodiments of Fig. 3A, the struts 148 may be formed with alternating bends that may be welded or otherwise secured to each other at junctions 150.

A prosthetic valve 140 further comprises one or more leaflets 152, e.g., three leaflets, configured to regulate blood flow through the prosthetic valve 140 from the inflow end 145 to the outflow end 143. While three leaflets 152 arranged to collapse in a tricuspid arrangement, are shown in the exemplary embodiment illustrated in Fig. 3A, it will be clear that a prosthetic valve 140 can include any other number of leaflets 152. The leaflets 152 are made of a flexible material, derived from biological materials (e.g., bovine pericardium or pericardium from other sources), bio-compatible synthetic materials, or other suitable materials. The leaflets may be coupled to the frame 146 via commissures 154, either directly or attached to other structural elements connected to the frame 146 or embedded therein, such as commissure posts. Further details regarding prosthetic valves, including the manner in which leaflets may be mounted to their frames, are described in U.S. Patent Nos. 6,730,118, 7,393,360, 7,510,575, 7,993,394 and 8,252,202, and U.S. Patent Application No. 62/614,299, all of which are incorporated herein by reference.

According to some embodiments, the prosthetic valve 140 may further comprise at least one skirt or sealing member, such as the inner skirt 153 shown in the exemplary embodiment illustrated in Fig. 3A. The inner skirt 153 can be mounted on the inner surface of the frame 146, configured to function, for example, as a sealing member to prevent or decrease perivalvular leakage. The inner skirt 153 can further function as an anchoring region for the leaflets 152 to the frame 146, and/or function to protect the leaflets 152 against damage which may be caused by contact with the frame 146, for example during valve crimping or during working cycles of the prosthetic valve 140. Additionally, or alternatively, the prosthetic valve 140 can comprise an outer skirt (not shown) mounted on the outer surface of the frame 146, configure to function, for example, as a sealing member retained between the frame 146 and the surrounding tissue of the native annulus against which the prosthetic valve 140 is mounted, thereby reducing risk of paravalvular leakage past the prosthetic valve 140. Any of the inner skirt 153 and/or outer skirt can be made of various suitable biocompatible materials, such as, but not limited to, various synthetic materials (e.g., PET) or natural tissue (e.g. pericardial tissue).

Fig. 3B illustrates a mechanically expandable valve 140', which is a specific type of the prosthetic valve 140 described herein above, with like parts having a prime designation. According to some embodiments, the struts 148' are arranged in a lattice-type pattern. In the embodiment illustrated in Fig. 3B, the struts 148' are positioned diagonally, or offset at an angle relative to, and radially offset from, the valve longitudinal axis 141' when the prosthetic valve 140' is in an expanded position. It will be clear that the struts 148' can be offset by other angles than those shown in Fig 3B, such as being oriented substantially parallel to the valve longitudinal axis 141'.

According to some embodiments, as further shown in Fig. 3B, the frame 146' may comprise openings or apertures at the regions of apices 149', 151' and junctions 150' of the struts 148'. Respective hinges can be included at locations where the apertures of struts 148' overlap each other, via fasteners, such as rivets or pins, which extend through the apertures. The hinges can allow the struts 148' to pivot relative to one another as the frame 146' is radially expanded or compressed.

In alternative embodiments, the struts are not coupled to each other via respective hinges, but are otherwise pivotable or bendable relative to each other, so as to permit frame expansion or compression. For example, the frame can be formed from a single piece of material, such as a metal tube, via various processes such as, but not limited to, laser cutting, electroforming, and/or physical vapor deposition, while retaining the ability to collapse/expand radially in the absence of hinges and like.

According to some embodiments, a mechanically expandable valve 140' comprises a plurality of actuator assemblies 156, configured to facilitate expansion of the valve 140, and in some instances, to lock the valve 140' at an expanded state, preventing unintentional recompression thereof. Although Fig. 3B illustrates three actuator assemblies 156, mounted to, and equally spaced around, an inner surface of the frame 146, it should be clear that a different number of actuator assemblies 156 may be utilized, that the actuator assemblies 156 can be mounted to the frame 146 around its outer surface, and that the circumferential spacing between actuator assemblies 156 can be unequal.

While specific examples of prosthetic valves 140 and 140' are illustrated in Figs. 3A and 3B, respectively, it will be understood that a prosthetic valve 140 can take many other forms known in the art. Any reference to a prosthetic valve 140 throughout the current disclosure, relates to any type of a prosthetic valve, including the embodiment of the prosthetic valve 140 illustrated in Fig. 3A and the embodiment of a mechanically expandable valve 140' illustrated in Fig. 3B, unless stated otherwise.

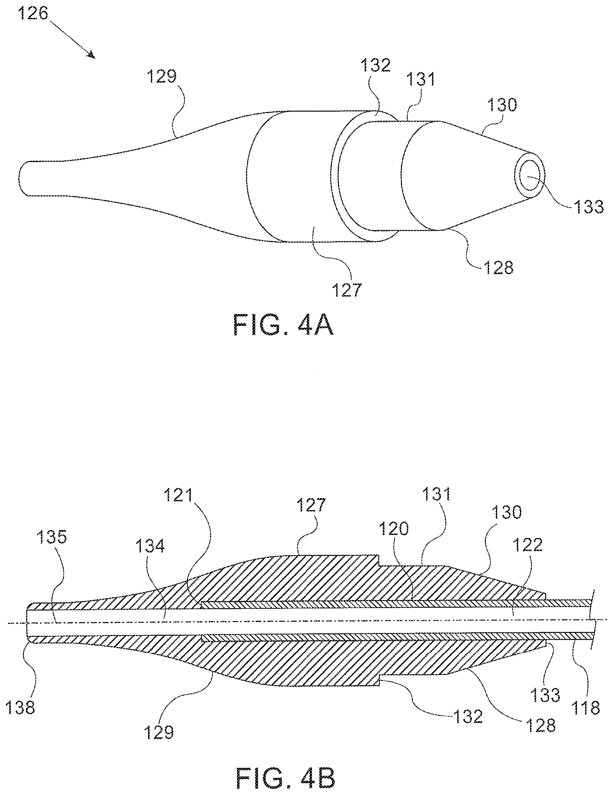

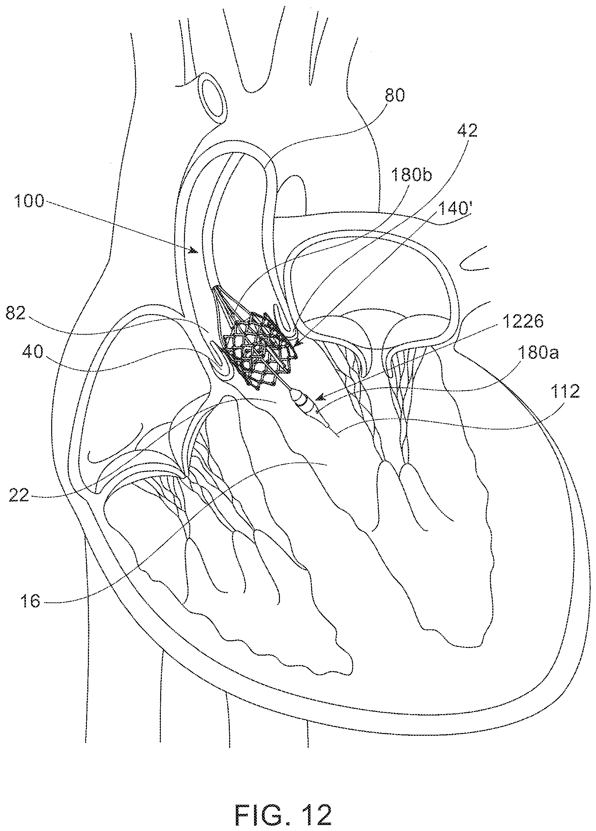

Figs. 4A and 4B constitute a view in perspective and a cross-sectional side view of an exemplary conventional nosecone 130, having a nosecone outer surface (also termed herein an NC outer surface) 127. The nosecone 126 can be connected to the distal end of the NC shaft 118. A guidewire (GW) 112 (not shown in Fig. 2, but visible, for example, in Fig. 12) can extend through a nosecone shaft guidewire lumen (also termed herein an NC shaft GW lumen) 122 and a nosecone guidewire lumen (also termed herein NC GW lumen) 134, so that the delivery apparatus 102 can be advanced over the guidewire 112 through the patient's vasculature. According to some embodiments, the nosecone 126 can be made of a low durometer polymer, such as Pebax (e.g., a 35-shore Pebax).

According to some embodiments, the NC shaft 118 comprises an NC shaft distal portion 120 extending into the nosecone 126 through a nosecone proximal opening (also termed herein an NC proximal opening) 133. The nosecone 126 can be overmolded onto the NC shaft distal portion 120 or formed as a separate part and bonded thereto. According to some embodiments, a retention ring (not shown) can be rigidly attached to the NC shaft distal portion 120, and the nosecone 126 can then be overmolded over the NC shaft distal portion 120 with the retention ring, to create a retention channel over the retention ring, thereby forming a tight fit between the nosecone 126 and the NC shaft distal portion 120, configured to prevent spontaneous axial displacement there-between.

According to some embodiments, a nosecone shaft distal end (also termed herein an NS shaft distal end) 121 is rigidly attached to a proximal surface of the nosecone 126, around the edge of the NC proximal opening 133 (embodiments not shown).

According to some embodiments, the NC shaft distal portion 120 extends at least up to the nosecone distal end (also termed herein the NC distal end) 138, or extends beyond the NC distal end 138, such that the NC GW lumen 134 overlays along its entire length a portion of the outer surface of the NC shaft distal portion 120 (embodiments not shown). The nosecone 126 can define a guidewire lumen longitudinal axis (also termed herein a GW lumen longitudinal axis) 135 extending through the NC proximal opening 133 and the NC distal end 138. In some instances, the GW lumen longitudinal axis 135 and the valve longitudinal axis 141 may coincide.

According to some embodiments, the nosecone 126 comprises an atraumatic nosecone distal portion (also termed herein an NC distal portion) 129 tapering in a distal direction, formed to provide smooth transition with the guidewire 112 when extending there through.

According to some embodiments, the nosecone 126 further comprises a nosecone proximal portion (also termed herein an NC proximal portion) 128, which may have an outer diameter that is smaller than the proximal-most end of the NC distal portion 129, so as to define a shoulder or nosecone ridge (also termed herein an NC ridge) 132.

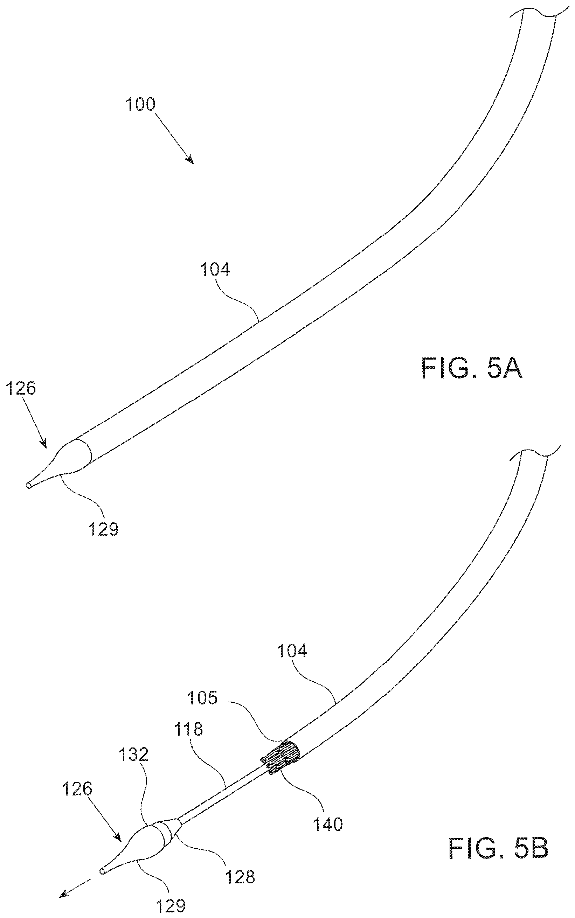

The NC proximal portion 128 can include, as illustrated in Figs. 4A-4B, a nosecone proximal cylindrical portion (also termed herein an NC proximal cylindrical portion) 131 extending proximally from the NC ridge 132, having a uniform outer diameter, desirably sized to allow the distal portion of the outer shaft 104 to extend over it, and allow the outer shaft distal lip 105 (shown for example in Figs. 5B-5C) to abut or press against the nosecone ridge 132. The NC proximal portion 128 can further include a nosecone proximal inclined portion (also termed herein an NC proximal inclined portion) 130, tapering from a larger diameter at the proximal most end of the NC proximal cylindrical portion 131 to a smaller diameter at the proximal-most end of the nosecone 126. This may aid in retracting the nosecone 126 back through the prosthetic valve 140 into the delivery shaft 106 after the prosthetic valve 140 has been expanded. However, in other embodiments, the NC proximal portion 128 may not include NC proximal inclined portion 130. Similarly, the NC proximal portion 128 may be also provided with different geometrical shapes than those described above.

Figs. 5A-5C show the distal portion of the delivery assembly 100 at different phases of a prosthetic valve 140 delivery and expansion procedure. Prior to implantation, the prosthetic valve 140 can be crimped onto the delivery apparatus 102. This step can include placement of the radially compressed valve 140' within the outer shaft 104. A distal end portion of the outer shaft 104 can extend over the prosthetic valve 140 and contact the nosecone 126 in a delivery configuration of the delivery apparatus 102. Thus, the distal end portion of the outer shaft 104 can serve as a delivery capsule that contains, or houses, the prosthetic valve 140 in a radially compressed or crimped configuration for delivery through the patient's vasculature. Fig. 5A shows an exemplary embodiment of a distal portion of the outer shaft 104 extending over a crimped prosthetic valve (hidden from view), having the outer shaft distal lip 105 pressed against the NC ridge 132 (both are visible in Figs. 5B-5C). According to some embodiments, the maximal diameter of the NC distal portion 129 is substantially equal to the outer diameter of the outer shaft 104, to provide a smooth transition between the nosecone 126 and the outer shaft 104.

The outer shaft 104 and the delivery shaft 106 can be configured to be axially movable relative to each other, such that a proximally oriented movement of the outer shaft 104 relative to the delivery shaft 106, or a distally oriented movement of the delivery shaft 106 relative to the outer shaft 104, can expose the prosthetic valve 140 from the outer shaft 104 as shown in Fig. 5B. In alternative embodiments, the prosthetic valve 140 is not housed within the outer shaft 104 during delivery. Thus, according to some embodiments, the delivery apparatus 102 does not include an outer shaft 104.

According to some embodiments, the prosthetic valve 140 is a mechanically expandable valve 140', comprising a plurality of actuator assemblies 156 secured to a frame 146, and configured to radially expand and/or compress the frame 146 via appropriate actuation control mechanisms operable by the handle 110.

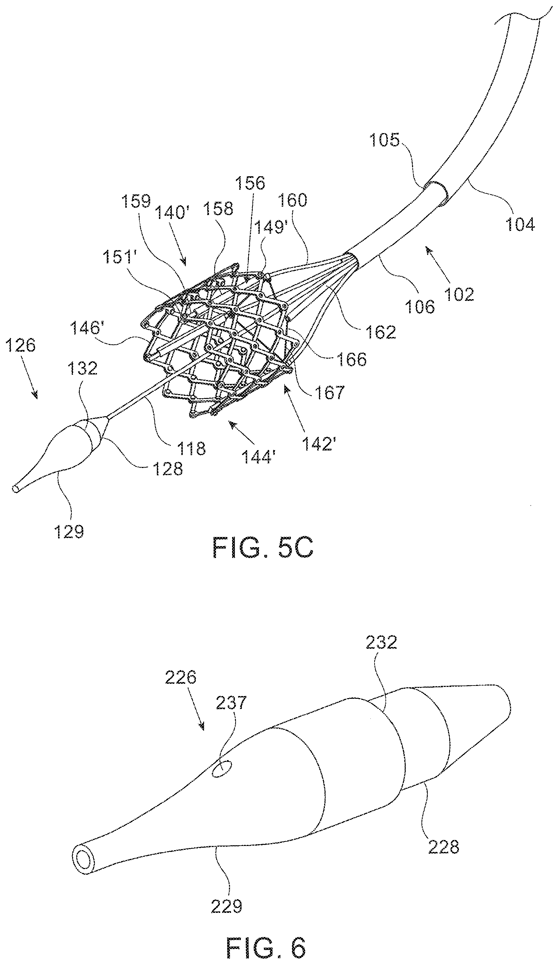

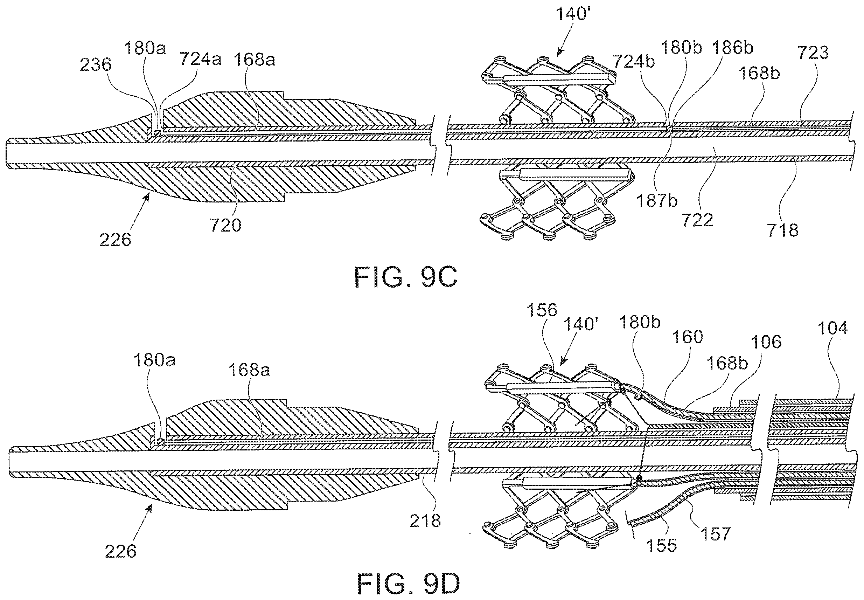

Fig. 5C shows an exemplary mechanically expandable valve 140' in an expanded state, wherein the delivery apparatus 102 further comprises a plurality of actuation arm assemblies 160 extending from the handle 110 through the delivery shaft 106. The actuation arm assemblies 160 can generally include actuation members 155 releasably coupled at their distal ends to respective actuator assemblies 156, and support sleeves 157 disposed around the respective actuation members 155 (actuation members 155 and support sleeves 157 are visible, for example, in a cross-section view of Fig. 9D). Each actuation member 155 may be axially movable relative to the support sleeve 157 covering it. Unless stated otherwise, the leaflets 132, 132' and skirt 136, 136' are omitted from view throughout the figures, for purposes of clarity.

According to some embodiments, each actuator assembly 156 comprises an inner member 159 that may partially extend through a lumen of an outer member 158. The inner member can be attached to the frame 146' at one end thereof, such as an inflow apex 151' or another junction 150' along the inflow end portion 144'. The outer member can be attached to the frame 146' at an opposite end thereof, such as an outflow apex 149' or another junction 150' along the outflow end portion 142'.

According to some embodiments, the actuation arm assemblies 160 are configured to releasably couple to the prosthetic valve 140', and to move the prosthetic valve 140' between the radially compressed and the radially expanded states. For example, the actuation member 155 of the actuation arm assemblies 160 can be threadedly attached at its distal end, to a receiving threaded bore at the proximal end of the inner member 159. The distal edge of the support sleeve 157, covering the actuation member 155, can abut or engage the proximal end of the outer member 158, so as to prevent the outer member 158 from moving proximally beyond the support sleeve 157.

In order to radially expand the frame 146', and therefore the prosthetic valve 140', the support sleeve 157 can be held firmly against the outer member 158. The actuation member 155 can then be pulled in a proximally oriented direction. Because the support sleeve 157 is being held against the outer member 158, which is connected to an outflow apex 149', the outflow end 143' of the frame 146' is prevented from moving relative to the support sleeve 157. As such, movement of the actuation member 155 in a proximally oriented direction can cause movement of the inner member 159 in the same direction, thereby causing the frame 146' to foreshorten axially and expand radially. More specifically, when the inner member 159 is moved axially, for example in a proximally oriented direction, within the outer member 158, the junction 150' to which the inner member 159 is attached, moves there along in the same direction toward the opposite junction, to which the outer member 158 is attached. This, in turn, causes the frame 146' to foreshorten axially and expand radially.

Once the desired diameter of the prosthetic valve 140' is reached, the actuation member 155 may be rotated so as to unscrew it from the inner member 159. This rotation serves to disengage between the distal threaded portion of the actuation member 155 and the threaded bore of the inner member (not shown), enabling the actuation arm assemblies 160 to be pulled away, and retracted, together with the delivery apparatus 102, from the patient's body, leaving the prosthetic valve 140' implanted in the patient.

While radial expansion of the frame 146' is achievable by axially moving the inner member 159 in a proximally oriented direction, relative to the outer member 158, it will be understood that similar frame expansion may be achieved by axially pushing an outer member 158 in a distally oriented direction, relative to an inner member 159. Moreover, while the illustrated embodiment of Fig. 5C shows the outer member 158 affixed to an outflow end portion 142' of the frame 146', and an inner member 159 affixed to an inflow end portion 144' of the frame 146', in alternative embodiments, the outer member 158 may be affixed to the inflow end portion 144' of the frame 146', while the inner member 159 may be affixed to the outflow end portion 142' of the frame 146'.

According to some embodiments, the handle 110 can comprise control mechanisms which may include steerable or rotatable knobs, levers, buttons and such, which are manually controllable by an operator to produce axial and/or rotatable movement of different components of the delivery apparatus 102. For example, the handle 110 may comprise one or more manual control knobs, such as a manually rotatable control knob that is effective to pull the actuation members 155 of the actuation arm assemblies 160 when rotated by the operator.

According to other embodiments, control mechanisms in handle 110 and/or other components of the delivery apparatus 102 can be electrically, pneumatically and/or hydraulically controlled. According to some embodiments, the handle 110 can house one or more electric motors which can be actuated by an operator, such as by pressing a button or switch on the handle 110, to produce movement of components of the delivery apparatus 102. For example, the handle 110 may include one or more motors operable to produce linear movement of components of the actuation arm assemblies 160, and/or one or more motors operable to produce rotational movement of the actuation members 155 to disconnect them from the inner members 159. According to some embodiments, one or more manual or electric control mechanism is configured to produce simultaneous linear and/or rotational movement of all of the actuation members 155.

While a specific actuation mechanism is described above, other mechanisms may be employed to promote relative movement between inner and outer members of actuation assemblies, for example via threaded or other engagement mechanisms. Further details regarding the structure and operation of mechanically expandable valves and delivery system thereof are described in US Patent No. 9,827,093, U.S. Patent Application Publication Nos. 2019/0060057, 2018/0153689 and 2018/0344456, and US Patent Application Nos. 62/870,372 and 62/776,348, all of which are incorporated herein by reference.

In some cases, it may be desirable to re-compress an expanded prosthetic valve 140 in situ, for example in order to allow repositioning or re-crossing procedures to be performed, and/or to allow readjustment of the prosthetic valve expansion diameter. According to some embodiments, the delivery apparatus 102 further comprises a re-compression mechanism, configured to facilitate re-compression of a partially or fully expanded prosthetic valve 140. Fig. 5C shows an exemplary re-compression mechanism configured to compress a mechanically expandable prosthetic valve 140', though the mechanism may be similarly applied to other types of prosthetic valves 140.

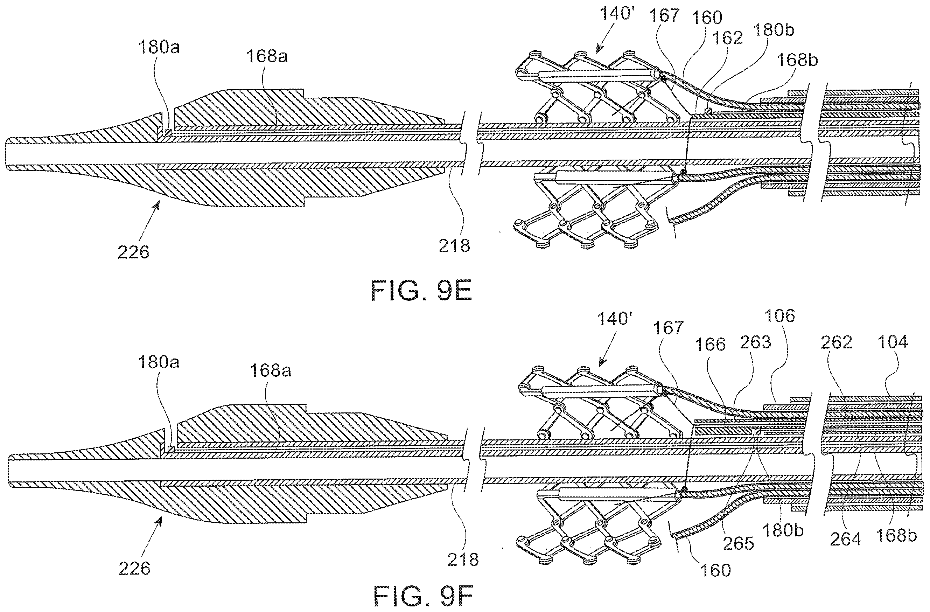

According to some embodiments, the re-compression mechanism comprises a flexible re-compression member 166 extending through a re-compression shaft main lumen 163 (similar to the main lumen 263 shown in Fig. 9F). The re-compression shaft 162 extends through the lumen of the delivery shaft 106. The re-compression member 166 may be formed of a flexible wire, cable, suture and the like. The flexible re-compression member 166 is configured to extend distally through an opening formed at the distal end of the re-compression shaft 162, forming a distal loop 167 that may circumscribe the prosthetic valve 140', or extend around and/or between the actuation arm assemblies 160 attached to the prosthetic valve 140'.

In the exemplary embodiment of Fig. 5C, the distal loop 167 is coupled to and extends between the actuation arm assemblies 160. According to some embodiments, each actuation arm assembly 160 comprises a loop attachment member 161. For example, the support sleeve 157 of each actuation arm assembly 160 can include a loop attachment member 161 at its distal portion, proximate to the prosthetic valve 140' when the actuation arm assembly 160 is attached thereto. The loop attachment members 161 can be provided in the form of eyelets, hooks, rings, clips, apertures within the support sleeves 157, or any other structural elements configured to retain and enable extension of the distal loop 167 there-between. In the specific embodiment illustrated in Fig. 5C, the distal loop 167 extends through loop attachment members 161 in the form of eyelets.

According to some embodiments, relative movement between the re-compression member 166 and the re-compression shaft 162 in the axial direction, is effective to tighten the distal loop 167 connected to and extending between the actuation arm assemblies 160, thereby radially compressing the prosthetic valve 140'. For example, the handle 110 may be maneuvered to pull the re-compression member 166, so as to apply an inwardly directed force on the actuation arm assemblies 160. As long as the actuation arm assemblies 160 are attached to the actuator assemblies 156, the frame 146' of the valve 140' is also proportionally radially compressed.

In alternative embodiments, the distal loop 167 can circumscribing the valve 140' itself, to compress it directly while tightening the distal loop 167, instead of via actuation arm assemblies 160 (embodiments not shown).

As noted above, the re-compression mechanism can be alternatively utilized in conjunction with other types of prosthetic valves 140, such as a self-expandable valve (not shown). A self-expandable valve comprises a flexible frame, configured to expand to an expanded free-state thereof when the prosthetic valve is released from a delivery capsule which retains it in a crimped state during delivery. In such embodiments, the distal loop 167 can circumscribe a self-expandable valve instead of a mechanically-expandable valve, configured to compress it when the re-compression member 166 is pulled via the handle 110. Valve re-expansion may be allowed when the re-compression member 166 is released from the tensioned state (embodiments not shown).

The delivery apparatus 102 further comprises a first sensor 180a retained within a nosecone, such that the first sensor 180a is exposed through a nosecone side opening to the surrounding environment of the nosecone, i.e., exposed to the blood flow around the nosecone of through the nosecone guidewire lumen. The first sensor 180a is configured to measure a physiological flow-related property, such as blood pressure and/or blood flow.

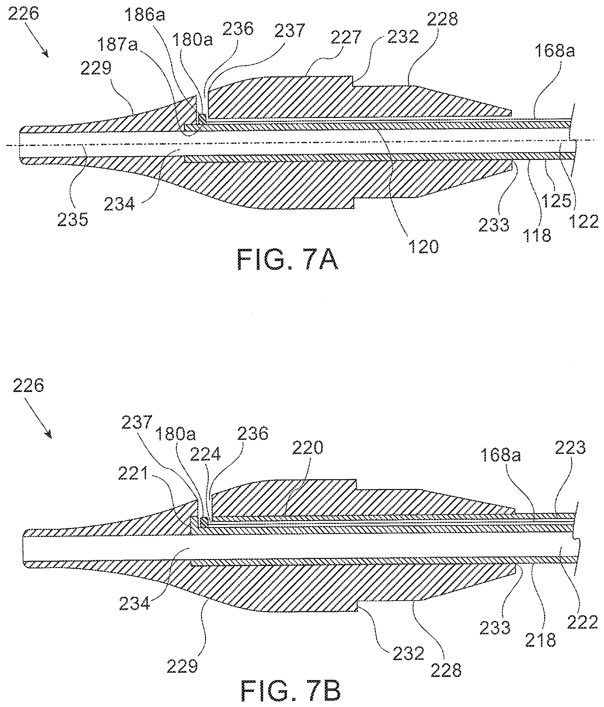

Fig. 6 constitutes a view in perspective of a nosecone 226 configured to retain a first sensor 180a therein (shown, for example, in Fig. 7A). The nosecone 226 is similar to nosecone 126 in structure and function, except that it further comprises a nosecone lateral port (also termed herein an NC lateral port) 236 (shown, for example, in Fig. 7A) for providing transverse access to a first sensor 180a, which may be retained within nosecone 226. The first sensor 180a is positioned within the nosecone in alignment with the NC lateral port 236. As shown in Fig. 6, the NC lateral port 236 may terminate at a nosecone port opening (also termed herein an NC port opening) 237 at an end thereof, such that the first sensor 180a is positioned within the NC lateral port 236, in alignment with the NC port opening 237. According to some embodiments, the NC port opening 237 is formed at the NC outer surface 227, such that the first sensor 180a may be positioned in alignment with the NC port opening 237. Other elements of the nosecone 226 are essentially similar to the elements of the nosecone 126, wherein like reference numerals refer to like parts throughout the figures, and thus will not be further described.

The term "retained within nosecone", with respect to a sensor such as the first sensors 180a, refers to the sensor being positioned within the volume bound between the outer surface of the nosecone and the nosecones longitudinal axis. For example, a first sensor 180a being retained within a nosecone 226, refers to the first sensor 180a being positioned between the NC outer surface 227 and the GW lumen longitudinal axis 235. According to some embodiments, a first sensor 180a is defined as being retained within the nosecone 226 if no portion of the first sensor 180a protrudes radially away from the NC outer surface 227.

Advantageously, configurations in which the first sensor 180a is retained within the nosecone 226, ensure that the NC outer surface 227 remains smooth so as to allow it to easily navigate in the patient's vasculature.

According to some embodiments, the NC distal portion 229 comprises the NC lateral port 236, such that the NC port opening 237 is formed at the outer surface of the NC distal portion 229, as shown in Fig. 6.

Figs. 7A-7G show different configurations of nosecones and NC shafts of a delivery apparatus 102 comprising a first sensor 180a retained within the nosecone. Fig. 7A shows an exemplary configuration of the first sensor 180a retained within a nosecone 226. According to some embodiments, as shown in Fig. 7A, the first sensor 180a is attached to the outer surface of the NC shaft distal portion 120, and positioned such that the first sensor 180a is positioned in, and aligned with, the NC lateral port 236, and more specifically, in alignment with the NC port opening 237. According to some embodiments, the NC lateral port 236 is substantially orthogonal to the NC GW lumen 234, as shown in Fig. 7A.

According to some embodiments, a sensor comprises an active face and a passive face. For example, the first sensor 180a comprises a first active face 186a, defined as the side or surface of the first sensor 180a directed at the measurement region, and an opposite first passive face 187a, which can be the side or surface of the first sensor 180a facing and/or being attached to a component of the delivery assembly 100. In the exemplary embodiment of Fig. 7A, the first sensor 180a is attached at its first passive face 187a to an outer surface of the NC shaft distal portion 120.

According to some embodiments, the first sensor 180a is retained within the nosecone 226, such that the first passive face 187a is oriented toward the GW lumen longitudinal axis 235, while the first active face 186a is oriented toward, and is optionally flush with, the NC port opening 237.

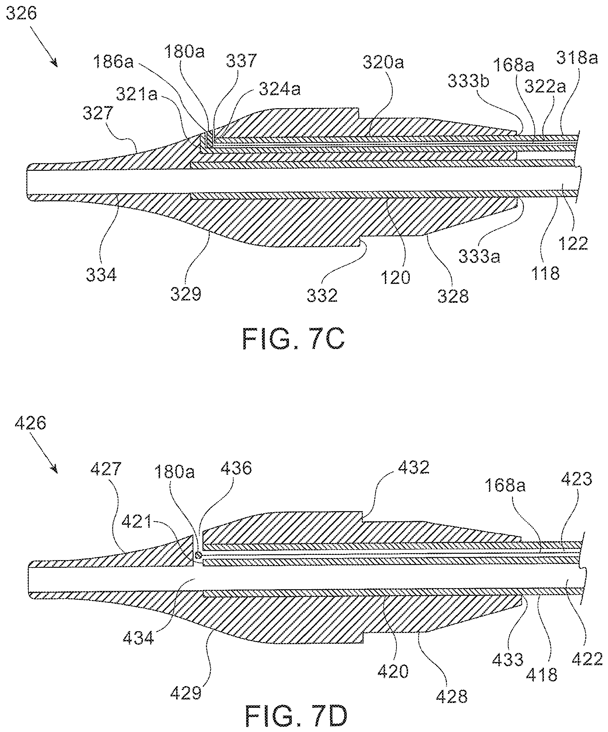

According to some embodiments, the length of the NC lateral port 236 is larger than the height of the first sensor 180a, such that the first active face 186a is positioned radially inward relative to the NC outer surface 127 (as shown for example in Fig. 7A). According to alternative embodiments, the length of the NC lateral port 236 is substantially equal to the height of the first sensor 180a, such that the first active face 186a is flush with the NC outer surface 127 (as shown for example in Fig. 7C). The height of the first sensor 180a is defined as the distance between the first passive face 187a and the first active face 186a.

According to some embodiments, a first transmission line 168a is coupled to the first sensor 180a and extends proximally therefrom, toward the handle 110. According to some embodiments, the first transmission line 168a is configured to deliver power to the first sensor 180a. According to some embodiments, the first transmission line 168a is connected to a proximal power source, for example within the handle 110, configured to provide power to operate the first sensor 180a.

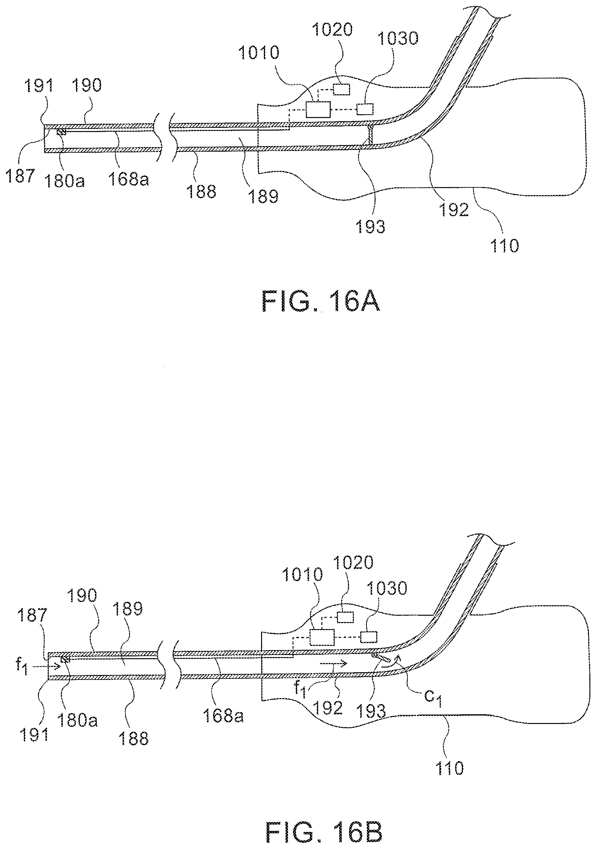

According to some embodiments, the first transmission line 168a is configured to deliver signals (e.g., electric signals and/or optic signals) from, and/or to, the first sensor 180a. According to some embodiments, the first transmission line 168a is connected to an internal control unit 1010 (schematically shown, for example, in Fig. 16A) comprising a processor. The internal control unit 1010 may be embedded within the handle 110, and is configured to receive signals from, and/or transmit signals to, the first sensor 180a.

According to some embodiments, the first transmission line 168a is connected, directly or indirectly (e.g., via the internal control unit 1010) to a proximal communication component 1030 (schematically shown, for example, in Fig. 16A). The proximal communication component 1030 may be operatively coupled to the internal control unit 1010. The proximal communication component 1030 can comprise a transmitter, a receiver, a transceiver, and/or a data communication socket, embedded within the handle 110, and is configured to receive signals from, and/or transmit signals to, components and/or devices external to the delivery assembly 100.

According to some embodiments, the first transmission line 168a is attached to a nosecone shaft outer surface (also termed herein an NC shaft outer surface) 125, or wrapped there-around, for example in a helical pattern (not shown), extending from the first sensor 180a to the handle 110, and optionally further extending into the handle 110.

According to some embodiments, as shown in Fig. 7A, the NC proximal opening 233 is shaped and dimensioned so as to enable both the NC shaft 118 and the first transmission line 168a to extend there-through.

According to some embodiments, the NC shaft is a multi-lumen shaft, including at least one nosecone shaft guidewire lumen and at least one nosecone shaft sensor lumen.

Fig. 7B shows another exemplary configuration of the first sensor 180a retained within the nosecone 226. In the embodiment of Fig. 7B, the nosecone 226 is attached to an NC shaft distal portion 220 of an NC shaft 218. NC shaft 218 is similar in structure and function to NC shaft 118, except that it is provided as a multi-lumen shaft, comprising a nosecone shaft sensor lumen (also termed herein an NC shaft sensor lumen) 223 in addition to the NC shaft GW lumen 222, and a nosecone shaft side opening (also termed herein an NC shaft side opening) 224 at the NC shaft distal portion 220. The NC shaft side opening 224 extends radially outward from the NC shaft sensor lumen 223. The NC shaft side opening 224 can be adjacent to, or spaced axially away from, the nosecone shaft distal end (also termed herein the NC shaft distal end) 221. Other elements of the NC shaft 218 are essentially similar to the elements of the NC shaft 118, wherein like reference numerals refer to like parts throughout the figures, and thus will not be further described. According to some embodiments, the NC shaft sensor lumen 223 is close-ended at the NC shaft distal end 221, as shown in Fig. 7B.

According to some embodiments, the first sensor 180a is positioned within the NC shaft sensor lumen 223, in alignment with the NC shaft side opening 224. According to some embodiments, the first sensor 180a is attached to an inner surface of the NC shaft sensor lumen 223. According to some embodiments, the first sensor 180a is attached at its first passive face 187a to the inner surface of the NC shaft sensor lumen 223, while the first active face 186a is oriented toward, and is optionally flush with, the NC shaft side opening 224.

The NC shaft side opening 224 is aligned with, and in fluid communication with, the NC lateral port 236, together forming a continuous channel or port configured to expose the first sensor 180a, in particular the first active face 186a, to the blood flow adjacent the NC port opening 237 when in use.

According to some embodiments, the first transmission line 168a extends axially through the NC shaft sensor lumen 223, from the first sensor 180a toward the handle 110. According to some embodiments, the first transmission line 168a is attached to the inner surface of the NC shaft sensor lumen 223. According to some embodiments, the NC proximal opening 233 is shaped and dimensioned so as to enable a multi-lumen NC shaft 218 to extend there-through.

According to some embodiments, the delivery apparatus 102 further comprises at least one sensor shaft 318 extending distally from the handle 110, and configured to carry at least one sensor attached thereto, or retained therein. The at least one sensor can be attached to a sensor shaft distal portion 320. According to some embodiments, the at least one sensor shaft is a first sensor shaft 318a comprising a first sensor shaft distal portion 320a, and defining a first sensor shaft lumen 322a. According to some embodiments, the first sensor shaft 318a extends axially through the lumen of the delivery shaft 108. According to some embodiments, the first sensor shaft distal portion 320a is attached to the nosecone.

Fig. 7C shows an exemplary configuration of the first sensor 180a retained within a nosecone 326, wherein the first sensor 180a is attached to a first sensor shaft distal portion 320a. The nosecone 326 is similar in structure and function to nosecone 226, except that nosecone 326 is attached both to the NC shaft 118, via the NC shaft distal portion 120, and to the first sensor shaft 318a, via the first sensor shaft distal portion 320a. Specifically, the NC shaft 118 and the first sensor shaft 318a extend into the nosecone 326 through a first nosecone proximal opening 333a and a second nosecone proximal opening 333b, respectively. Other elements of the nosecone 326 are essentially similar to the elements of the nosecone 226, wherein like reference numerals refer to like parts throughout the figures, and thus will not be further described.

According to some embodiments, the first sensor shaft 318a comprises a first sensor shaft side opening 324a at the first sensor shaft distal portion 320a. The first sensor shaft side opening 324a can be adjacent to, or spaced axially away from, a first sensor shaft distal end 321a. According to some embodiments, the first sensor shaft lumen 322a is close-ended at the first sensor shaft distal end 321a, as shown in Fig. 7C. Alternatively, the first sensor shaft 318a may comprise a distal axial opening (not shown), for example a distal axial opening through which the first sensor 180a may extend.

The first sensor 180a shown in Fig. 7C is positioned within first sensor shaft lumen 322a, in alignment with the first sensor shaft side opening 324a. According to some embodiments, the first sensor 180a is attached to an inner surface of the first sensor shaft lumen 322a. According to some embodiments, the first sensor 180a is attached, at its first passive face 187a, to the inner surface of the first sensor shaft lumen 322a, while the first active face 186a is oriented toward the first sensor shaft side opening 324a. In the embodiment illustrated in Fig. 7C, the first active face 186a is substantially flush with the NC outer surface 327.

The first sensor shaft side opening 324a is aligned with the NC lateral port 236, so as to form fluid connection between both, together forming a continuous channel or port configured to expose the first sensor 180a to the blood flow adjacent the NC port opening 237 when in use.

According to some embodiments, the first transmission line 168a extends axially through the first sensor shaft lumen 322a, from the first sensor 180a toward the handle 110. According to some embodiments, the first transmission line 182a is attached to the inner surface of the first sensor shaft lumen 322a. According to some embodiments, the second nosecone proximal opening 333b is shaped and dimensioned so as to enable the first sensor shaft 318a to extend there-through.

According to some embodiments, the first sensor 180a is retained within a nosecone in such a manner that it may be exposed to either the surrounding environment around the NC outer surface, the NC GW lumen, or both.

Fig. 7D shows an exemplary configuration of the first sensor 180a retained within the nosecone 426, such that the first sensor 180a may be exposed to either the surrounding environment around the NC outer surface 427, the NC GW lumen 434, or both. In the embodiment of Fig. 7D, the nosecone 426 is attached to the NC shaft distal portion 420 of an NC shaft 418. NC shaft 418 is a multi-lumen shaft which is similar in structure and function to the multi-lumen NC shaft 218, except that the NC shaft sensor lumen 423 is open-ended at the NC shaft distal end 421. The NC shaft 418 may or may not include an NC shaft side opening. In the exemplary embodiment of Fig. 7D, the NC shaft 418 is devoid of an NC shaft side opening. Other elements of the NC shaft 418 are essentially similar to the elements of the NC shaft 218, wherein like reference numerals refer to like parts throughout the figures, and thus will not be further described.

The nosecone 426 is similar in structure and function to nosecone 226, except that the NC lateral port 436 extends radially from the NC GW lumen 434 to the NC outer surface 427. The NC shaft 418 may terminate at or proximal to the NC lateral port 436, without protruding into the NC lateral port 436. For example, the NC shaft distal end 421 may be flush with a proximal edge of the NC lateral port 436. Other elements of the nosecone 426 are essentially similar to the elements of the nosecone 226, wherein like reference numerals refer to like parts throughout the figures, and thus will not be further described.

According to some embodiments, the first sensor 180a may be positioned within the NC lateral port 436. For example, the first transmission line 168a can extend through the NC shaft sensor lumen 423 such that the first sensor 180a may extend distally to the NC shaft distal end 421.

A first sensor 180a positioned within the NC lateral port 436 may be exposed to the surrounding environment around the NC outer surface 427, the NC GW lumen 434, or both. According to some embodiments, as illustrated in Fig. 7D, the first active face 186a is oriented toward the NC outer surface 427, while the first passive face 187a is oriented towards the NC GW lumen 434. According to some embodiments, the first active face 186a is oriented towards the NC GW lumen 434, while the first passive face 187a is oriented toward the NC outer surface 427. According to some embodiments, the first sensor 180a comprises at least two diametrically opposing first active faces, such that one active face is oriented towards the NC GW lumen 434, and the opposite active face is oriented towards the NC GW lumen 434. According to some embodiments, the first sensor 180a may be rotated around its axis of symmetry, for example, via the first transmission line 186, maneuverable by the handle 110, such that the orientation of the first active face 186a can be switched between the NC outer surface 427 and the NC GW lumen 434.

According to some embodiments, neither the first transmission line 168a nor the first sensor 180a are rigidly attached to the nosecone shaft 418, rather these elements are configured to be axially movable relative to the nosecone shaft 418. Such embodiments may enable insertion and retraction of a first sensor 180a through the NC shaft sensor lumen 423, for example, if removal or replacement of the first sensor 180a is required.

According to some embodiments, the delivery apparatus 102 comprises a first sensor 180a retained within a nosecone, such that the first sensor 180a is exposed to the NC GW lumen and not to the NC outer surface. It will be clear that the term "NC GW lumen" refers to the entire length of such a lumen, extending from the NC distal end to the NC proximal opening, which may coincide with a portion of the NC shaft GW lumen, at least along the portion of the NC shaft distal portion which is attached to the nosecone.

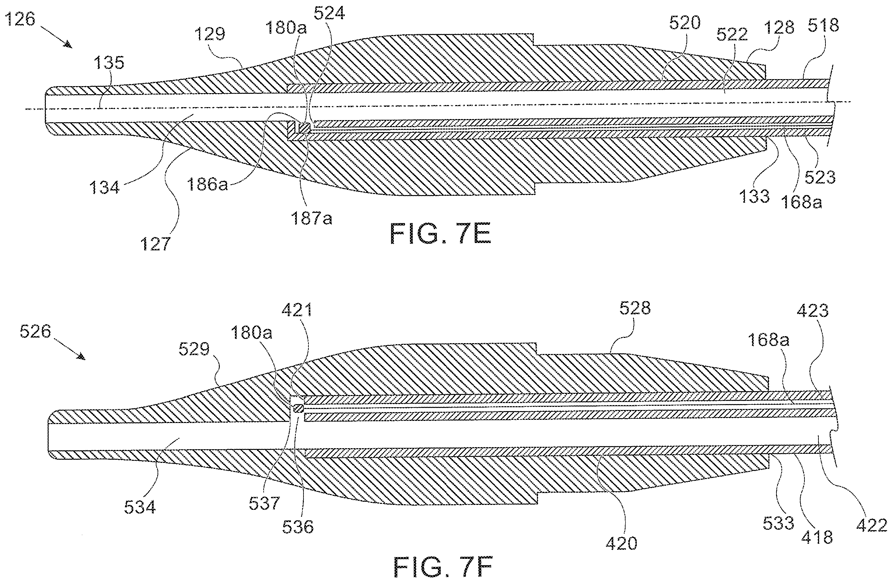

Fig. 7E shows an exemplary configuration of the first sensor 180a retained within a nosecone 126, such that the first sensor 180a is exposed to the NC GW lumen 134. In the exemplary configuration shown in Fig. 7E, the nosecone 126 is attached to an NC shaft distal portion 520 of a multi-lumen NC shaft 518. The NC shaft 518 is similar in structure and function to nosecone shaft 218, except that the NC shaft side opening 524 extends radially inward from the NC shaft sensor lumen 523, toward the NC GW lumen 134. Other elements of the NC shaft 518 are essentially similar to the elements of the NC shaft 218, wherein like reference numerals refer to like parts throughout the figures, and thus will not be further described.

According to some embodiments, the first sensor 180a is positioned within the NC shaft sensor lumen 523, in alignment with the NC shaft side opening 524. According to some embodiments, the first sensor 180a is attached to an inner surface of the NC shaft sensor lumen 523. According to some embodiments, the first sensor 180a is attached at its first passive face 187a to the inner surface of the NC shaft sensor lumen 523, while the first active face 186a is oriented toward, and is optionally flush with, the NC shaft side opening 524. More specifically, the first active face 186a is oriented toward the NC GW lumen 134.

According to some embodiments, the first sensor 180a is retained within the nosecone 126, such that the first active face 186a is oriented toward the GW lumen longitudinal axis 135, while the first passive face 187a is oriented toward the NC outer surface 127.

According to some embodiments, the first transmission line 168a extends axially through the NC shaft sensor lumen 523, from the first sensor 180a toward the handle 110. According to some embodiments, the first transmission line 168a is attached to the inner surface of the NC shaft sensor lumen 523. According to some embodiments, the NC proximal opening 133 is shaped and dimensioned so as to enable a multi-lumen NC shaft 518 to extend there-through.

Fig. 7F shows another exemplary configuration of the first sensor 180a retained within a nosecone 526. In the embodiment of Fig. 7F, the nosecone 526 is attached to the NC shaft distal portion 420 of the NC shaft 418. The nosecone 526 is similar in structure and function to the nosecone 426, except that the NC lateral port 536 extends radially from the NC GW lumen 534 toward the NC outer surface 527, but does not extend all the way to the NC outer surface 527. Thus, the NC lateral port 536 is in fluid communication with the NC GW lumen 534 at an NC port opening 537 formed there-between. Other elements of the nosecone 526 are essentially similar to the elements of the nosecone 426, wherein like reference numerals refer to like parts throughout the figures, and thus will not be further described.

According to some embodiments, the first sensor 180a may be positioned within the NC lateral port 536. A first sensor 180a positioned within the NC lateral port 536 may be exposed to the NC GW lumen 534.

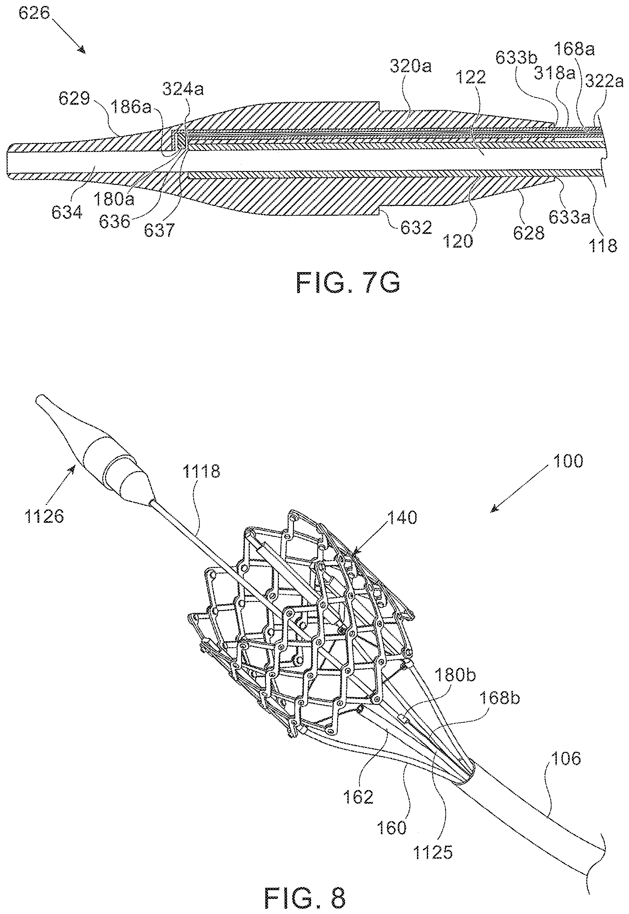

Fig. 7G shows an additional exemplary configuration of the first sensor 180a retained within a nosecone 626. As shown in Fig. 7G, the nosecone 626 attached both to the NC shaft 118, via the NC shaft distal portion 120, and the first sensor shaft 318a, via the first sensor shaft distal portion 320a. The nosecone 626 is similar in structure and function to nosecone 326, except that the NC lateral port 636 extends from the first sensor shaft distal portion 320a to the NC GW lumen 634. Other elements of the nosecone 626 are essentially similar to the elements of the nosecone 326, wherein like reference numerals refer to like parts throughout the figures, and thus will not be further described.

The first sensor 180a shown in Fig. 7G is positioned within first sensor shaft lumen 322a, in alignment with the first sensor shaft side opening 324a, wherein the first active face 186a is oriented towards the NC GW lumen 634.

The first sensor shaft side opening 324a is aligned with the NC lateral port 636, so as to form fluid connection between both, together forming a continuous channel or port configured to expose the first sensor 180a to the blood flow adjacent the NC port opening 637 when in use.

While several configurations for nosecones with respective nosecone shafts and/or a first sensor shaft are illustrated and described in conjunction with Figs. 7A-7G, it will be clear that other additional embodiments or configurations, having a first sensor 180a retained within a nosecone and exposed either to the surrounding environment around the NC outer surface, or to the NC GW lumen, are contemplated.

The terms "including" and/or "having", as used herein (including the specification and the claims), are defined as comprising (i.e., open language).

According to some embodiments, the delivery apparatus 102 comprises a nosecone 1226, configured to retain a first sensor 180a such that the first sensor 180a is exposed to a side opening at the NC outer surface. The nosecone 1226 may take the form of any of the nosecones 226, 326 or 426. According to some embodiments, the delivery apparatus 102 comprises a nosecone 1326, configured to retain a first sensor 180a such that the first sensor 180a is exposed to the NC GW lumen. The nosecone 1326 may take the form of any of the nosecones 126, 426, 526 or 626. According to some embodiments, the delivery apparatus 102 comprises a nosecone 1126, configured to retain a first sensor 180a therein. The nosecone 1126 may take the form of any of the nosecones 1226 or 1326. According to some embodiments, the delivery apparatus comprises an NC shaft 1118 attached to the nosecone 1126. The NC shaft 1118 may take the form of any of the NC shafts 118, 218, 318, 418 or 518. According to some embodiments, the delivery apparatus 102 further comprises a first sensor shaft 318a, coupled to the first sensor 180a.

According to some embodiments, the delivery apparatus 102 further comprises a second sensor 180b positioned proximal to the first sensor 180a. According to some embodiments, the second sensor 180b is positioned proximal to a nosecone 1126. According to some embodiments, the second sensor 180b is positioned proximal to a prosthetic valve 140. According to some embodiments, the prosthetic valve 140 of a delivery assembly 100 equipped with a first sensor 180a and a second sensor 180b, is a non-balloon expandable valve, and the second sensor 180b is positioned proximal to the non-balloon expandable valve.

The term "non-balloon expandable valve" refers to either self-expandable prosthetic valves or mechanically expandable prosthetic valves, but not to balloon-expandable prosthetic valves.

The second sensor 180b can be coupled to any one of: the NC shaft 1118, the delivery shaft, an actuation arm assembly 160 (when present), a re-compression shaft (when present) and/or a sensor shaft (when present).

Fig. 8 constitutes a view in perspective of a distal region of an exemplary delivery assembly 100, provided with a first sensor 180a retained within a nosecone 1126 (the first sensor 180a is hidden from view in Fig. 8), positioned distal to a mechanically expandable valve 140', and a second sensor 180b positioned proximal to the mechanically expandable valve 140'. In the exemplary embodiment shown in Fig. 8, the second sensor 180b is attached to the NC shaft outer surface 1125, at a position proximal to the mechanically expandable valve 140'.

The first and second sensors 180a and 180b, respectively, are capable of sensing and/or measuring a physiologic parameter, including real time blood pressure and/or blood flow velocity, and generate a signal (e.g., an electric signal or an optic signal) representative of the physiologic parameter. According to some embodiments, the first and second sensors 180a and 180b, respectively, are flow sensors. According to some embodiments, the first and second sensors 180a and 180b, respectively, are pressure sensors, configured to provide time-resolved blood pressure data which can be correlated to parameters of interest, based on known empirical correlations that are known in the art. The measurement range for the first and second sensors 180a and 180b, respectively, is sufficient to measure normal and high physiologic pressures and/or flow rates within the cardiovascular system, from which differential values can be calculated.

Similarly to first sensor 180a, the second sensor 180b may comprise a second active face 186b, defined as the side or surface of the second sensor 180b directed at the measurement region, and an opposite second passive face 187b, which can be the side or surface of the second sensor 180b attached to a component of the delivery assembly 100.

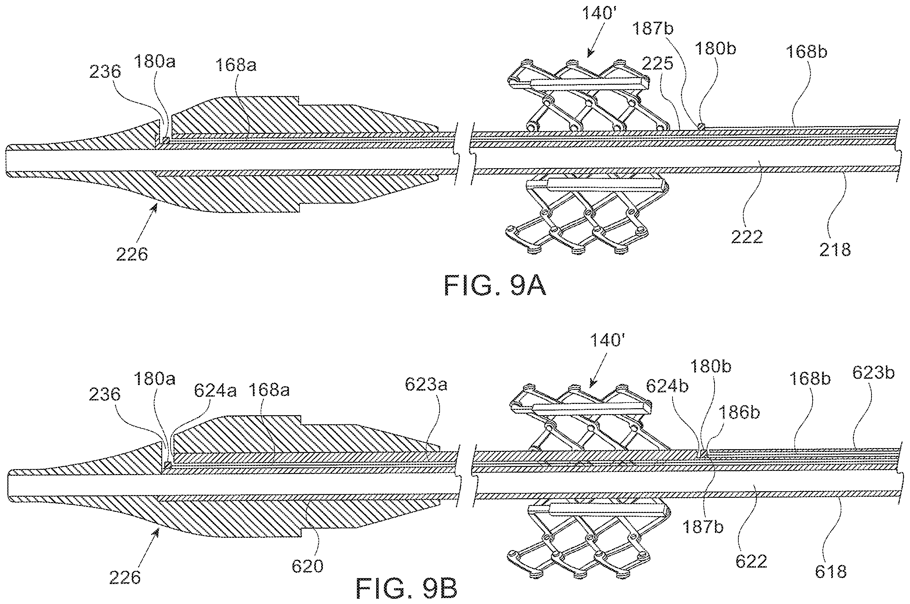

Figs. 9A-9G show cross-sectional views of various configurations of a delivery apparatus 100 comprising a first sensor 180a retained within the nosecone 1126, and a second sensor 180b positioned proximal to a prosthetic valve 140. While a mechanically expandable valve 140' is illustrated in Figs. 9A-9G, it will be clear that the configurations of these figures apply to other types of prosthetic valves 140 in a similar manner. Moreover, while the first sensor 180a is shown throughout Figs. 9A-9G as being retained within a nosecone 226 and attached to an NC shaft, such as NC shaft 218, in a configuration similar to that shown in Fig. 7B, it will be understood that this configuration is shown as a mere illustrative representation of the position of the first sensor 180a within a nosecone 1126, and that any of the configurations of a first sensor 180a retained within a nosecone, as illustrated and described above, can be implemented in combination with the configurations shown and described for the second sensor 180b in conjunction with Figs. 9A-9G. Similarly, an NC shaft 218 is shown in Figs. 9A and 9D-9G for illustration purpose only, and any NC shaft 1118 can be implemented in combination with the configurations shown and described for the second sensor 180b in conjunction with Figs. 9A and 9D-9G.

Fig. 9A shows one exemplary configuration of a second sensor 180b positioned proximal to a prosthetic valve 140'. According to some embodiments, the second sensor 180b is attached to the NC shaft outer surface 1125, at a position proximal to the prosthetic valve 140. In Fig. 9A, the second sensor 180b is shown attached to the NC shaft outer surface 225, at a position proximal to the prosthetic valve 140'. As further illustrated in the exemplary embodiment of Fig. 9A, the second sensor 180b may be attached at its second passive face 187b to the NC shaft outer surface 225.

According to some embodiments, a second transmission line 168b is coupled to the second sensor 180b and extends proximally toward the handle 110. According to some embodiments, the second transmission line 168b is configured to deliver power to the second sensor 180b. According to some embodiments, the second transmission line 168b is connected to a proximal power source, for example within the handle 110, configured to provide power to operate the second sensor 180b.

According to some embodiments, the second transmission line 168b is configured to deliver signals (e.g., electric signals and/or optic signals) from, and/or to, the second sensor 180b. According to some embodiments, the second transmission line 168b is connected to the internal control unit 1010. The internal control unit 1010 can may be configured to receive signals from, and/or transmit signals to, the second sensor 180a.

According to some embodiments, the second transmission line 168b is connected, directly or indirectly (e.g., via the internal control unit 1010) to the proximal communication component 1030.

According to some embodiments, the second transmission line 168b is attached to the NC shaft outer surface 1125 (such as the NC shaft outer surface 225 shown in Fig. 9A), or wrapped there-around, for example in a helical pattern (not shown), extending from the second sensor 180b to the handle 110, and optionally further extending into the handle 110.

Fig. 9B shows another exemplary configuration of the second sensor 180b positioned proximal to a prosthetic valve 140'. According to some embodiments, the nosecone 1126 is attached to an NC shaft distal portion 620 of a multi-lumen NC shaft 618. The multi-lumen NC shaft 618 is similar in structure and function to multi-lumen NC shaft 218, except that it comprises at least two NC shaft sensor lumens 623a and 623b. The first NC shaft sensor lumen 623a is similar to the NC shaft sensor lumen 223, and is provided with a first NC shaft side opening 624a, which is structured and positioned similarly to any embodiment disclosed for the NC shaft side opening 224. The NC shaft 618 further comprises a second NC shaft side opening 624b extending radially outward from the second NC shaft sensor lumen 623b. The second NC shaft side opening 624b is positioned proximal to the prosthetic valve 140 (illustrated as prosthetic valve 140' in Fig. 9B). Other elements of the NC shaft 618 are essentially similar to the elements of the NC shaft 218, wherein like reference numerals refer to like parts throughout the figures, and thus will not be further described.

According to some embodiments, the second sensor 180b is positioned within the second NC shaft sensor lumen 623b, in alignment with the second NC shaft side opening 624b. According to some embodiments, the second sensor 180b is attached to an inner surface of the second NC shaft sensor lumen 623b. According to some embodiments, the second sensor 180b is attached at its second passive face 187b to the inner surface of the second NC shaft sensor lumen 623b, while the second active face 186b is oriented toward, and is optionally flush with, the second NC shaft side opening 624b.

According to some embodiments, the second transmission line 168b extends axially through the second NC shaft sensor lumen 623b, from the second sensor 180b toward the handle 110. According to some embodiments, the second transmission line 168b is attached to the inner surface of the second NC shaft sensor lumen 623b.

While not explicitly illustrated, it will be clear that the second sensor 180b and the second transmission line 168b can be similarly retained within a second NC shaft sensor lumen of an NC shaft which is open ended at its NC distal end, with or without an NC shaft side opening extending from the first NC shaft sensor lumen. For example, an NC shaft similar in structure and function to the NC shaft 418, described and illustrated in conjunction with Figs. 7D and 7F, and include a second NC shaft sensor lumen similar to the second NC shaft sensor lumen 623b described herein above. Similarly, the second sensor 180b and the second transmission line 168b can be retained within a second NC shaft sensor lumen of an NC shaft that has a first NC shaft side opening open to the NC GW lumen, such as the NC shaft 518 described and illustrated in conjunction with Fig. 7E.

Fig. 9C shows another exemplary configuration of the second sensor 180b positioned proximal to a prosthetic valve 140'. According to some embodiments, the nosecone 1126 is attached to an NC shaft distal portion 720 of a multi-lumen NC shaft 718. The multi-lumen NC shaft 718 is similar in structure and function to multi-lumen NC shaft 218, except that it comprises at least two NC shaft side openings 724a and 724b, extending radially outward from the same NC shaft sensor lumen 723 at different axial positions. The first NC shaft side opening 724a is structured and positioned similarly to any embodiment disclosed for the NC shaft side opening 224. The second NC shaft side opening 724b is positioned proximal to the prosthetic valve 140 (illustrated as prosthetic valve 140' in Fig. 9C). Other elements of the NC shaft 718 are essentially similar to the elements of the NC shaft 218, wherein like reference numerals refer to like parts throughout the figures, and thus will not be further described.

According to some embodiments, both the first sensor 180a and the second sensor 180b are positioned within the NC shaft sensor lumen 723, wherein the first sensor 180a is positioned in alignment with the first NC shaft side opening 724a, and the second sensor 180b is positioned in alignment with the second NC shaft side opening 724b.

According to some embodiments, the second sensor 180b is attached to an inner surface of the NC shaft sensor lumen 723. According to some embodiments, the second sensor 180b is attached at its second passive face 187b to the inner surface of the NC shaft sensor lumen 723, while the second active face 186b is oriented toward, and is optionally flush with, the second NC shaft side opening 724b.

According to some embodiments, the second transmission line 168b extends axially through the second NC shaft sensor lumen 723, from the second sensor 180b toward the handle 110. According to some embodiments, the second transmission line 168b is attached to the inner surface of the NC shaft sensor lumen 723.

According to some embodiments, the NC shaft sensor lumen 723 is dimensioned to accommodate both the first 168a and the second 168b transmission lines, at least along a portion of the NC shaft sensor lumen 723 extending proximally from the second NC shaft side opening 724b.

While not explicitly illustrated, it will be clear that the second sensor 180b and the second transmission line 168b can be similarly retained within the NC shaft sensor lumen of an NC shaft which is open ended at its NC distal end, with or without an NC shaft side opening extending from the first NC shaft sensor lumen. For example, an NC shaft similar in structure and function to the NC shaft 418, described and illustrated in conjunction with Figs. 7D and 7F, and include a second NC shaft side opening similar to the second NC shaft side opening 624b described herein above. Similarly, the second sensor 180b and the second transmission line 168b can be retained within the NC shaft sensor lumen of an NC shaft that has a first NC shaft side opening open to the NC GW lumen, such as the NC shaft 518 described and illustrated in conjunction with Fig. 7E.

Fig. 9D shows yet another exemplary configuration of the second sensor 180b positioned proximal to a mechanically expandable valve 140', for a delivery apparatus 102 that includes a plurality of actuation arm assemblies 160. In the embodiment of Fig. 9D, the second sensor 180b is attached to the outer surface of one of the plurality of actuation arm assemblies 160. As mentioned, each actuation arm assembly 160 can include an actuation member 155 releasably coupled at their distal ends to respective actuator assemblies 156, and a support sleeve 157 disposed around the actuation member 155. According to some embodiments, the second sensor 180b is attached to the outer surface of a support sleeve 157.

According to some embodiments, the second transmission line 168b is attached to the outer surface of one of the plurality of actuation arm assemblies 160, or wrapped there-around, for example in a helical pattern (not shown), from the second sensor 180b to the handle 110, and optionally further extending into the handle 110. According to some embodiments, the second transmission line 168b is attached to the outer surface of a support sleeve 157.

Fig. 9E shows another exemplary configuration of the second sensor 180b positioned proximal to a prosthetic valve 140', for a delivery apparatus 102 that includes a re-compression mechanism. In the embodiment of Fig. 9E, the second sensor 180b is attached to the outer surface of the re-compression shaft 162.

According to some embodiments, the second transmission line 168b is attached to the outer surface of the re-compression shaft 162, or wrapped there-around, for example in a helical pattern (not shown), and is extending from the second sensor 180b to the handle 110, and optionally further extending into the handle 110.

Fig. 9F shows yet another exemplary configuration of the second sensor 180b positioned proximal to a prosthetic valve 140', for a delivery apparatus 102 that includes a re-compression mechanism. The re-compression mechanism shown in Fig. 9F includes a re-compression shaft 262, which is similar in structure and function to the re-compression shaft 162, except that it is a multi-lumen re-compression shaft that includes a re-compression shaft sensor lumen 264, in addition to the re-compression shaft main lumen 263. The re-compression shaft 262 further comprises a re-compression shaft side opening 265 extending radially outward from the re-compression shaft sensor lumen 264. The re-compression shaft side opening 265 is positioned proximal to the prosthetic valve 140 (illustrated as prosthetic valve 140' in Fig. 9F). Other elements of the re-compression shaft 262 are essentially similar to the elements of the re-compression shaft 162, wherein like reference numerals refer to like parts throughout the figures, and thus will not be further described.

According to some embodiments, the second sensor 180b is positioned within the re-compression shaft sensor lumen 264, in alignment with the re-compression shaft side opening 265. According to some embodiments, the second sensor 180b is attached to an inner surface of the re-compression shaft sensor lumen 264. According to some embodiments, the second sensor 180b is attached at its second passive face 187b to the inner surface of the re-compression shaft sensor lumen 264, while the second active face 186b is oriented toward, and is optionally flush with, the re-compression shaft side opening 265.

According to some embodiments, the second transmission line 168b extends axially through the re-compression shaft sensor lumen 264, from the second sensor 180b toward the handle 110. According to some embodiments, the second transmission line 168b is attached to the inner surface of the re-compression shaft sensor lumen 264.

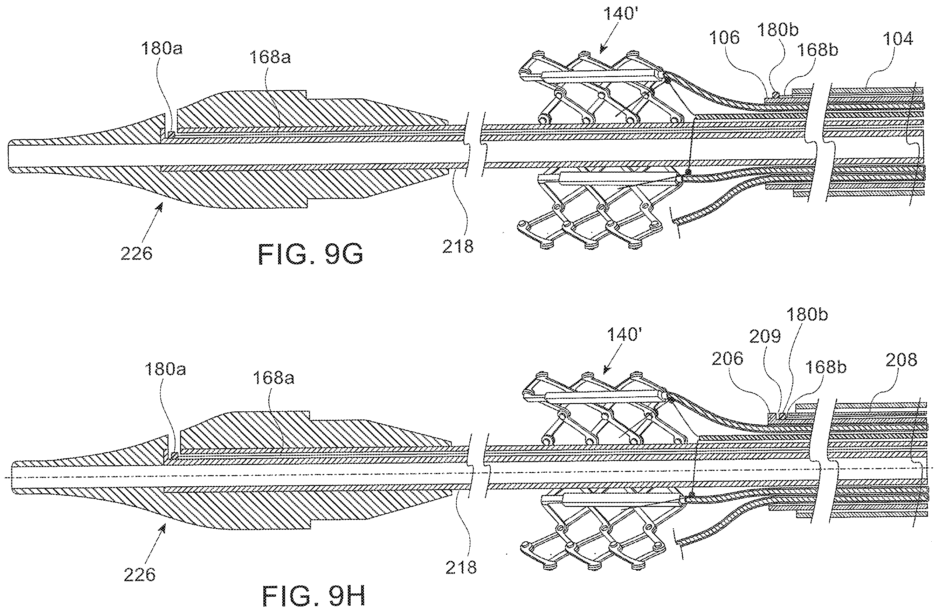

Fig. 9G shows another exemplary configuration of the second sensor 180b positioned proximal to a prosthetic valve 140'. According to some embodiments, the second sensor 180b is attached to the delivery shaft 106. In the embodiment shown in Fig. 9G, the second sensor 180b is attached to the outer surface of the delivery shaft 106. Specifically, the second sensor 180b may be attached at its second passive face 187b to the outer surface of the delivery shaft 106. Alternatively, the second sensor 180b can be attached to the inner surface of the delivery shaft 106.

According to some embodiments, the second transmission line 168b is attached to the outer surface of the delivery shaft 106, or wrapped there-around, for example in a helical pattern (not shown), extending from the second sensor 180b to the handle 110, and optionally further extending into the handle 110. Alternatively or additionally, the second transmission line 168b can be attached to the inner surface of the delivery shaft 106.

Fig. 9H shows an additional exemplary configuration of the second sensor 180b positioned proximal to a prosthetic valve 140'. In the embodiment of Fig. 9H, the second sensor 180b is attached to a delivery shaft 206, which is similar in structure and function to the delivery shaft 106, except that the delivery shaft 206 is a multi-lumen shaft, wherein at least one of the lumens is a delivery shaft sensor lumen 208. The delivery shaft 206 further comprises a delivery shaft side opening 209, extending radially outward from the delivery shaft sensor lumen 208. In use, the delivery shaft 206 is positioned proximal to the prosthetic valve 140 prior to valve expansion, such that the delivery shaft side opening 209 is positioned proximal to the prosthetic valve 140 (illustrated as prosthetic valve 140' in Fig. 9H). Other elements of the delivery shaft 206 are essentially similar to the elements of the delivery shaft 106, wherein like reference numerals refer to like parts throughout the figures, and thus will not be further described.

According to some embodiments, the second sensor 180b is positioned within the delivery shaft sensor lumen 208, in alignment with the delivery shaft side opening 209. According to some embodiments, the second sensor 180b is attached to an inner surface of the delivery shaft sensor lumen 208. According to some embodiments, the second sensor 180b is attached at its second passive face 187b to the inner surface of the delivery shaft sensor lumen 208, while the second active face 186b is oriented toward, and is optionally flush with, the delivery shaft side opening 209. According to some embodiments, the delivery shaft side opening 209 is positioned at an outer surface of the delivery shaft 206. Alternatively, the delivery shaft side opening 209 may be directed toward the GW lumen longitudinal axis 135.

According to some embodiments, the second transmission line 168b extends axially through the delivery shaft sensor lumen 208, from the second sensor 180b toward the handle 110. According to some embodiments, the second transmission line 168b is attached to the inner surface of the delivery shaft sensor lumen 208.

While not explicitly shown, other configurations of a second sensor 180b attached to any component of the delivery apparatus 102, at a position proximal to the prosthetic valve 140, are contemplated. According to some embodiments, the second sensor 180b may be attached to the first sensor shaft 318a which is attached to the nosecone 1126, as shown and described in conjunction with Figs. 7C and 7G. According to some embodiments, the second sensor 180b is attached to the outer surface of the first sensor shaft outer surface 325 at a position proximal to the prosthetic valve 140, in a similar manner described for the attachment of the second sensor 180b to an NC shaft outer surface 225 in conjunction with Fig. 9A. In such embodiments, the second transmission line 168b may be attached to, or wrapped around the, first sensor shaft outer surface 325, extending from the second sensor 180b to the handle 110 (embodiments not shown).

According to some embodiments, a first sensor shaft, such as the first sensor shaft 318a, may include two sensor shaft lumens, each having a side opening, extending radially outward therefrom. The second sensor 180b may be positioned within the second sensor shaft lumen, in alignment with the second sensor shaft side opening at a position proximal to the valve 140, in a similar manner described for the positioning of the second sensor 180b within the second NC shaft sensor lumen 623b in conjunction with Fig. 9B. In such embodiments, the second transmission line 168b may extend axially through the second sensor shaft lumen, from the second sensor 180b toward the handle 110 (embodiments not shown).

According to some embodiments, a first sensor shaft, such as the first sensor shaft 318a, may include two sensor shaft side openings, extending radially outward from the same sensor shaft lumen, at different axial positions. The second sensor 180b may be positioned within the sensor shaft lumen, in alignment with the second sensor shaft side opening, in a similar manner described for the positioning of the second sensor 180b within the NC shaft sensor lumen 723 in conjunction with Fig. 9C. In such embodiments, the second transmission line 168b may extend axially through the sensor shaft lumen, from the second sensor 180b toward the handle 110 (embodiments not shown).

According to some embodiments, the delivery apparatus 102 may further comprise a second sensor shaft 318b, which may be identical to the first sensor shaft 318a, except that the second sensor shaft 318b is not attached to the nosecone 1126, and may optionally translate in an axial direction within the delivery shaft 106. The elements of the second sensor shaft 318b are essentially similar to the elements of the first sensor shaft 318a, wherein like reference numerals refer to like parts throughout the figures, and thus will not be further described. The second sensor 180b can be attached to the second sensor shaft 318b in a similar manner described for the attachment of the first sensor 180a to the first sensor shaft 318a in conjunction with Fig. 7C. However, in use, the second sensor shaft 318b is positioned such that the second sensor shaft side opening 324b, and the second sensor 180b aligned therewith, are proximal to the prosthetic valve 140. The second transmission line 168b may extend axially through the second sensor shaft lumen 322b, from the second sensor 180b toward the handle 110 (embodiments not shown).

Although not treated in full detail, it should be readily understood that a first sensor 180a retained within a nosecone 1126 according to any of the configurations described herein above, can be used in combination with a second sensor 180b positioned and or arranged within the delivery apparatus 102, at a proximal position to the prosthetic valve 140, according to any of the configurations described herein above.

According to some embodiments, any of the first and second sensors 180a and 180b, respectively, may be piezo-resistive pressure sensors, such as MEMS piezo-resistive pressure sensors. According to other embodiments, any of the first and second sensors 180a and 180b, respectively, may be capacitive pressure sensors, such as MEMS capacitive pressure sensors. In such embodiments, the transmission lines 168a and 168b may comprise an electrically conductive medium, such as one or more electrical conductive wires.

According to some embodiments, the first and second sensors 180a and 180b, respectively, are optic fiber pressure sensors, such as Fabry-Perot type pressure sensors 280a and 280b, and the respective transmission lines 182a and 182b are optic fibers 268a and 268b, respectively. Utilization of optic fiber sensors may be advantageous due to their light weight, miniature dimension, low power consumption, high sensitivity, environmental ruggedness and low cost.

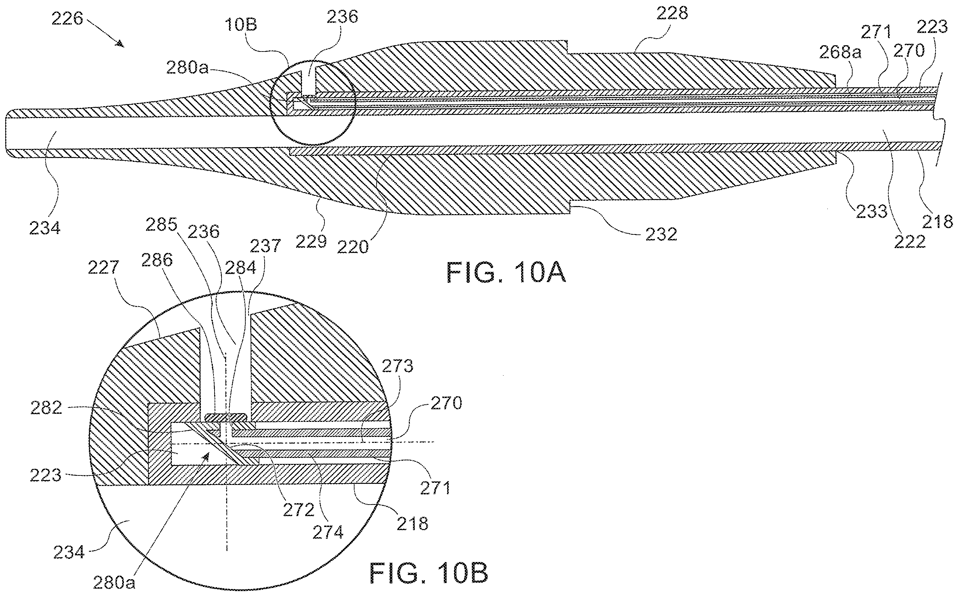

Fig. 10A shows an exemplary nosecone 226 attached to a multi lumen NC shaft 218, similar to the configuration described and illustrated in conjunction with Fig. 7B, wherein the transmission line is an optic fiber 268a and the first sensor is a first optic pressure sensor 280a. Fig. 10B is a zoomed in view of the region 10B in Fig. 10A. In the embodiments illustrated in Figs. 10A-10B, the first optic fiber 268a comprises an optic core 270 surrounded by a cladding 271. According to some embodiments, the first optic fiber 268a may further include a surrounding polymeric buffer coating (not shown) around the cladding 271, serving as a protective buffer from the surrounding environment.

According to some embodiments, the first optic pressure sensor 280a is a Fabry-Perot cavity based sensing head. Fabry-Perot sensors are attractive due to their miniature size and low costs of the sensing elements. A Fabry-Perot sensor detects pressure applied to the diaphragm in a direction perpendicular to the surface of a diaphragm. The Fabry-Perot sensor 280a may include a housing 282 attached to the optic fiber distal end 274, to which a diaphragm 286 is attached. Pressure may be monitored by detecting and measuring the deflection of the housing 282 to which pressure is applied.

According to some embodiments, the first optic pressure sensor 280a is a cross-axial pressure sensor, configured to measure pressure applied thereto in a direction substantially orthogonal to the core axis 273. As shown in Figs. 10A-10B, the optic core 270 terminates at an inclined surface 272, which is angled relative to the core axis 273. An optical side cavity 284 extends through the cladding 271 and the housing 282, overlaid by the diaphragm 286. According to some embodiments, the first optic sensor 280a is devoid of a housing 282, such that the optical side cavity 284 extends through the cladding 271, and the diaphragm 286 is attached to the outer surface of the cladding 271, or any protective layer surrounding the cladding 271 if present (embodiments not shown).

The inclined surface 272 is preferably angled to provide a critical incidence angle for a light beam passing along the optic core 270, in order to ensure a full reflection from the inclined surface 272. Preferably, the inclined surface 272 is angled at a 45° angle relative to the core axis 273. However, it should be understood that other angles between the inclined surface 272 and the core axis 273 may be applicable, as long as a critical incidence angle is provided for the light beam passing through the optic core 270.

As further shown in Fig. 10B, since the inclined surface 272 is angled relative to the core axis 273, a light beam passing through optic core 270 is redirected by 90° relative to the core axis 273. When a redirected light beam impinges on the diaphragm 286, it reflects and is returned to the inclined surface 272 to be redirected back through the optic fiber 268a, for example toward the internal control unit 1010 in the handle 110. Stated otherwise, the diaphragm 286 and the optical side cavity 284 are cross-axially aligned with the core axis 273.

When pressure is applied to the diaphragm 286, the diaphragm 286 bends into the optical side cavity 284, thereby changing the path of the light beam, which changes the phase of the reflected signal.

While Figs. 10A-10B illustrate the structural components of a first sensor 180a and a first transmission line 168a, realized as a cross-axial optic pressure sensor 280a and an optic fiber 286a, it will be clear that the same functional and structural principles similarly apply to the second sensor 180b and the second transmission line 168b, respectively.

Moreover, while the optic pressure sensor 280a and the optic fiber 268a are illustrated in conjunction with a specific configuration in Figs. 10A-10B, positioned within a multi lumen NC shaft 218 attached to a nosecone 226, it will be clear that this configuration is shown for illustrative purpose only, and that any one of sensors 180a, 180b and transmission lines 168a, 168b, illustrated in Figs. 7A-9H, may be realized as an optic pressure sensor and an optic fiber, such as the optic sensor 280a and the optic fiber 268a described herein.

Any reference to a sensor, such as a first sensor 180a or a second sensor 180b, throughout the current disclosure, relates to any type of sensor, including embodiments of the optic pressure sensor illustrated in Figs. 9A-8B, unless stated otherwise. Similarly, any reference to a transmission line, such as a first transmission line 168a or a second transmission line 168b, throughout the current disclosure, relates to any type of a transmission line, including embodiments of the optic fiber illustrated in Figs. 10A-10B, unless stated otherwise.

According to some embodiments, a single optic fiber, similar to the optic fiber 268a, may be a multi-core optic fiber, wherein each core terminates at an optic pressure sensors. The plurality of optic pressure sensors can be axially spaced from each other, such that a first optic pressure sensor is positioned within a nosecone 1126, corresponding to any of the positions disclosed herein for the first sensor 180a, and the second optic pressure sensor is positioned proximally to the prosthetic valve 140, corresponding to any of the positions disclosed herein for the second sensor 168a (embodiments not shown).

Attaching any of the first sensor 180a, first transmission line 182a, second sensor 180b and/or second transmission line 182b, to any component of the delivery apparatus 102 according to any of the embodiments and configuration illustrated and described herein, may be implemented by suturing, screwing, clamping, gluing with bio-compatible adhesives, fastening, welding, or any other suitable technique.

According to some embodiments, any of the first or second sensors 180a and 180b, respectively, include radiopaque markings that may provide a visible indication of the location of the sensors when viewed under fluoroscopy.

It should be noted that in some embodiments, the delivery apparatus 102 can be equipped with more than two sensors. For example, delivery apparatuses having a plurality of first sensors 180a and/or a plurality of second sensors 180b, are contemplated within the scope of the invention.

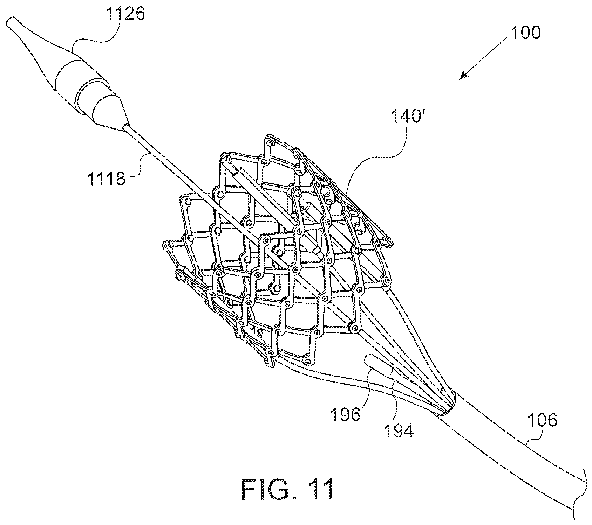

According to some embodiments, the delivery apparatus 102 further comprises a sensing catheter 194 extending from the handle 110 through the delivery shaft 106. Fig. 11 shows a distal region of the delivery assembly 100, wherein the sensing catheter 194 comprises a sensing head 196, which is illustrated extending distally from the delivery shaft 106. While a mechanically expandable valve 140' is illustrated in Fig. 11, it will be clear that the configurations of these figures apply to other types of prosthetic valves 140 in a similar manner.

The sensing catheter 194 may be axially movable relative to the delivery shaft 106. The movement of the sensing catheter 194 may be controlled by the handle 110. The sensing head 196 may comprise a sensor, such as the first sensor 180a or the second sensor 180b according to any of the embodiments disclosed herein. The sensing catheter 194 may further comprise a transmission line extending from the sensor head toward the handle 100, such as the first transmission line 168a or the second transmission line 168b according to any of the embodiments disclosed herein.

According to some embodiments, the delivery apparatus comprises a first sensor 180a retained within a nosecone 1126, and a sensing catheter 194 equipped with a sensing head 196, wherein the sensing head 196 comprises the second sensor 180b, and wherein the sensing head 196 may be positioned proximal to the prosthetic valve 140.

Reference is now made to Fig. 12. By way of example only, the use of a delivery assembly 100 equipped with a first pressure sensor 180a retained within a nosecone 1226, and a second pressure sensor 180b positioned proximal to a non-balloon expandable valve, for transvalvular pressure measurement, will be described with reference to a mechanically-expandable aortic valve 140' and with reference to the native aortic valve 40.

A delivery assembly 100 may be utilized according to conventional transcatheter valve replacement procedures, to advance the nosecone 1126 (illustrated more specifically as a nosecone 1226 in Fig. 12) over the guidewire 122 to a position distal to a native heart valve. For example, advancing the nosecone 1126 towards the left ventricle 16, to position it in the LVOT 22 as shown in Fig. 12. A non-balloon expandable aortic valve 140, such as a mechanically expandable valve 140', is positioned at the aortic annulus 42 such that the second sensor 180b is disposed within the aorta 80, for example within, or in the vicinity of, the aortic root 82.

In this position, the first 180a and the second 180b pressure sensors may measure, simultaneously, the pressures within the left ventricle 16 and the aorta 80. Thus, the signals acquired from both the first 180a and the second 180b pressure sensors can be used to calculate, and thereby provide the pressure difference between the left ventricle 16 and aorta 80, and determine the pressure drop across a non-balloon expandable aortic valve 140 prior to, during and/or after, expansion against the native aortic annulus 42. Such measurements may provide real-time feedback regarding the hemodynamic adequacy of valve expansion diameter and the valve positioning during an implantation procedure. Measurement results may be displayed graphically, for example on an LCD screen 1022 or LED lights 1124 provided on the handle 110.

The proposed assembly and method are primarily applicable for delivery assemblies 100 comprising non-balloon expandable prosthetic valves 140. Balloon expandable valves block the blood flow through the prosthetic valve during balloon inflation, therefore rendering utilization of pressure sensors positioned proximal and distal to the prosthetic valve, for pressure drop measurements during such a procedure, impractical. In contrast, non-balloon expandable valves, such as self-expandable valves or mechanically expandable valves, may be expanded without blocking blood flow there-through.

According to alternative embodiments, delivery assemblies 100 equipped with a first sensor 180a retained within a nosecone 1126, and a second sensor 180b positioned proximal to a prosthetic valve, may be utilized in conjunction with balloon expandable valves, for example to provide measurements of the pressure drop across an expanded valve once the balloon is deflated.

According to some embodiments, a method of utilizing the delivery assemblies 100 equipped with the first 180a and the second 180b pressure sensors described herein above includes a step of partially expanding the non-balloon expandable aortic valve 140, and deriving real-time pressure values during the expansion procedure, such that the non-balloon expandable aortic valve 140 can be recompressed and re-positioned, if required.

Delivery assemblies 100 equipped with the first 180a and the second 180b pressure sensors, further comprising a re-compression mechanism, can be advantageously utilized according to the proposed method, as the re-compression mechanism enables re-compressing a non-balloon expandable prosthetic valve 140 in order to re-orient or reposition it, if required, in light of real-time pressure measurements received from the first 180a and the second 180b pressure sensors.

Although shown in Fig. 12 in relation to a delivery system carrying a prosthetic aortic valve (illustrated as a mechanically expandable valve 140' in Fig. 12), the method can be similarly implemented using a delivery system carrying a prosthetic valve for implantation at other locations of the heart, such as within the native mitral valve, the native pulmonary valve, and the native tricuspid valve.

As illustrated in Fig. 12, pressure may be measured by at least the first pressure sensor 180a even when the guidewire 12 is still retained within the NC GW lumen 1234 of any nosecone 1226 (hidden from view in Fig. 12), since the active face 186a of the first pressure sensor 180a is oriented toward the blood flow surrounding the nosecone 1226, for example through the NC lateral port 1236.

The same method described above and illustrated in conjunction with Fig. 12 may be implemented for a first sensor 180a retained within a nosecone 1326, by performing a further step of retracting the guidewire 12 from the NC GW lumen 1334. The active face 186a of the first pressure sensor 180a may be oriented toward the NC GW lumen 1334, such that pressure reading cannot be performed as long as the guidewire 12 occupies the space of the NC GW lumen 1334. However, guidewire 12 retraction may enable blood flow through the NC GW lumen 1334, thereby enabling the first sensor 180a to measure blood pressure within the NC GW lumen 1334.

According to some embodiments, there is provided a system 200 comprising a delivery assembly 100 equipped with a first sensor 180a retained within a nosecone 1126, and a sensing catheter 294 provided with a sensing head 296.

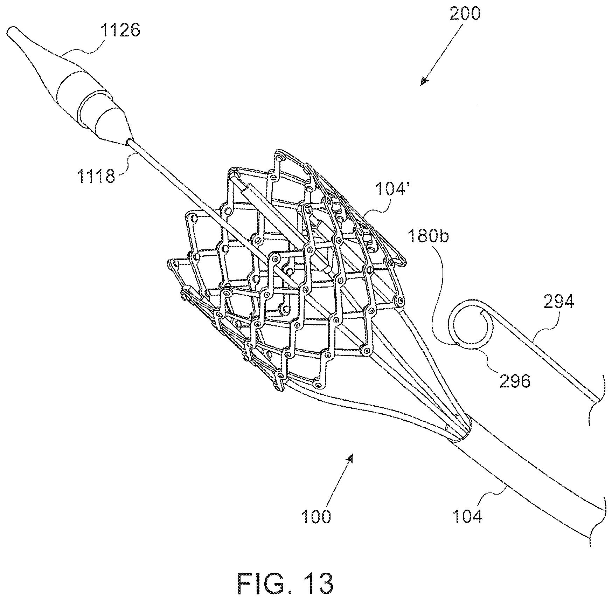

Fig. 13 shows a distal region of the system 200, comprising a delivery assembly 100 provided with a valve 140' and a first sensor 180a retained within a nosecone 1126 (first sensor 180a is hidden from view). While a mechanically expandable valve 140' is illustrated in Fig. 13, it will be clear that the configuration of this figure applies to other types of prosthetic valves 140 in a similar manner. The system 200 further includes a sensing catheter 294, which may be similar in structure and function to the sensing catheter 194, except that the sensing catheter 294 is provided as a separate component which is not part of the delivery apparatus 102.

The sensing catheter 294 may be axially movable relative to any component of the delivery assembly 100. The sensing catheter 294 comprises a sensing head 296, which may comprise a sensor, such as the second sensor 180b according to any of the embodiments disclosed herein. According to some embodiments, the sensing catheter 294 may be provided in the form of a pigtail catheter, as illustrated in Fig. 13.

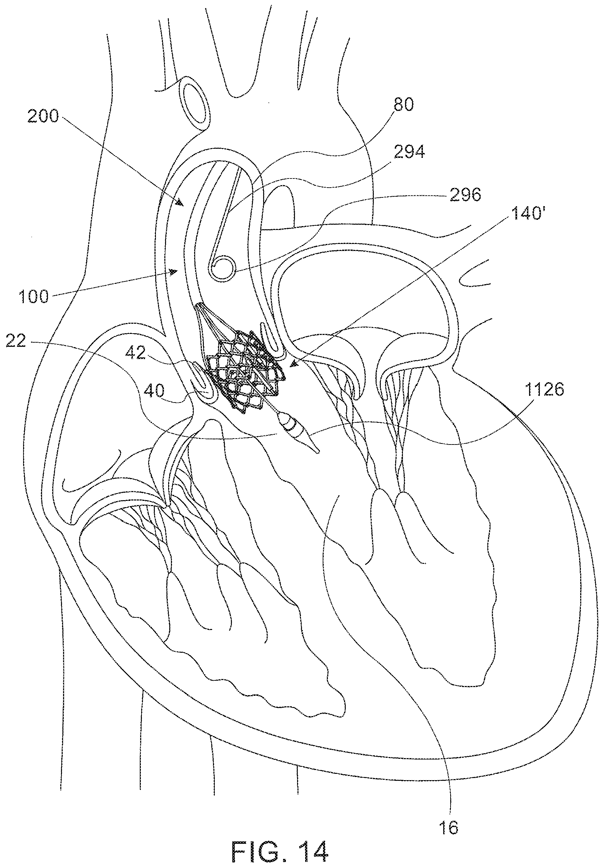

Reference is now made to Fig. 14. By way of example only, the use of a system 200 will be described with reference to a mechanically-expandable aortic valve 140' and with reference to the native aortic valve 40. A delivery assembly 100 may be utilized according to conventional transcatheter valve replacement procedures, to advance the nosecone 1126 toward the left ventricle 16, for example to position it in the LVOT 22 as shown in Fig. 14. A non-balloon expandable aortic valve 140, such as a mechanically expandable valve 140', is positioned at the aortic annulus 42 while a sensing catheter 294 is advanced through the aorta 80, to position the sensing head 296 proximal to the non-balloon expandable aortic valve 140.

In this position, the first sensor 180a and the sensing head 296 may measure, simultaneously, the pressures within the left ventricle 16 and the aorta 80. Thus, the signals from both the first sensor 180a and the sensing head 296 can be used to provide the pressure difference between the left ventricle 16 and aorta 80, and determine the pressure drop across a non-balloon expandable aortic valve 140 prior to, during and/or after, expansion against the native aortic annulus 42. Such measurements may provide real-time feedback regarding the hemodynamic adequacy of valve expansion diameter and the valve positioning during an implantation procedure. Measurement results may be displayed graphically, for example on an LCD screen 1022 or LED lights 1124 provided on the handle 110.

While the proposed assembly and method are primarily applicable for delivery assemblies 100 comprising non-balloon expandable prosthetic valves 140, they may be utilized in conjunction with balloon expandable valves as well, for example to provide measurements of the pressure drop across an expanded valve once the balloon is deflated.

Further steps of the method, including derivation of real-time pressure values during the expansion procedure, and/or guidewire 112 retraction, may be implemented in the same manner described above in conjunction with Fig. 12.

According to some embodiments, the delivery apparatus 102 comprises valved shaft extending from the handle 110, and defining a valve shaft lumen. The valved shaft comprises at least one sensor within the valved shaft lumen. The valved shaft further comprises a shaft valve which is movable between a closed position, blocking fluid flow through the valved shaft lumen, and an opened position, allowing fluid flow (e.g., blood flow) there-through.

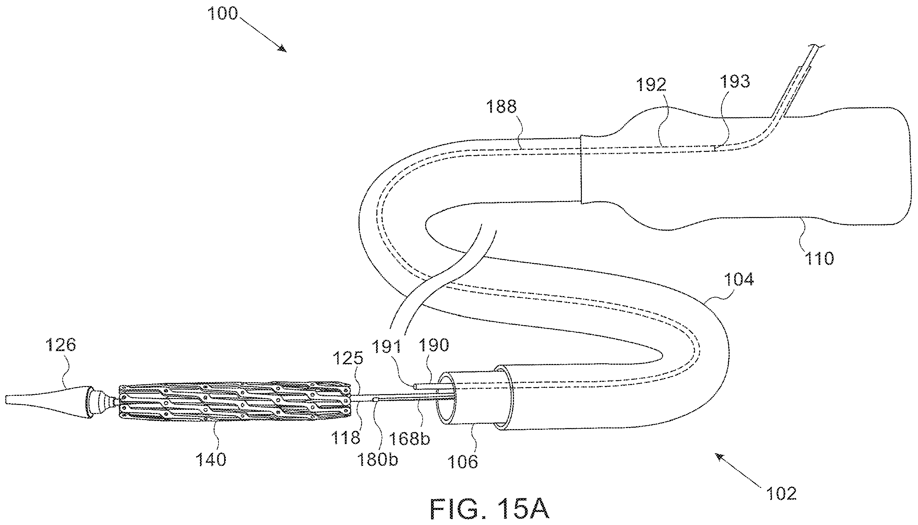

Figs. 15A-15B show a delivery assembly 100 comprising a valved shaft 188, in closed and open states of the shaft valve 193, respectively, according to some embodiments. Figs. 16A-16B show sectional side views of the valved shaft 188, corresponding to configurations of the valved shaft 188 in Figs. 15A-15B, respectively. The valved shaft 188 defines a valved shaft lumen 189, and comprises a valved shaft proximal portion 192, which may extend into the handle 110, and a valved shaft distal portion 190 terminating at a valved shaft distal end 191. The valved shaft 188 may extend from the handle 110 through the delivery shaft 106, and may be axially movable relative to the delivery shaft 106. The axial movement of the valved shaft 188 may be controlled by the handle 110.

According to some embodiments, the valved shaft 188 comprises the first sensor 180a attached thereto, disposed within the valved shaft lumen 189. In the exemplary embodiments shown in Figs. 15A-16B, the first sensor 180a is attached to the valved shaft inner surface 187. According to some embodiments, the first sensor 180a is attached to the inner surface of the valved shaft distal portion 190. The valved shaft 188 may further comprise a first transmission line 168a extending from the first sensor 180a toward the handle 110. According to some embodiments, the first transmission line 168a is attached to the valved shaft inner surface 187.

According to some embodiments, the delivery apparatus 102 comprises a first sensor 180a attached to the valved shaft distal portion 190, and a second sensor 180b positioned proximal to the prosthetic valve 140. While the second sensor 180b is shown in Figs. 15A-15B attached to the NC shaft outer surface 125 for illustrative purposes, it will be clear that the second sensor 180b can be position proximal to the prosthetic valve 140 in accordance to any of the configurations described and illustrated in conjunction with Figs. 9A-9H.

The valve shaft 188 further comprises a shaft valve coupled to the shaft proximal portion 192, schematically shown in Figs. 15A-16B as a leaf valve disposed within the valved shaft lumen 189. The shaft valve 193 can be any type of valve movable between an opened and a closed position, such as, but not limited to, a gate valve, a butterfly valve, a check valve, a ball valve and so on. The shaft valve 193 is configured to prevent flow through the valved shaft lumen 189 in the closed position, and to allow flow there-through in the opened position. The shaft valve 193 may be operated manually or electrically by a user of the delivery assembly 100, for example by maneuvering an appropriate actuation mechanism in the handle 110 (not shown). According to some embodiments, the valved shaft 188 comprises a continuous wall surrounding the valved shaft lumen 189, devoid of any cuts, opening or apertures extending radially outward from the valved shaft lumen 189.

Fig. 15A shows the prosthetic valve 140 carried in a crimped state by the delivery apparatus 102, prior to valve expansion. In this state, the shaft valve 193 is in a closed position, as shown in greater detail in Fig. 16A. The valved shaft distal portion 190 may be positioned proximal to the prosthetic valve 140 in this state, as illustrated in Fig. 15A, or in any other position relative thereto. As long as the shaft valve 193 remains in a closed position, blood is blocked from flowing through the valved shaft lumen 189.

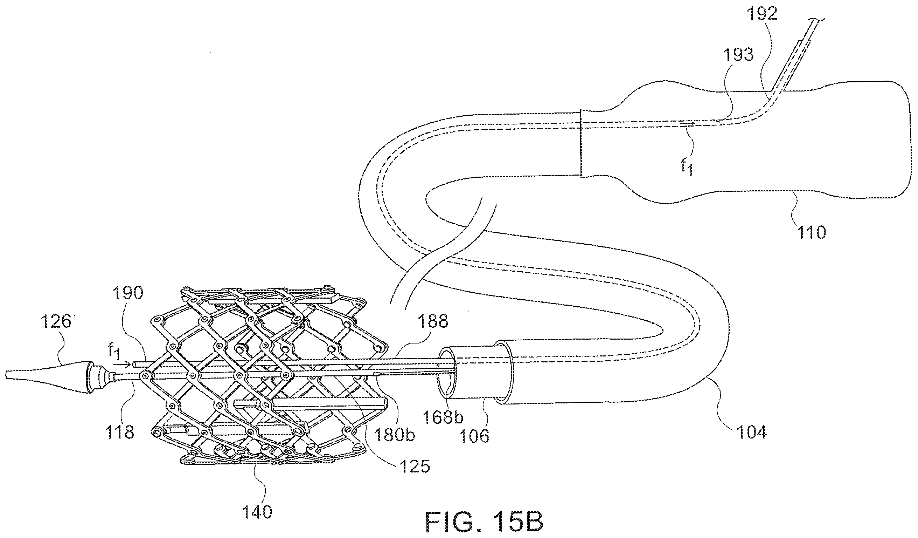

Fig. 15B shows the prosthetic valve 140 in an expanded state, which can be either partially or fully expanded against the native annulus, for example. In this state, the valve shaft 188 can be advanced distally through the prosthetic valve 140, to position the first sensor 180a at a position distal to the prosthetic valve 140, for example by advancing the valve shaft distal portion 190 distal to the prosthetic valve 140. At this position, the shaft valve 193 is moved to the opened position. For example, a shaft valve 193 hinged to the inner surface of the valved shaft proximal portion 192 may be pivoted around its hinge in the direction of arrow c1 in Fig. 16B. However, other types of valves implemented for the shaft valve 193 may be associated with different translation mechanisms from the closed to the opened position. In the opened position of the shaft valve 193, blood may flow through the valved shaft lumen 189 in the direction of arrows f1 in Figs. 15B and 16B. Once blood flow is allowed through the valved shaft lumen 189, pressure or flow may be reliably measured by the first sensor 180a.

Measurement signals can be transmitted from the first sensor 180a to the internal control unit 1010 via the first transmission line 168a. Figs. 16A-16B schematically show an exemplary internal control unit 1010 embedded within the handle 110, and operatively coupled with the display 1020 (such as a digital screen 1022 or LED lights 1024, shown in Fig. 2) and/or the proximal communication component 1030. While not explicitly illustrated in Figs. 16A-16B, it will be clear that measurement signals can be similarly transmitted from the second sensor to the internal control unit 1010 via the second transmission line 168b. Thus, the configuration shown in Figs. 15B and 16B enables derivation of pressure measurements proximal to the prosthetic valve 140 by the second sensor 180b, and distal to the prosthetic valve 140 by the first sensor 180a, when the prosthetic valve 140 is expanded and the shaft valve 193 is in an opened position.

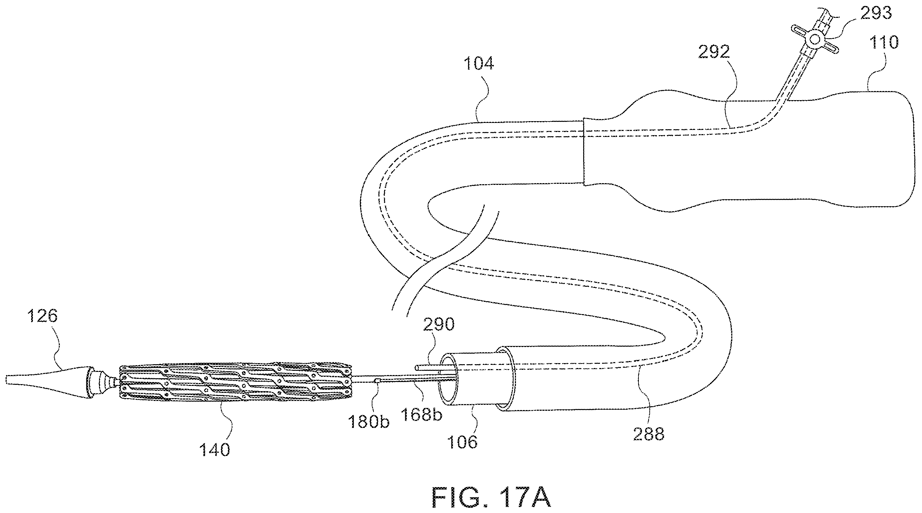

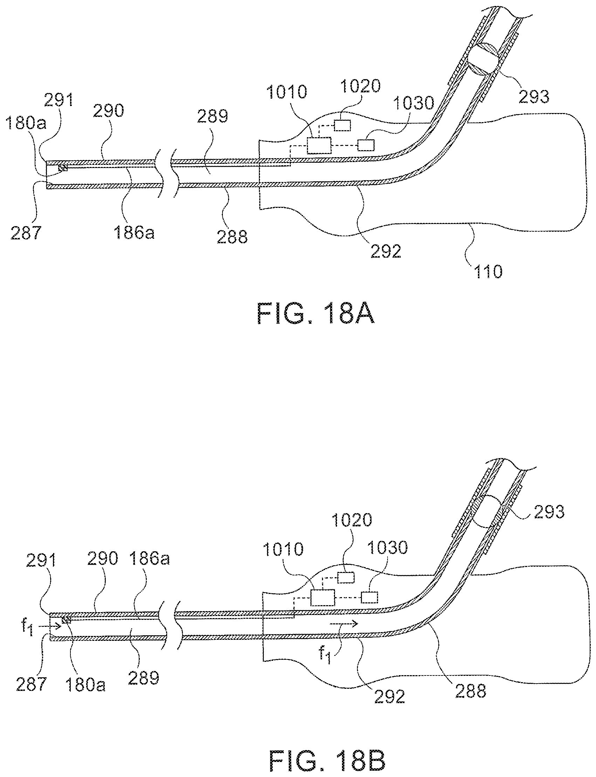

Figs. 17A-17B show a delivery assembly comprising a valved shaft 288, in closed and open states of the shaft valve 293, respectively, according to some embodiments. Figs. 18A-18B show sectional side views of the valved shaft 288 in the states corresponding to the states shown in Figs. 17A-17B, respectively. The valved shaft 288 is similar in structure and function to the valved shaft 188, except that the shaft valve is a stopcock valve 293, which can be switched between the closed position and the opened position. Other elements of the valved shaft 288 are essentially similar to the elements of the valved shaft 188, wherein like reference numerals refer to like parts throughout the figures, and thus will not be further described.

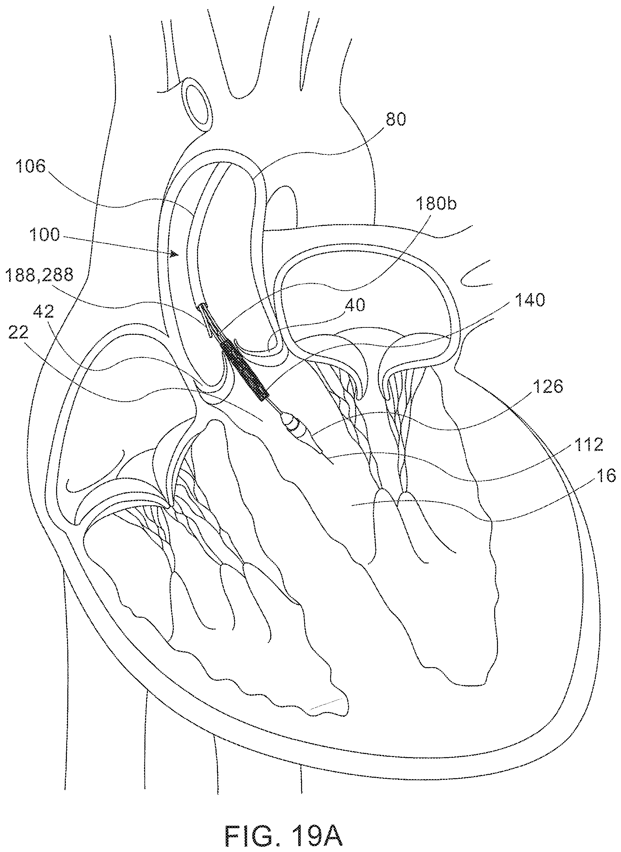

Reference is now made to Figs. 19A-19B. By way of example only, the use a delivery assembly 100 equipped with a first pressure sensor 180a retained within the lumen of a valved shaft 188, 288, and a second pressure sensor 180b positioned proximal to a prosthetic valve 140, for transvalvular pressure measurement, will be described with reference to a prosthetic aortic valve and with reference to a native aortic valve 40. A delivery assembly 100 may be utilized according to conventional transcatheter valve replacement procedures, to deliver the prosthetic valve toward a desired site of implantation, such as the native aortic valve 40. During the delivery procedure, and as long as the prosthetic valve 140 is in the crimped state shown in Fig. 19A, the valved shaft 188, 288 can be positioned such that its distal end is proximal to the prosthetic valve 140, and the shaft valve 193, 293 (not shown in Figs. 19A-19B) is in a closed position, as illustrated in Figs. 15A and 16A for the shaft valve 193, or in Figs. 17A and 18A for the shaft valve 293.

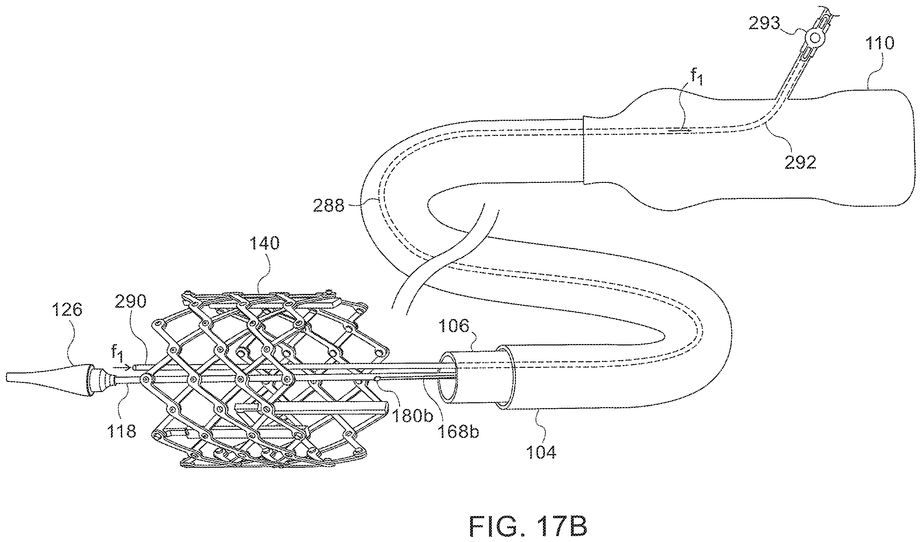

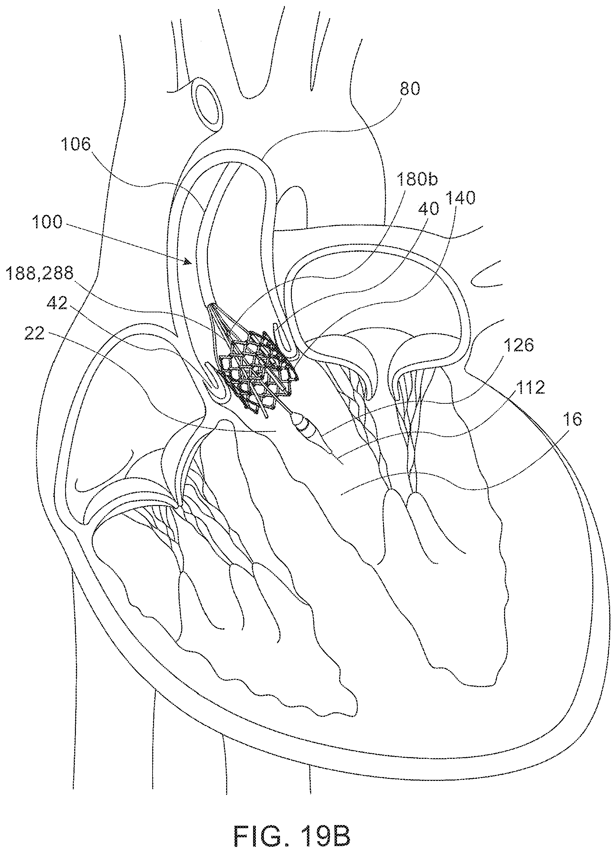

In Fig. 19B, the prosthetic valve 140, which can be a non-balloon expandable aortic valve, is expanded against the aortic annulus 42, allowing the valved shaft 188, 288 to be distally advanced there-through, to position the first sensor 180a distal to the prosthetic valve. As shown in Fig 19B, the valved shaft 188, 288 is advanced toward the left ventricle 16, for example to position the first sensor 180a (hidden from view) in the LVOT 22. In this state, the shaft valve 193, 293 is moved or switched to the opened position, allowing blood flow through the valved shaft 188, 288, as illustrated in Figs. 15B and 16B for the shaft valve 193, or in Figs. 17B and 18B for the shaft valve 293.

In this state, the first 180a and the second 180b pressure sensors may measure, simultaneously, the pressures within the left ventricle 16 and the aorta 80. Thus, the signals acquired from both the first 180a and the second 180b pressure sensors can be used to calculate, thereby provide the pressure difference between the left ventricle 16 and aorta 80, and determine the pressure drop across a non-balloon expandable aortic valve 140 prior to, during and/or after, expansion against the native aortic annulus 42. Such measurements may provide real-time feedback regarding the hemodynamic adequacy of valve expansion diameter and the valve positioning during an implantation procedure. Measurement results may be displayed graphically, for example on an LCD screen 1022 or LED lights 1124 provided on the handle 110.

While the proposed assembly and method are primarily applicable for delivery assemblies 100 comprising non-balloon expandable prosthetic valves 140, they may be utilized in conjunction with balloon expandable valves as well, for example to provide measurements of the pressure drop across an expanded valve once the balloon is deflated. Further steps of the method, including derivation of real-time pressure values during the expansion procedure, may be implemented in the same manner described above in conjunction with Fig. 12.

While not explicitly shown, other embodiments of a valved shaft, such as the valved shaft 188 or 288, which includes a second sensor 180b, optionally connected to a second transmission line 168b extending there-from toward the handle 110, are contemplated. The attachment of the second sensor 180b can be implemented according to any of the configuration described and illustrated for the first sensor 180a in conjunction with Figs. 15A-18B. A valved shaft comprising a second sensor 180b within the valved shaft lumen, can be used in conjunction with a delivery apparatus 102 equipped with a first sensor 180a retained within a nosecone 1126. In such cases, the valved shaft is not advanced through the prosthetic valve 140 upon expansion thereof, but rather remains proximal to the prosthetic valve 140 so as to keep the second sensor 180b positioned proximal to the prosthetic valve 140. When the prosthetic valve 140 is expanded, the shaft valve may be switched to the opened position, allowing the first 180a and the second 180b sensors, which can be pressure sensors, to simultaneously measure the pressure distal to and proximal to, the prosthetic valve 140, respectively.

According to some embodiments, the delivery apparatus 102 comprises valved guidewire (also termed herein a valved GW) 212 extending from the handle 110 through the NC shaft GW lumen 122 and the NC GW lumen 134, and defining a guidewire internal lumen (also termed herein a GW internal lumen) 213. The valved GW comprises at least one sensor within the GW internal lumen 213. The valved GW 212 further comprises a guidewire valve (also termed herein a GW valve) 217 coupled thereto, which is movable between a closed position, blocking fluid flow through the GW internal lumen 213, and an opened position, allowing fluid flow (e.g., blood flow) there-through.

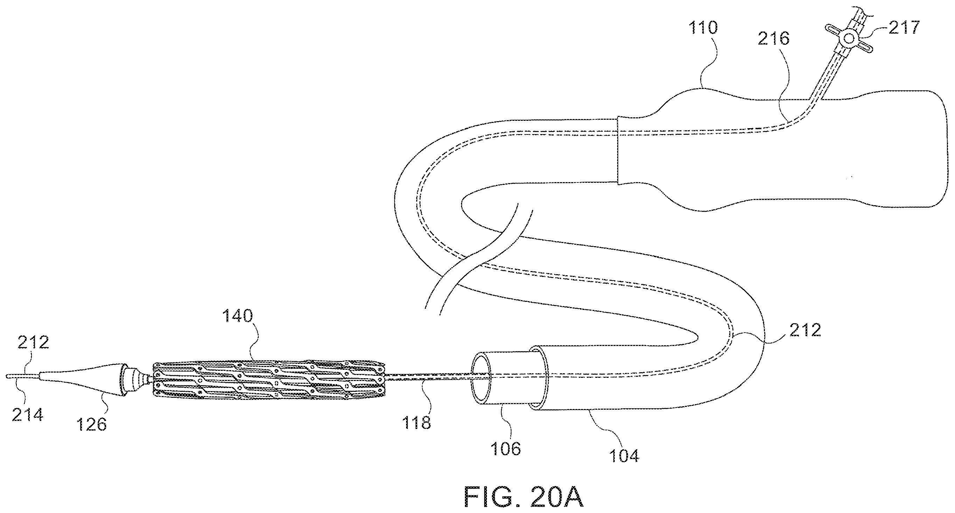

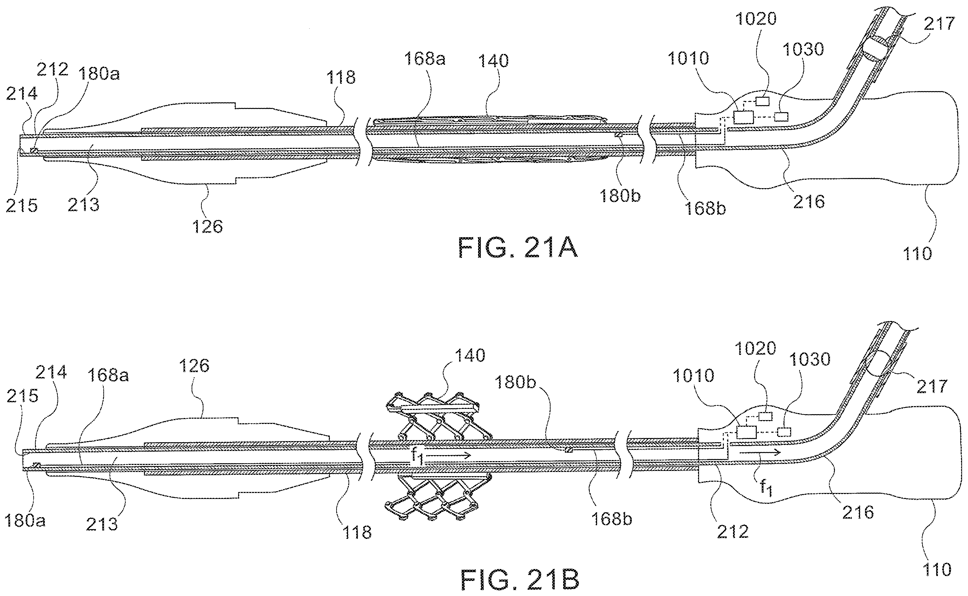

Figs. 20A-20B show a delivery assembly 100 comprising a valved GW 212, in closed and open states of the GW valve 217, respectively, according to some embodiments. Figs. 21A-21B show sectional side views of the valved GW 212, corresponding to the states of Figs. 20A-20B, respectively. The valved GW 212 comprises a valved guidewire proximal portion (also termed herein a valved GW proximal portion) 216, which may extend into the handle 110, and a valved guidewire distal portion (also termed herein a valved GW distal portion), which may extend through and/or distal to the nosecone 126. According to some embodiments, the GW valve 217 is coupled to the valved GW proximal portion 216.

According to some embodiments, the valved GW 212 comprises at least two sensors, axially spaced from each other within the GW internal lumen 213. A valve guidewire inner surface (also termed herein a valved GW inner surface) 215 is defined around the GW internal lumen 213. According to some embodiments, the valved GW 212 comprises the sensor 180a attached to valved GW inner surface 215 at a position distal to the prosthetic valve 140, and a second sensor 180b attached to the valved GW inner surface 215 at a position proximal to the prosthetic valve 140. According to some embodiments, the GW 212 further comprises a first transmission line 168a attached to the first sensor 180a and extending toward the handle 110, and a second transmission line 168b attached to the second sensor 180b and extending toward the handle 110. According to some embodiments, the first transmission line 168a and/or the second transmission line 168b may be attached to the GW inner surface 215. According to some embodiments, the valved GW 212 comprises a continuous GW inner surface 215, devoid of any cuts, opening or apertures extending radially outward from the GW internal lumen 213 through the GW inner surface 215.

The valved GW proximal portion 216 comprises a GW valve 217, shown in Figs. 20A-21B as a stopcock valve. The GW valve 217 can be any other type of valve movable between the opened and closed positions, such as, but not limited to, a gate valve, a butterfly valve, a check valve, a ball valve and so on. The GW valve 217 is configured to prevent flow through the GW internal lumen 213in the closed position, and to allow flow there-through in the opened position. The GW valve 217 may be operated manually or electrically by a user of the delivery assembly 100, for example by maneuvering an appropriate actuation mechanism in the handle 110.

Fig. 20A shows the prosthetic valve 140 carried in a crimped state by the delivery apparatus 102, prior to valve expansion. In this state, the GW valve 217 is in a closed position, as shown in greater detail in Fig. 21A. As long as the GW valve 217 remains in a closed position, blood is blocked from flowing through the valved GW internal lumen 213.

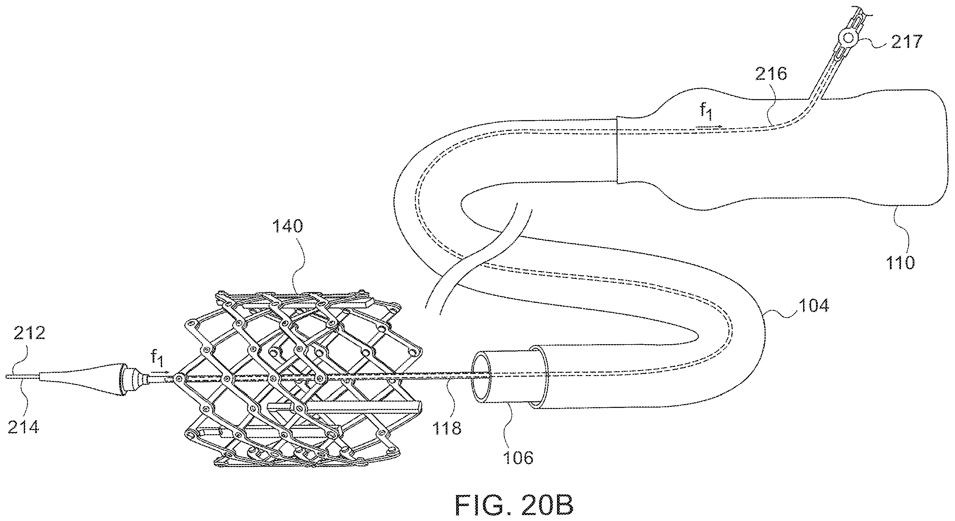

Fig. 20B shows the prosthetic valve 140 in an expanded state, representing a state in which transvalvular pressure measurement may be desirable. At this stage, the GW valve 217 is moved or switched to the opened position, enabling blood flow through the valved GW internal lumen 213 in the direction of arrows f₁ in Figs. 20B and 21B. Once blood flow is allowed through the valved GW internal lumen 213, pressure or flow may be reliably measured by the first sensor 180a and/or the second sensor 180b. Measurement signals can be transmitted from the first sensor 180a and/or the second sensor 180b, to the internal control unit 1010, via the first transmission line 168a and/or the second transmission line 168b.

While not explicitly shown, further embodiments of a valved shaft, such as the valved shaft 188 or 288, which includes both a first sensor 180a and a second sensor 180b within its lumen, are contemplated. The first and second sensors 180a and 180b, respectively, can be attached to the valved shaft in a similar manner to that disclosed for a valve GW 212, i.e. both attached to the valved shaft 188, 288, and disposed within the valved shaft lumen 189, 289 such that the first sensor 180a is attached to the valved shaft distal portion 190, 290, and the second sensor 180b is proximally distanced from the first sensor 180a.

A valved shaft with both the first and second sensors 180a and 180b, respectively, can be used according to any of the methods described for the valved shaft 188 or 288, wherein such a valved shaft may be advanced through the expanded prosthetic valve 140 to a position such that the first sensor 180 is distal to the prosthetic valve 140, while the second sensor 180b is proximal to the prosthetic valve 140.

According to some embodiments, the delivery assembly 100 comprises at least one sensor 380, and preferably a plurality of sensors 380, attached to the prosthetic valve 140. A sensor 380 is adapted to measure a physiological parameter such as blood pressure, blood flow velocity, temperature, distance to a tissue, deposits accumulation and/or electric conductivity, and to generate a signal representative of the physiological parameter. The at least one sensor 380 can be attached to the inflow end portion 144, to the prosthetic valve outflow end portion 142, or to any other region in between. The at least one sensor 380 can be attached to the frame 146, to commissures 154, to actuator assemblies 156 or to any other structural component of the prosthetic valve 140. According to some embodiments, the at least one sensor 380 may be attached to the prosthetic valve 140 by suturing, screwing, clamping, gluing with bio-compatible adhesives, fastening, welding, or any other suitable technique.

The at least one sensor 380 can be oriented radially inward (i.e., toward the valve longitudinal axis 141), to measure one or more types of physiological parameters within the prosthetic valve 140, or oriented radially outward, to measure one or more types of physiological data outside of, or in contact with, the outer surface of the prosthetic valve 140.

According to some embodiments, the prosthetic valve 140 comprises a first sensor 380a, attached to the inflow end portion 144, and a second sensor 380b, attached to the outflow end portion 142. Each of the first sensor 380a and the second sensor 380b is configured to measure a physiological flow-related property, such as blood pressure and/or blood flow. According to some embodiments, the first sensor 380a and the second sensor 380b are pressure sensors. According to some embodiments, the first sensor 380a and the second sensor 380b are flow sensors.

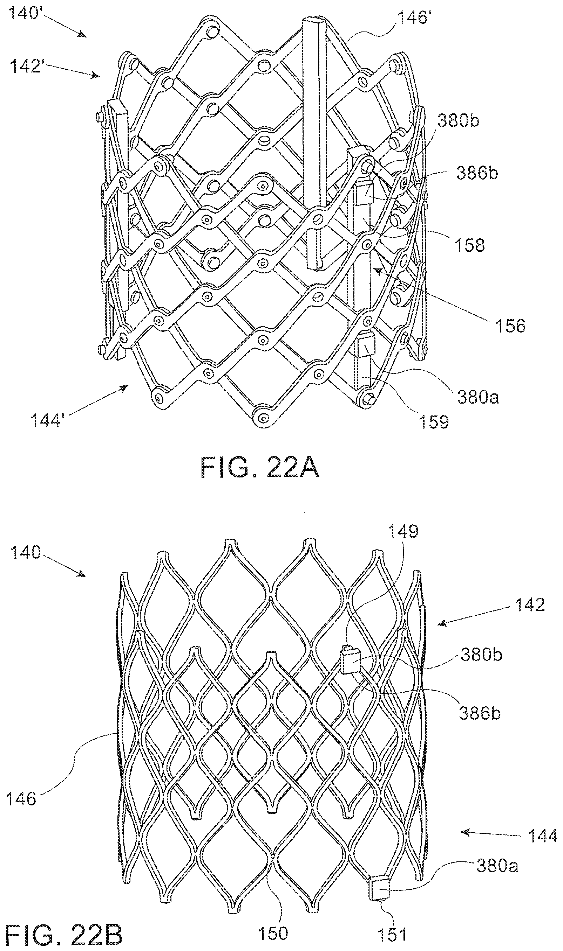

Reference is now made to Figs. 22A-22B, illustrating a first sensor 380a and a second sensor 380b attached to a prosthetic valve. Fig. 22A shows an exemplary embodiment of a first sensor 380a and a second sensor 380b attached to a mechanically-expandable valve 140', and more specifically, attached to at least one actuator assembly 156 of the prosthetic valve 140'. In the illustrated example, both the first sensor 380a and a second sensor 380b are axially spaced apart, attached to the same outer member 158. Alternatively, or additionally, each of the first 380a and/or the second 380b sensors can be attached to other components of the actuator assembly 156, such as the inner member 159, attached to different actuator assemblies 156, or attached to any other component of the prosthetic valve 140'.

Fig. 22B shows an exemplary embodiment of a first sensor 380a and a second sensor 380b attached to the frame 146 of a prosthetic valve 140, and more specifically, attached to junctions 150 of the prosthetic valve 140. In the illustrated example, the first sensor 380a and a second sensor 380b are axially spaced apart, attached to an inflow apex 151 and an outflow apex 149, respectively. Alternatively, or additionally, each of the first 380a and/or the second 380b sensors can be attached to other junctions 150 or to any other component of the prosthetic valve 140.

According to some embodiments, a sensor 380 comprises an active face 386 and a passive face 387. For example, the first sensor 380a comprises a first active face 386a, defined as the side or surface of the first sensor 380a directed at the measurement region, and a first passive face 387a, which can be the side or surface of the first sensor 380a attached to a component of the prosthetic valve 140. The second sensor 380b similarly comprises a second active face 386b, and a second passive face 387b, which can be the side or surface attached to a component of the prosthetic valve 140.

The passive face 387 can be opposite to the active face 386, or any other face, for example a face orthogonal to the active face 386. In the exemplary embodiment illustrated in Fig. 22A, the second active face 386b is the face oriented radially outward from the frame 146'. In the exemplary embodiment illustrated in Fig. 22A, the second active face 386b is the face oriented distally toward the inflow end portion 144'.

According to some embodiments, any of the first and second sensors 380a and 380b, respectively, may be piezo-resistive pressure sensors, such as MEMS piezo-resistive pressure sensors. According to other embodiments, any of the first and second sensors 380a and 380b, respectively, may be capacitive pressure sensors, such as MEMS capacitive pressure sensors.

According to some embodiments, each sensor 380 is coupled to a transmission line 368, extending proximally there-from toward the handle 110. For example, the first sensor 380a may be coupled to a first transmission line 368a, and the second sensor 380b may be coupled to a second transmission line 368b. According to some embodiments, the transmission line 368 is attached to a component of the delivery apparatus 102. According to some embodiments, the transmission line 368 comprises an electrically conductive medium, such as one or more electrical conductive wires.

According to some embodiments, the transmission line 368 is configured to deliver power to the sensor 380. According to some embodiments, the transmission line 368 is connected to a proximal power source, for example within the handle 110, configured to provide power to operate the first sensor 380a. According to some embodiments, the transmission line 368 is configured to deliver signals from, and/or to, the sensor 380. According to some embodiments, the transmission line 368 is connected to the internal control unit 1010. According to some embodiments, the transmission line 368 is connected, directly or indirectly (e.g., via the internal control unit 1010) to the proximal communication component 1030.

According to some embodiments, the transmission line 368 is releasably coupled to the sensor 380. In such embodiments, the transmission line 368 may be coupled to the sensor 380 during prosthetic valve 140 delivery to the implantation site, and during the implantation procedure, and may be decoupled or released from the sensor 380 after the implantation procedure is completed, allowing the transmission line 368 to be retracted along with the remainder of the delivery apparatus 102 from the patient's body. In such embodiments, the prosthetic valve 140 may remain implanted in the patient's body, having the at least one sensor 380 attached thereto in a non-operative mode.

According to some embodiments, the sensor 380 is retained within a sensor housing 382, such that its active face 386 is directed at the desired measurement region. In such embodiments, a sensor 380 is coupled to the prosthetic valve 140 via the sensor housing 382. According to some embodiments, the sensor 380 is attached to the sensor housing 382 via its passive face 386, while the housing is coupled to the prosthetic valve 140. In such embodiments, the transmission line 368 extends through a lumen of a transmission line shaft 376, wherein the transmission line shaft 376 is releasably coupled to the sensor housing 382. The transmission line 368 further extends into the sensor housing 382, and is releasably coupled to the sensor 380. The transmission line shaft 376 is configured to isolate the transmission line 368 extending there-through, and the sensor 380 attached to the transmission line 368, from the ambient flow (e.g. blood flow), when the transmission line shaft 376 is coupled to the sensor housing 382.

The transmission line shaft 376 can extend from the handle 110 through the delivery shaft 106. According to some embodiments, the transmission line shaft 376 is axially movable relative to the prosthetic valve 140. According to some embodiments, the transmission line shaft 376 is axially movable relative to the delivery shaft 106. The transmission line 368 extends from the handle 100 through the corresponding transmission line shaft 376, and is axially movable relative to the transmission line shaft 376 when released from the corresponding sensor 380.

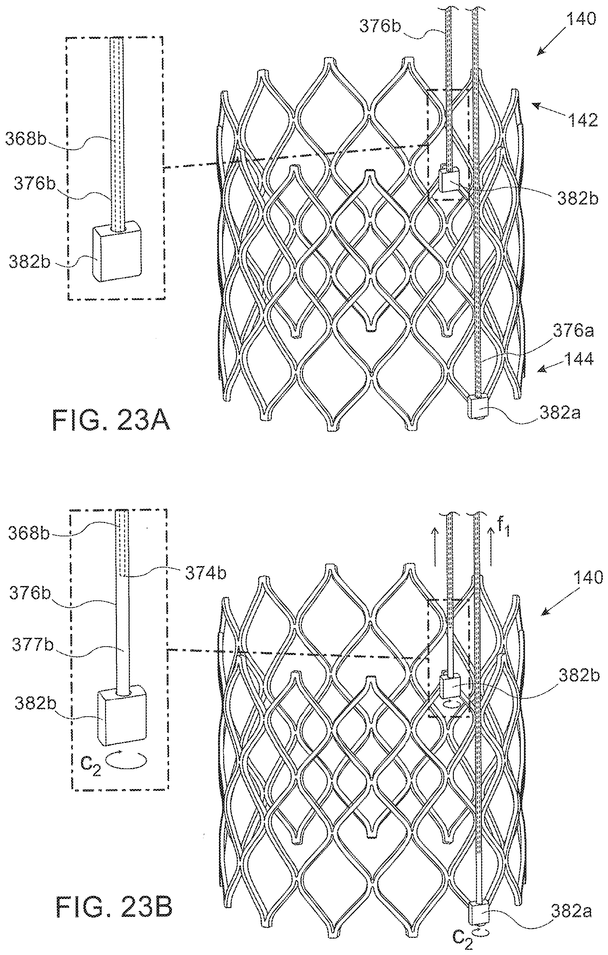

Figs. 23A-23C illustrate a non-binding configuration representing detachable coupling mechanism between a transmission line 368 extending through a transmission shaft lumen 377, and a sensor 380 retained within a sensor housing 382. The sensors 380a and 380b are hidden from view within the corresponding sensor housing 382a and 382b, respectively, in Figs. 23A-23C. Fig. 23A shows a first sensor housing 382a attached to the inflow end portion 144, and a second sensor 382b attached to the outflow end portion 142.

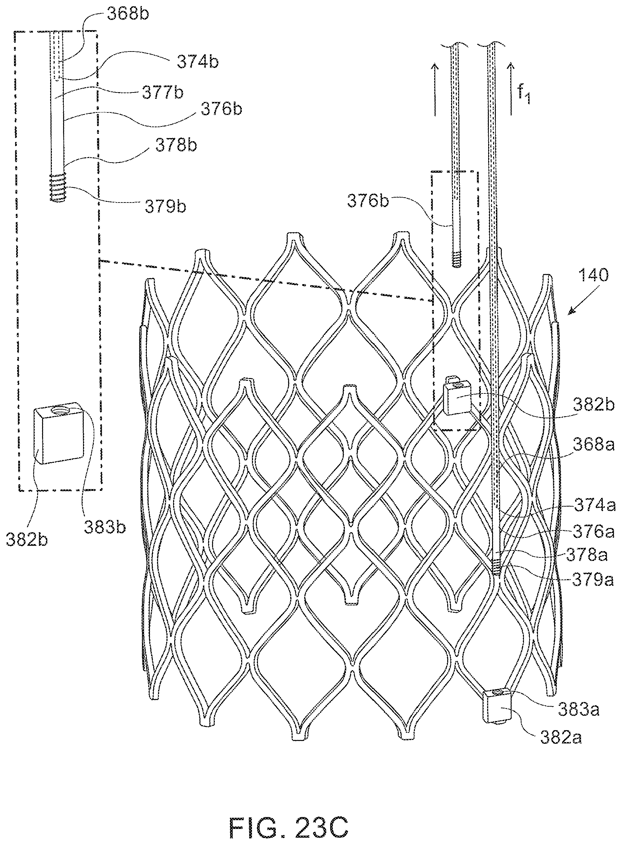

According to some embodiments, a transmission line 186 comprises a transmission line distal end 174, releasably coupled to the sensor 380. Similarly, a transmission line shaft 176 comprises a transmission shaft distal end 378 (see Fig. 23C), releasably coupled to the sensor housing 382. According to some embodiments, the sensor housing 382 comprises a housing threaded bore 383 (see Fig. 23C), and the transmission shaft distal end 378 comprises a transmission shaft external threading 379, configured to threadedly engage with the housing threaded bore 383.

In the state shown in Fig. 23A, the first 374a and second 374b transmission line distal ends are coupled to the first 380a and second 380b sensors, respectively, and the first 378a and second 378b transmission shaft distal ends are coupled to (e.g., threaded with) the first 383a and second 383b sensor housing bores, respectively. In this state, power may be supplied to the sensor 380a and 380b via the transmission lines 368a and 368b, respectively, and signals may be transmitted from and to the sensors 380a and 380b via the transmission lines 368a and 368b, respectively.

Fig. 23B shows a state during disengaging the transmission lines 368a and 368b from the sensors 380a and 380b, respectively. According to some embodiments, the transmission line 368 may be coupled to the sensor 380 such that application of a pull force in the direction f₁, beyond a predetermined threshold magnitude, may disengage the transmission line 368 from the sensor 380. According to some embodiments, the force required to disengage the transmission line 368 from the sensor 380 may be applied manually. According to some embodiments, the force required to disengage the transmission line 368 from the sensor 380 may be applied by a mechanical or electrical actuation mechanism at the handle 110.

As shown in Fig. 23B, while the transmission line 368 is decoupled from the sensor 380, the transmission line shaft 376 remains coupled to the sensor housing 382, thereby isolating the transmission line 368 from the surrounding environment of the blood flow. This allows the transmission line 368 to be decoupled and pulled from the sensor 380 while avoiding the risk of exposing the surrounding blood flow or other tissues to electrical current thereof.

Once the transmission line 368 is decoupled from the sensor 380 and pulled away therefrom, the transmission line shaft 376 may be rotated, for example in a direction c₂ around its axis of symmetry, so as to decouple from the sensor housing 382. According to some embodiments, the transmission line 368 is pulled along a sufficient distance prior to disengaging the transmission line shaft 376 from the sensor housing 382, such that once the transmission line shaft 376 is disengaged, the transmission line 368 cannot be exposed to the blood flow flowing through the transmission shaft lumen 377.

Fig. 23C shows a more advanced state of disengaging the transmission line 368b from the sensor 380b, compared to the state shown in Fig. 23B. The state shown in Fig. 23C is achieved by further pulling the transmission line shaft 376 in a proximal direction f₁, away from the sensor housing 382 after being disengaged therefrom. This mechanism allows the transmission line 368, along with the transmission line shaft 376, to be disengaged from the sensor 380 and sensor housing 382, and retracted from the patient's body at the end of the implantation procedure, without risking exposure of the native tissues or blood flow to electrical current flowing through the transmission line 368 during such disengagement.

A delivery assembly 100 comprising a first pressure sensor 380a and a second pressure sensor 380b attached to the inflow end portion 144 and the outflow end portion 142, respectively, of a prosthetic valve 140, may be utilized to provide pressure readings across the prosthetic valve 140 during an implantation procedure in the same manner described above for any of the configurations provided with a first sensor 180a and a second sensor 180b attached to components of the delivery apparatus 102.

Alternatively, or in addition to, the releasable coupling between sensor 380 and transmission lines 362, a prosthetic valve 140 may be coupled to at least one post-procedural sensor 380. A post-procedural sensor 380 is defined as a sensor configured to measure a physiological parameter, inter alia, after prosthetic valve implantation, without being wired to any component of the delivery apparatus. According to some embodiments, a post-procedural sensor 380 may be releasably coupled to a transmission line 362, through which it may receive power from a power source within the handle 110, and communicate with an internal control circuit 1110 and/or a proximal communication component 1130 during the implantation procedure, and include additional components enabling the post-procedural sensor 380 to operate once detached from the transmission line 362. Alternatively, a post-procedural sensor 380 may be configured to operate either during and/or after the implantation procedure, without being connected to a transmission line 362 or any other external power source.

According to some embodiments, the post-procedural sensor 380 comprises, or is coupled to, a transmitter (not shown), configured to wirelessly transmit signals, e.g. measurement signals, acquired by the post-procedural sensor 380. According to some embodiments, the prosthetic valve 140 comprises at least one transmitter coupled to at least one post-procedural sensor 380. According to some embodiments, the post-procedural sensor 380 can be electromagnetically coupled to a transmitting/receiving antenna (not shown).

Advantageously, post-procedural sensor 380 configured to acquire and transmit post-procedural measurement signals, enable post-procedural monitoring. For example, prosthetic valve performance may be monitored to detect deterioration over time, due to reduced motility of the leaflets 152, which may be caused by leaflet thrombosis, leaflet calcification and/or any other deposits formed thereon.

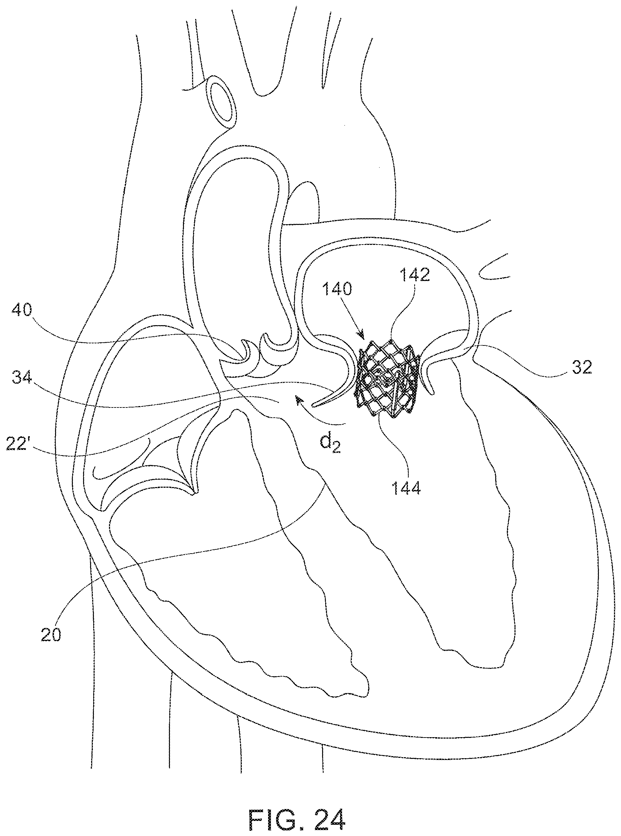

Reference is now made to Figs. 24-26B, illustrating exemplary flow disturbances that may occur during prosthetic mitral valve 140 implantation. The unique anatomical position of the native mitral valve 30 close to the LVOT 22 requires careful prosthetic valve positioning, so as to avoid hemodynamic disturbances in the LVOT 22, for example.

Fig. 24 shows a prosthetic mitral valve 140, which may take the form of any of the prosthetic valve 140, including a mechanically expandable valve 140', described hereinabove. The prosthetic mitral valve 140 shown in Fig. 24 is implanted within the mitral annulus 32. In some instances, as demonstrated in Fig. 24, placement of the inflow end portion 144 within the left ventricle 16 may form a neo-LVOT 22' region, which might be narrower than the anatomic LVOT 22 (shown in Fig. 1) and create an undesired stenotic region that disturbs hemodynamic behavior in this region. A neo-LVOT 22' may be formed, for example, when the inflow end portion 144 is oriented toward the lower septum 20, pushing the native mitral leaflet 34 in the same direction, thereby narrowing the LVOT 22' and constricting blood flow in the direction of arrow d₂ toward the aortic valve 40.

It is therefore desirable to provide real-time hemodynamic measurements, such as flow and/or pressure measurements, during the prosthetic valve 140 implantation procedure. Advantageously, real-time detection of flow disturbances in areas of interest, such as the LVOT 22, can be followed by corrective actions, such as, but not limited to: repositioning of the prosthetic valve 140, reorienting the valve's angle relative to the LVOT 22, or recompressing the prosthetic valve 140 (as long as such maneuvers are mechanically feasible, for example via re-compression mechanisms), in order to prevent or reduce, for example, interference with the LVOT 22.

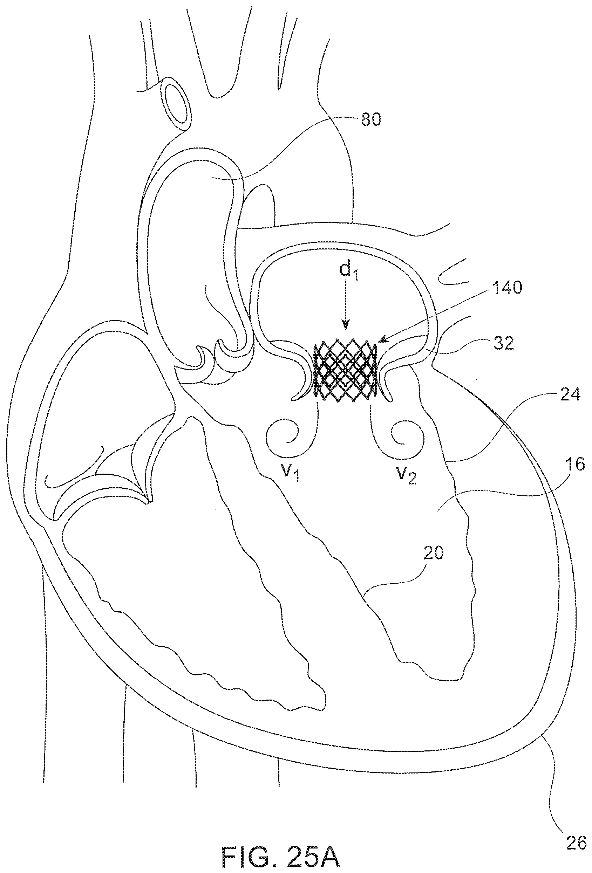

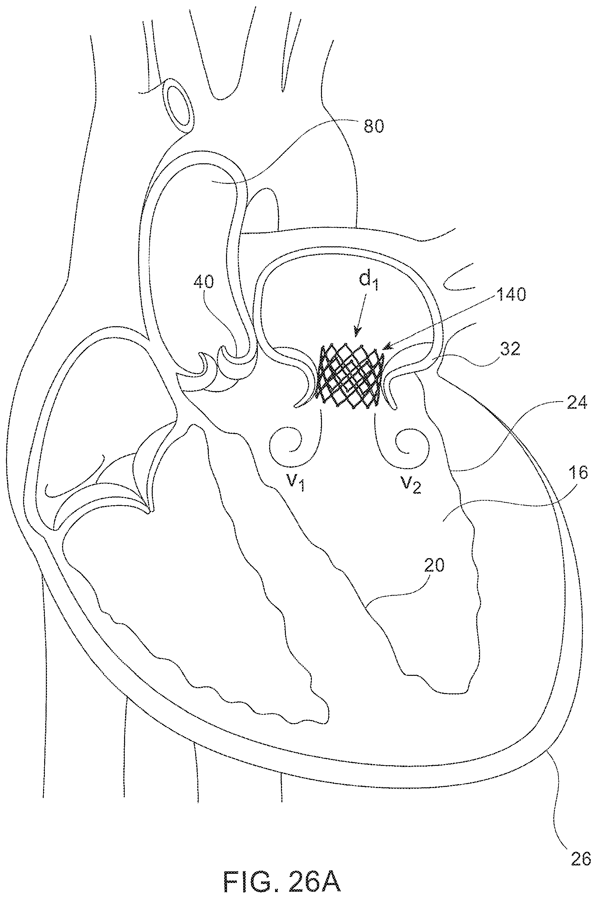

Figs. 25A-25B constitute sectional views of a prosthetic mitral valve 140 implanted within the mitral annulus 32 such that the mitral inflow d₁ is directed toward the heart's apex 26, and Figs. 26A-26B constitute sectional views of a prosthetic mitral valve 140 implanted within the mitral annulus 32 such that the mitral inflow d₁ is directed toward the lower septum 20.

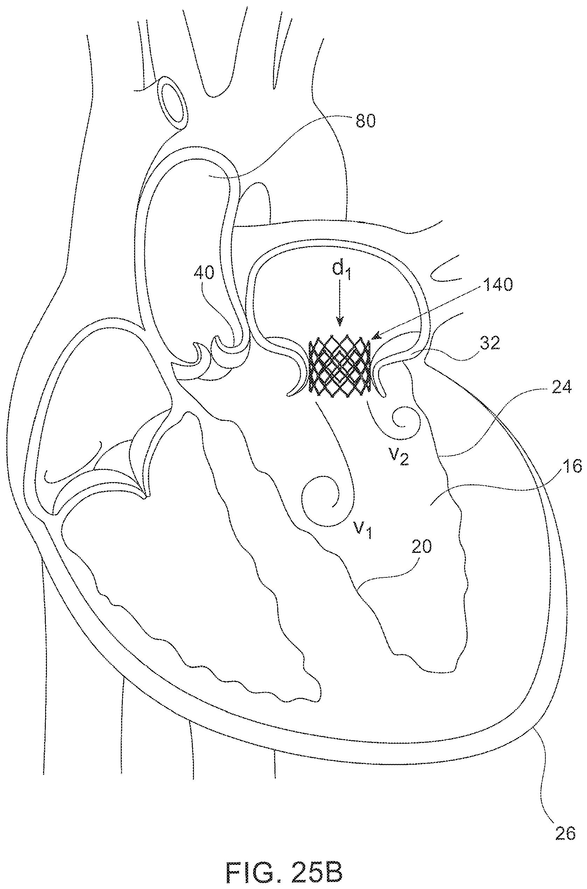

Fig. 25A shows two vortex rings: a first vortex ring v₁ facing the septal wall 20 of the left ventricle 16, and a second vortex ring v₂ formed next to the free wall 24 of the left ventricle 16. As diastole progresses and the left ventricle fills in (see Fig. 25B), the first vortex ring v₁ grows asymmetrically, capturing the momentum transfer that guides the blood flow toward the native aortic valve 40 in concert with left ventricle systole. The vortex structures dissipate as blood is ejected into the aorta 80 and are reformed during the next cardiac cycle.

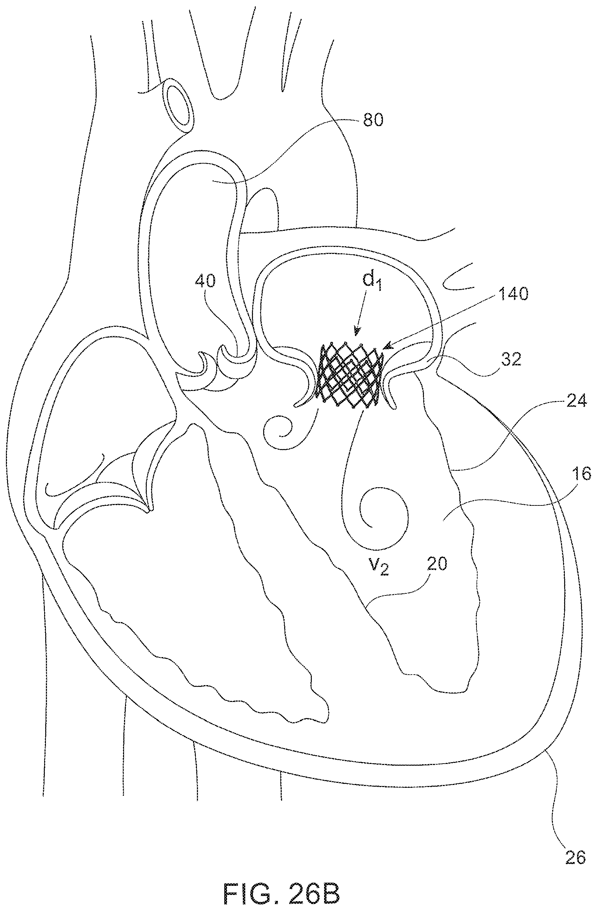

Fig. 26A shows vortex rings v₁, v₂ formed at the onset of diastole, but in this case, as shown in Fig. 26B, the vortex v₂ (opposite the LVOT 22) grows and redirects the blood away from the native aortic valve 40. During systole, the blood flow must cross the incoming vortex path v₁ at the LVOT 22 in order to exit through the aortic valve 40.

Since vortex structures v₁, v₂ are dependent on prosthetic mitral valve 140 orientation and position, hemodynamic parameters, such as, fluid flow or pressure should be monitored during prosthetic mitral valve 140 positioning, to provide a clinician with real-time feedback regarding valve mounting configurations. A mounting configuration of a prosthetic valve 140 refers to a set of positioning parameters, such as the depth of prosthetic valve protrusion into the left ventricle 16 and its angle relative to the plane of the mitral annulus 32.

According to some embodiments, there is provided a delivery assembly equipped with a prosthetic mitral valve 140, and a nosecone 1226 comprising a first sensor 180a retained therein, wherein the first sensor 180a is a Doppler sensor.

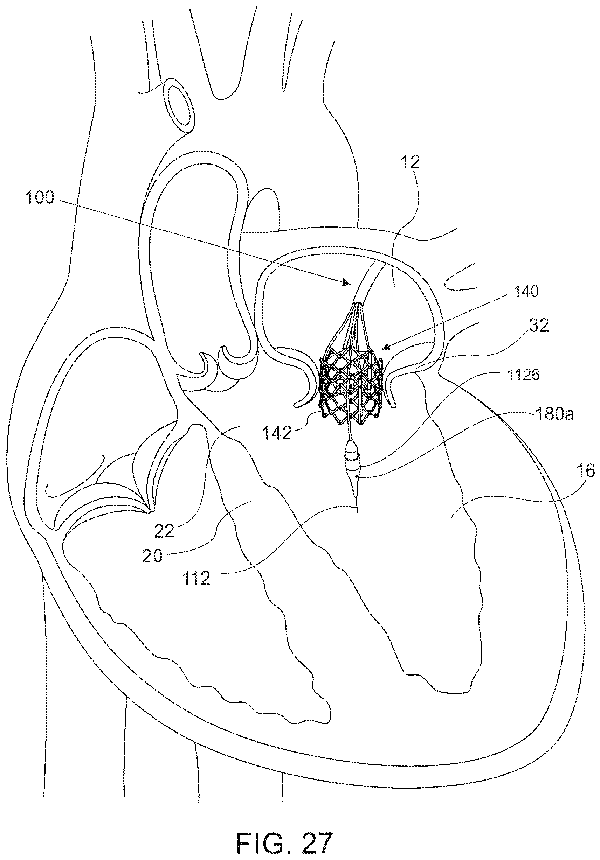

Reference is now made to Fig. 27, illustrating an embodiment of a delivery apparatus 102 equipped with a Doppler sensor 180a retained within a nosecone 1126, carrying a prosthetic mitral valve 140 for mounting against the native mitral annulus 32. A delivery assembly 100 may be utilized according to conventional transcatheter valve replacement procedures, to advance the prosthetic valve 140 toward the mitral annulus 32 for mitral valve replacement. During such a procedure, the nosecone 1126, equipped with a Doppler sensor 180a retained therein, can be advanced toward the left ventricle 16, as shown in Fig. 27.

The Doppler sensor 180a transmits ultrasonic waves, and receives reflected ultrasonic waves or echoes. The frequency or pitch of the signal is proportional to the blood velocity, and distinctive tonal patterns are produced. The tonal patterns are indicative of the flow patterns in term of time-varying velocity. Specifically, determination of the Doppler shift of the echoes provides means to detect and assess blood flow, and thereby to obtain information regarding the position and/or orientation of the prosthetic mitral valve 140. According to some embodiments, the Doppler sensor 180a comprises a piezoelectric crystal (not shown), which transmits and receives the ultrasound signals.

According to some embodiments, the nosecone 1126 can be oriented so as to direct the Doppler sensor 180a to measure flow in the LVOT 22. According to some embodiments, the nosecone 1126 can be rotated around its axis, for example by maneuvering the handle 110, to direct the Doppler sensor 180a toward various regions of the left ventricle 16. For example, the nosecone 1126 may be rotated 360 degrees around its longitudinal axis to map the flow in all lateral directions, or at least rotated at least between two diametrically opposing regions, so as to measure the flow within the LVOT 22 and the opposite region of the left ventricle 16, enabling detection of flow abnormalities such as undesirable vortex structures v₁ and v₂.

According to some embodiments, a method of measuring flow at different regions surrounding the outflow end portion 142 of a prosthetic valve 140, utilizing the delivery assembly 100 equipped with a Doppler sensor 180a retained within the nosecone 1126 as described herein above, is disclosed herein. The method comprises steps of partially expanding the prosthetic mitral valve 140 and deriving real-time Doppler flow readings during the expansion procedure, and accordingly recompressing and/or re-positioned the prosthetic mitral valve 140, as/if required.

The Doppler sensor 180a is utilized to acquire flow measurements from at least two diametrically opposing regions. According to some embodiments, the Doppler sensor 180a is first directed at one direction toward a first region, utilized to acquire measurement signals therefrom, and then the nosecone is rotated to orient the Doppler sensor 180a at a diametrically opposite direction, toward the second region. The Doppler sensor 180a can then be utilized to acquire measurement signals from the second region. Alternatively or additionally, the Doppler sensor 180a may be provided with a plurality of ultrasonic transducers, oriented toward both the first region and the opposing second region.

A re-compression mechanism can be advantageously utilized in combination with the delivery assemblies 100 having a Doppler sensor 180a retained within a nosecone 1126, enabling prosthetic valve re-compression in order to re-orient or reposition it, if required, in light of real-time flow measurements received from the Doppler sensor 180a.

In some instances, it may be desirable to assess the distance between a prosthetic valve 140, such as the prosthetic mitral valve shown in Fig. 27, and a native tissue, such as the septum 20. The distance between the prosthetic mitral valve 140 and the septum 20 may provide additional data that can influence a desired prosthetic valve mounting configuration.

According to some embodiments, there is provided a delivery assembly equipped with a prosthetic mitral valve 140, and a nosecone 1226 comprising a first sensor 180a retained therein, wherein the first sensor 180a is a distance measurement sensor. According to some embodiments, the first sensor 180a is an ultrasonic distance sensor, comprising at least one ultrasonic transducer for measuring distance within a chamber of the heart. The delivery assembly 100 shown in Fig. 27 can be equipped with an ultrasonic distance sensor 180a retained within the nosecone 1126, which can be oriented toward a region of interest, such as the septum 20 or any other wall of the left ventricle 16, to measure distance from the position of the ultrasonic distance sensor 180a to the septum 20 or any other structure.

According to some embodiments, a method of measuring the distance between the prosthetic valve 140 and a heart chamber wall such as the septum 20, using an ultrasonic distance sensor 180a retained within a nosecone 1126 is disclosed herein. According to some embodiments, the method comprises the steps of positioning the nosecone 1126 such that the ultrasonic distance sensor 180a is positioned at the level of the outflow end portion 142 of the prosthetic valve 140, and oriented toward the septum 20 and measuring through operation of the ultrasonic sensor 180a, a distance to a side wall of the prosthetic valve 140, such as the side of the frame 146 facing the septum 20, as well as the distance to the septum 20, thereby obtaining the distance between the outflow end portion 142 of the prosthetic valve 140 and the septum 20.

An ultrasonic distance sensor measures distance based on a pulse-echo method, which determines the distance to an object by measuring time-of-flight of an ultrasonic pulse. This differs from an ultrasonic Doppler sensor, which is based in a pulse-Doppler method in accordance with the principles described above. Nevertheless, according to some embodiments, the first sensor 180a retained within the nosecone 1126, as illustrated and described in conjunction with Fig. 27, is an ultrasonic sensor that may be utilized both for flow measurements, based on a pulse-Doppler method, and for distance measurements, based on a pulse-echo method.

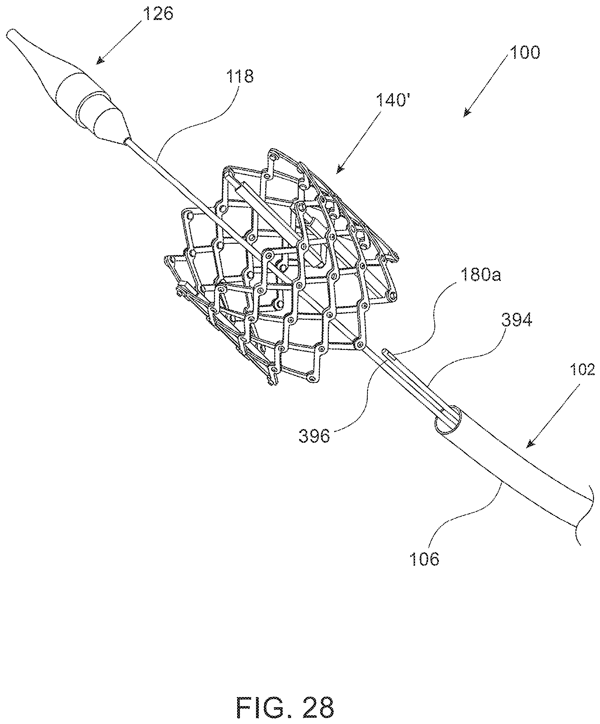

According to some embodiments, there is provided delivery assembly 100 comprising a prosthetic mitral valve 140 and a delivery apparatus 102, wherein the delivery apparatus 102 further comprises an ultrasonic measurement catheter 394 extending from the handle 110 through the delivery shaft 106. Fig. 28 shows a distal portion of a delivery assembly 100 comprising a prosthetic valve 140 carried over a delivery apparatus 102, wherein the delivery apparatus 102 further comprises the ultrasonic measurement catheter 394 having a sensing head 396 equipped with a first sensor 180a, which may include at least one ultrasonic transducer and perform either as a Doppler sensor for flow measurements, or a distance sensor, as described for such sensors in conjunction with Fig. 27. While a mechanically expandable valve 140' is illustrated in Fig. 28, it will be clear that the configurations of this figure applies to other types of prosthetic valves 140 in a similar manner.

The ultrasonic measurement catheter 394 may further comprise a transmission line extending from the ultrasonic sensor 180a toward the handle 100, such as the first transmission line 168a according to any of the embodiments disclosed herein.

The ultrasonic measurement catheter 394 may be axially movable relative to the delivery shaft 106. The movement of the ultrasonic measurement catheter 394 may be controlled by the handle 110. The ultrasonic measurement catheter 394 may be axially movable so as to extend through a lumen of the prosthetic valve 140, when the prosthetic valve 140 is expanded sufficiently to provide free passage there-through.

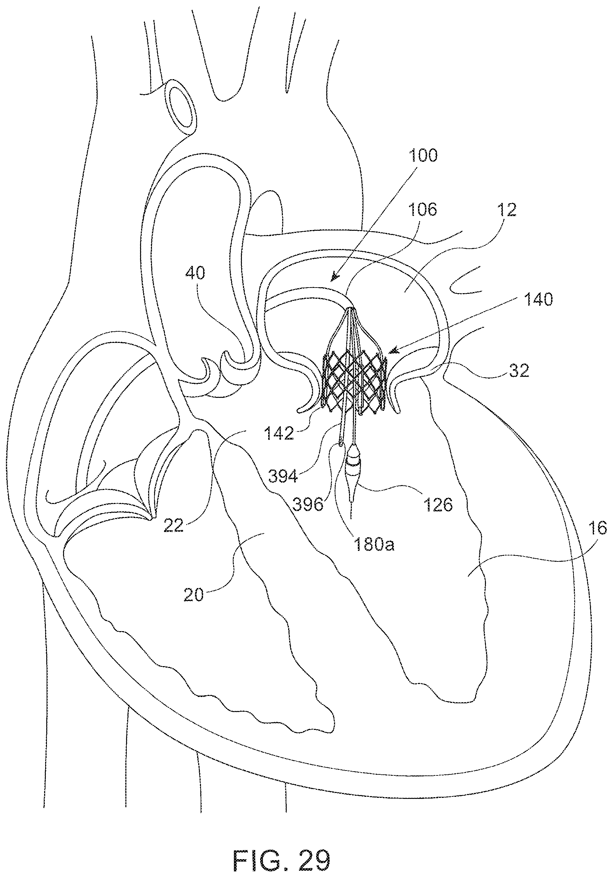

Reference is now made to Fig. 29, illustrating an embodiment of a delivery apparatus 102 equipped with an ultrasonic measurement catheter 394, carrying a prosthetic mitral valve 140 for mounting against the native mitral annulus 32. A delivery assembly 100 may be utilized according to conventional transcatheter valve replacement procedures, to advance the prosthetic valve 140 toward the mitral annulus 32 for mitral valve replacement. For example, the delivery assembly 100 may be utilized for delivering the prosthetic valve 140 in a crimped state, in a trans-septal procedure, as shown in Fig. 29, crossing the upper septum 20 toward the left atrium 12 using a conventional technique of first puncturing the fossa ovalis location with a sharpened device (not shown), such as a needle or a wire, optionally passing a dilator over the sharpened device, and then retracting the sharpened device while leaving the dilator in place, over which the delivery apparatus 102 may be advanced.

When the prosthetic mitral valve 140 is sufficiently expanded to provide passage there-through, the ultrasonic measurement catheter 394 may be distally advanced, as shown in Fig. 29, to a desired position so as to orient the ultrasonic sensor 180a toward a desired region of measurement.

According to some embodiments, the first sensor 180a retained within the sensing head 396 is a Doppler sensor. In such embodiments, the ultrasonic measurement catheter 394 can be oriented so as to direct the Doppler sensor 180a to measure flow in the LVOT 22. According to some embodiments, the ultrasonic measurement catheter 394 can be rotated around its axis, for example by maneuvering the handle 110, to direct the Doppler sensor 180a toward various regions of the left ventricle 16. For example, the ultrasonic measurement catheter 394 may be rotated 360 degrees around its longitudinal axis to map the flow in all lateral directions, or at least rotated so as to measure the flow within the LVOT 22 and the opposite region of the left ventricle 16, enabling detection of flow abnormalities such as undesirable vortex structures v₁ and v₂.

According to some embodiments, the Doppler sensor 180a is first directed at one direction toward a first region, utilized to acquire measurement signals therefrom, and then the nosecone is rotated to orient the Doppler sensor 180a at a diametrically opposite direction, toward the second region. The Doppler sensor 180a can then be utilized to acquire measurement signals from the second region. Alternatively, or additionally, the Doppler sensor 180a comprises an array of ultrasonic transducers spanning circumferentially within the sensing head 396, configured to provide measurement signals across 360 degrees, namely, around the longitudinal axis of the sensing head 396.

According to some embodiments, a method of utilizing the delivery assembly 100 equipped with the ultrasonic measurement catheter 394 having a Doppler sensor 180a, is disclosed herein. The method comprising the steps of partially expanding the prosthetic mitral valve 140, advancing the ultrasonic measurement catheter 394 through the lumen of the prosthetic mitral valve 140, potentially further expanding the prosthetic mitral valve 140 against the mitral annulus 32, and deriving real-time Doppler flow readings during the expansion procedure, thereby recompressing and re-positioning the prosthetic mitral valve 140, as/if required.

A re-compression mechanism can be advantageously utilized in combination with the delivery assemblies 100 having an ultrasonic measurement catheter 394 equipped with a Doppler sensor 180a, enabling prosthetic valve re-compression in order to re-orient or reposition it, if required, in light of real-time flow measurements received from the Doppler sensor 180a.

According to some embodiments, the first sensor 180a retained within the sensing head 396 is an ultrasonic distance sensor. In such embodiments, a method of using an ultrasonic distance sensor 180a can include a step of advancing the ultrasonic measurement catheter 394 through a partially (or fully) expanded prosthetic valve 140 (e.g., a prosthetic mitral valve) such that the ultrasonic sensor 180a is positioned at the level of the outflow end portion 142 of the prosthetic valve 140, and oriented toward the septum 20. The ultrasonic distance sensor 180a can then measure a distance to the side of the frame 146 facing the septum 20, as well as the distance to the septum 20, from which the distance between the outflow end portion 142 of the prosthetic valve 140 and the septum 20 can be derived.

While shown in Figs. 27 and 29 in relation to a delivery system carrying a prosthetic mitral valve, it will be clear that an ultrasonic sensor 180a, retained within a nosecone 1126 or an ultrasonic measurement catheter 394, can be implemented in a similar manner in combination with a delivery system carrying a prosthetic valve 140 for implantation at other locations of the heart, such as within the native aortic valve, the native pulmonary valve, and/or the native tricuspid valve.

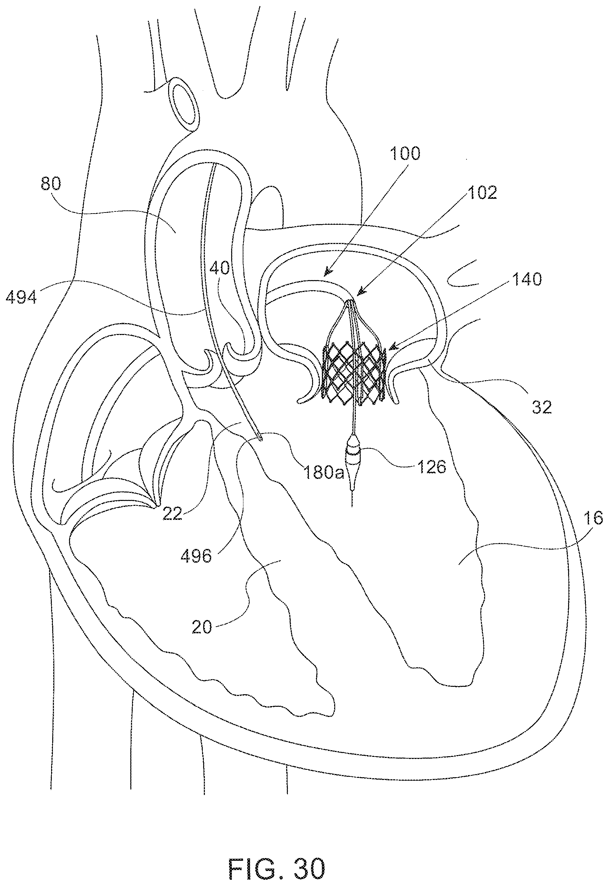

According to some embodiments, there is provided transcatheter Doppler regulated system, comprising a delivery assembly 100 and a separate Doppler catheter 494. The delivery assembly 100 comprises a prosthetic mitral valve 140 and a conventional delivery apparatus 102 as shown in Fig. 30, for example. The Doppler catheter 494 comprises a sensing head 496 equipped with a Doppler sensor 180a. The Doppler catheter is a stand-alone intravascular catheter which is not physically connected to the delivery assembly 100, such that each of the delivery assembly 100 and the Doppler catheter 494 can follow a different intravascular path along the patient's vasculature.

According to some embodiments, there is provided a method of measuring flow in a region adjacent a prosthetic valve 140, is disclosed herein. The method comprising the steps of delivering the prosthetic valve 140 over a delivery apparatus 102 to a first native valve (e.g., the native mitral valve 30), expanding the prosthetic valve 140 against the first native valve such that at least a portion of the prosthetic valve 140 extends into a heart chamber (e.g., the left ventricle 16), extending the Doppler catheter 494 through a second native valve (e.g., the native aortic valve 40) such that the sensing head 496 is positioned within the heart chamber, orienting the Doppler sensor 180a toward the prosthetic valve 140, and utilizing the Doppler sensor 180a to acquire measurement signals from at least one region adjacent a prosthetic valve 140 (e.g., the LVOT 22), and, optionally, from at least two diametrically opposite regions adjacent the prosthetic valve 140.

Reference is now made to Fig. 30, illustrating an embodiment of a transcatheter Doppler regulated system. A delivery assembly 100 may be utilized according to conventional transcatheter valve replacement procedures, to advance the prosthetic valve 140 toward the mitral annulus 32 for mitral valve replacement. For example, the delivery assembly 100 may be utilized for delivering the prosthetic valve 140 in a crimped state, in a trans-septal procedure, such as shown in Fig. 30. The Doppler catheter 494 is advanced, either along a similar or a different pathway of the delivery assembly 100, toward a desired flow measurement region, which can be in the vicinity of the prosthetic mitral valve 140.

According to some embodiments, the Doppler catheter 494 may be advanced through the patient's vasculature prior to utilizing delivery apparatus 102, such that the sensing head 496 may be disposed within a desired flow measurement region prior to positioning the prosthetic mitral valve 140 in the desired implantation site.

In the embodiment shown in Fig. 30, the prosthetic mitral valve 140 is expanded against the mitral annulus 32, and the distal portion of the Doppler catheter 494 extends through the aorta 80 and the aortic valve 40 so as to position the sensing head 496 within the LVOT 22. Advantageously, such a configuration enables the prosthetic mitral valve 140 to be delivered to the mitral annulus 32 via any delivery approach, such as tans-femoral, trans-septal, trans-apical or other percutaneous approach, without restricting the available measurement regions for a Doppler sensor 180a.

In comparison, a Doppler sensor 180a retained within a nosecone 1126, as described and illustrated in conjunction with Fig. 27, or a Doppler sensor 180a retained within an ultrasonic measurement catheter 394 extendable through a delivery shaft 106, as described and illustrated in conjunction with Fig. 29, may be more suitable for a trans-septal approach, wherein the Doppler sensor 180a can be positioned within the lumen of a prosthetic mitral valve 140 or extendable distal thereto, and less suitable for a trans-apical approach (not shown), wherein components of the delivery apparatus 102, such as the delivery shaft 106, may partially obstruct the Doppler sensor's ability to measure certain regions within the left ventricle 16. Moreover, having the Doppler catheter 494 provided separately from the delivery apparatus 102 allows the clinician to independently control the Doppler catheter 494 and the delivery assembly 100, as necessary.

According to some embodiments, the Doppler sensor 180a comprises an array of ultrasonic transducers spanning circumferentially within the sensing head 496, configured to provide measurement signals spanning 360 degrees across the longitudinal axis of the sensing head 496.

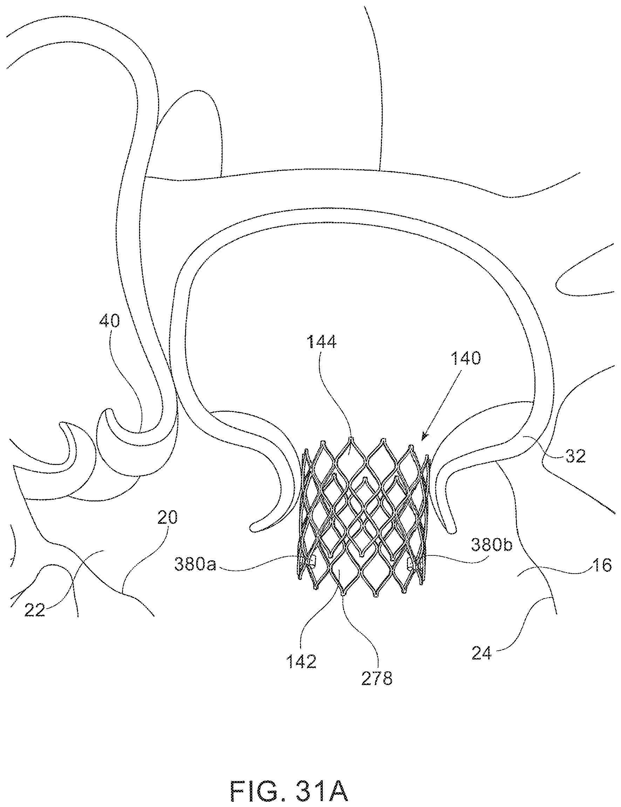

Reference is now made to Figs. 31A-31E, illustrating various configurations of a prosthetic valve 140 comprising a plurality of sensors 380 attached thereto. The positioning of the sensor 380 are demonstrated in Figs. 31A-31E for a mitral prosthetic valve positioned within the mitral annulus 32, such that the inflow end portion 144 is facing the left atrium 12 and the outflow end portion 142 extends into the left ventricle 16. According to some embodiments, the plurality of sensor 380 shown and described in conjunction with Figs. 31A-31E are configured to measure a physiological flow-related property, such as blood pressure and/or blood flow. According to some embodiments, the plurality of sensors 380 are pressure sensors. According to some embodiments, plurality of sensors 380 are flow sensors.

According to some embodiments, a prosthetic valve 140, such as a prosthetic mitral valve, comprises a plurality of sensors 380 attached to the outflow end portion 142, as shown in Fig. 31A. According to some embodiments, at least two sensors of the plurality of sensors 380 are circumferentially distanced from each other. According to some embodiments, at least two sensors of the plurality of sensors 380 are circumferentially equi-spaced from each other. According to some embodiments, at least two sensors of the plurality of sensors 380 are attached to the outflow end portion 142 at diametrically opposite positions.

According to some embodiments, at least two sensors of the plurality of sensors 380 are positioned at the same valve horizontal plane, defined as any plane which is substantially orthogonal to the valve longitudinal axis 141.

Fig. 31A shows an embodiment of a prosthetic mitral valve 140 equipped with the first sensors 380a and the second sensor 380c attached to the outflow end portion 142 at diametrically opposite positions across the same horizontal plane. In this configuration, the first sensor 380a may face the septum 20 while the second sensor 380b may face the free wall 24 of the left ventricle 16.

Fig. 31A may represent an exemplary embodiment of two flow sensors 380a, 380b that can simultaneously measure flow at two diametrically opposite regions of the prosthetic valve 140. For example, the first flow sensor 380a can measure flow in vicinity of the LVOT 22, while the second flow sensor 380b can measure flow at the opposite region bound between the prosthetic mitral valve 140 and the free wall 24 of the left ventricle 16. According to some embodiments, abnormal flow patterns may be detected by comparing the measurements of the first and the second flow sensors 380a and 380b, respectively, for example to detect abnormal vortex formations v₁ and v₂ during the cardiac cycle.

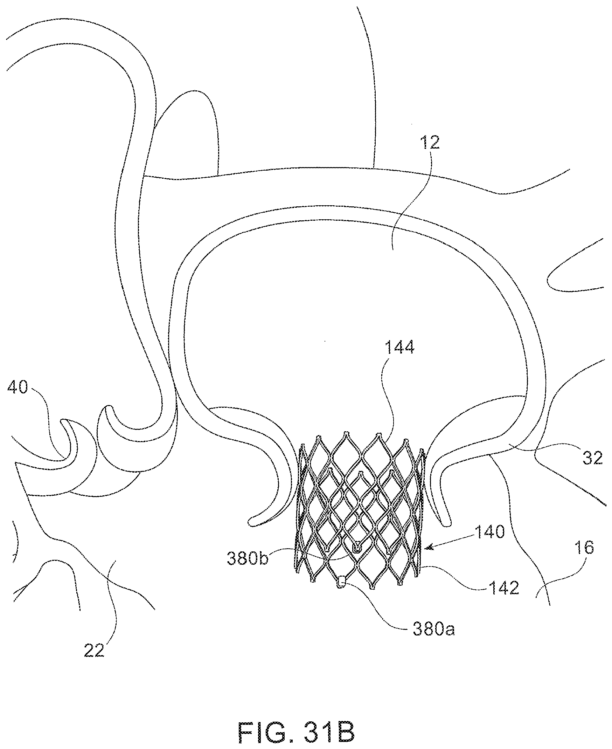

Adequate positioning of the first and second sensors 380a and 380b, respectively, may be required in order to derive meaningful measurements from desired regions of interest. Fig. 31B shows an inadequate circumferential orientation of the prosthetic valve 140, wherein both sensors 380a and 380b are substantially equally spaced from the septum 20 and/or the free wall 24 of the left ventricle 16. Such a position is inadequate if measurements from different regions, such as regions closer to vortex rings v₁ and v₂, are desired.

According to some embodiments, at least two of the plurality of sensors 380 comprise radiopaque markings, to enable visual detection of their positions during prosthetic valve implantation and positioning procedures. The markings may enable a clinician to reposition or reorient the prosthetic valve 140 (e.g., a prosthetic mitral valve), such that the plurality of sensors 380 are positioned and oriented at desired regions of interest, for example, the positions of the first sensor 380a and the second sensor 380b shown in Fig. 31A.

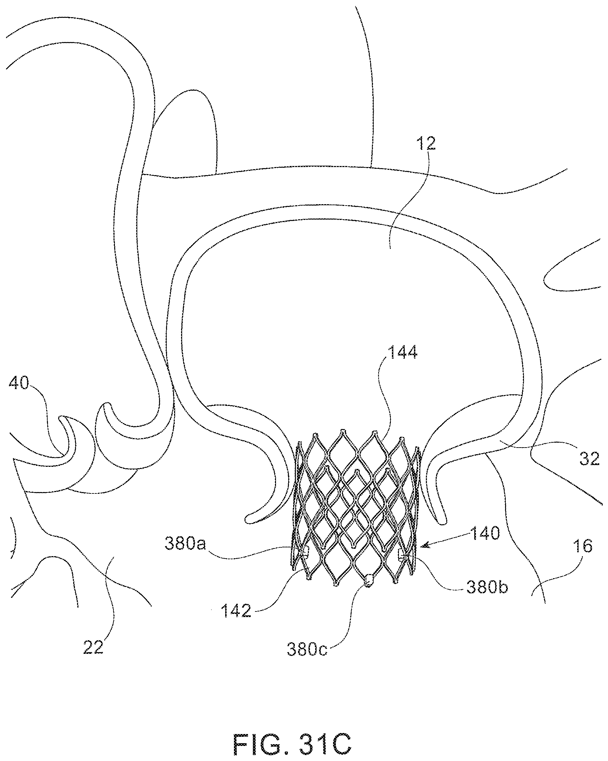

According to some embodiments, the plurality of sensors 380 include more than two sensors. Fig. 31C shows an exemplary embodiment of three sensors 380a, 380b and 380c attached to the outflow end portion 142. Advantageously, more than two sensors 380 may provide a better resolution by mapping the flow at several points along the outflow end portion 142. Furthermore, more than two sensors 380 may enable easier circumferential valve orientation to position the sensors 380 at desired regions of interest.

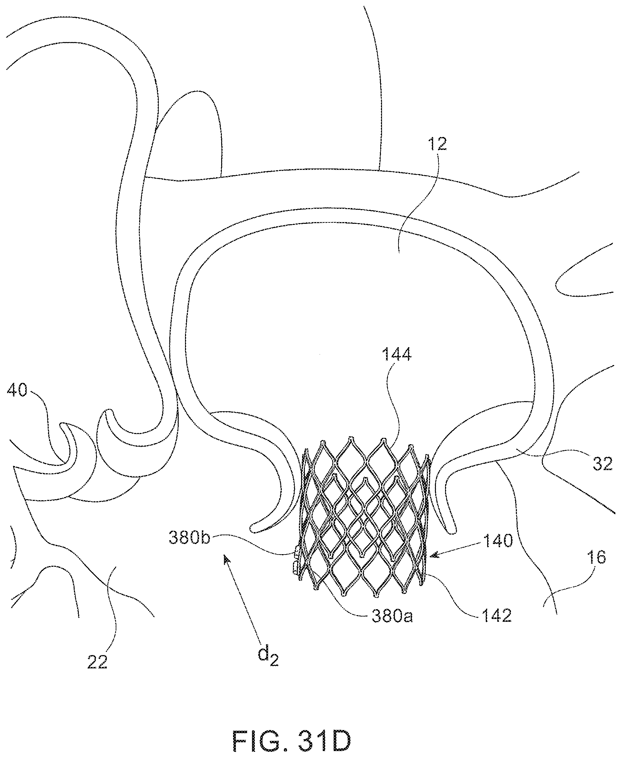

According to some embodiments, at least two sensors of the plurality of sensors 380 are axially distanced from each other along the outflow end portion 142. Fig. 31D shows an exemplary embodiment of the first sensor 380a and the second sensor 380b axially distanced from each other, such that each of the sensors 380a and 380b is positioned at a different horizontal plane along the outflow end portion 142.

In the exemplary embodiment of Fig. 31D, both the first sensor 380a and the second sensor 380b are axially distanced from each other, and are longitudinally aligned along the same circumferential position of the prosthetic valve 140, such that both may circumferentially face the same region of interest, which is the LVOT 22 in Fig. 31D.

Measurements taken along different axial, optionally longitudinally aligned, positions, may serve to detect flow disturbances such as stagnation or abnormal flow recirculation.

Fig. 31D may represent an exemplary embodiment of two pressure sensors 380a and 380b configured to detect pressure differences between different regions along the trajectory of flow d₂ through the LVOT 22 toward the aortic valve 40. For example, a comparison between the measured pressure at the region of the second sensor 380b with the pressure measured at the region of the first sensor 380a may be indicative of flow disturbances in the LVOT 22. Alternatively, or additionally, Fig. 31D may represent an exemplary embodiment of two flow sensors 380a and 380b configured to measure flow at different regions along the trajectory of flow d₂ to derive a flow profile through the LVOT 22.

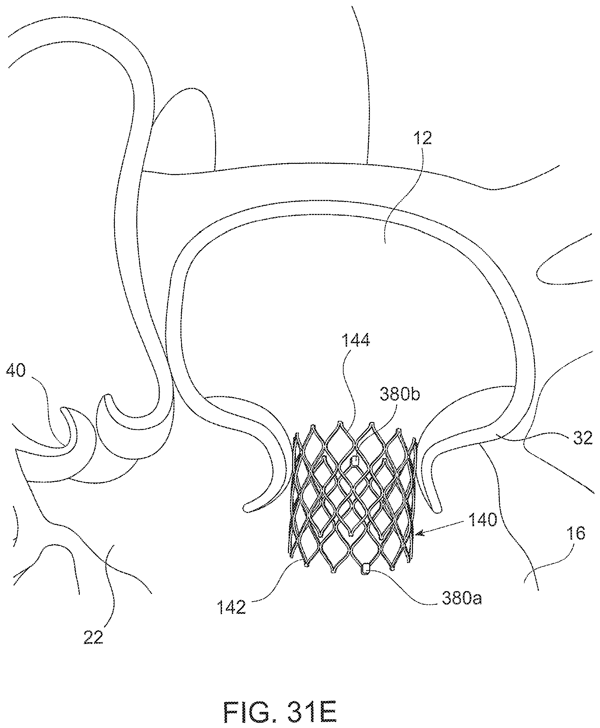

Fig. 31E shows an exemplary embodiment of the first sensor 380a attached to the inflow end portion 144 and the second sensor 380b attached to the outflow end portion 142, similar to the configuration illustrated and described in conjunction with Figs. 22A-22B. The axially distanced sensors 380a and 380b can be either pressure sensors utilized for derivation of a pressure drop profile between the inflow end portion 144 and the outflow end portion 142 of the prosthetic mitral valve 140, or flow sensors utilized to provide the flow profile through the prosthetic mitral valve 140.

Although shown in Figs. 31A-31E in combination with a prosthetic mitral valve, one or more sensors 380 can be similarly attached to any other type of a prosthetic valve 140 implanted at other location of the heart, such as within the native aortic valve, the native pulmonary valve, and the native tricuspid valve.

The position of a sensor 380 along the prosthetic valve 140 can influence the accuracy of measurement. Care must be taken, especially in the case of axially distanced flow or pressure sensors 380, to avoid contact between a sensor and native tissue, such as the mitral annulus 32 or the native mitral leaflets 34, as such contact may limit the ability to derive meaningful measurement signals. Furthermore, a sensor 380 pressed against a native tissue might induce a physiological reaction, such as neo-intimal growth, which can influence and/or impact long and short term accuracy of measurements.

According to some embodiments, one or more sensors 380 are coupled to the lumenal surface of the prosthetic valve 460. This configuration may help, for example, to avoid interference between the one or more sensors 380 and portions of the native anatomy that would otherwise contact the sensors 380. Moreover, such a configuration may help ensure that the one or more sensors 380 are exposed to blood passing through the prosthetic valve 140, which in some instances may allow more accurate measurements compared with sensors facing away from the valve longitudinal axis 141.

Alternatively, or additionally, one or more sensors 380 may be coupled to the external surface of the prosthetic valve 140. This configuration may useful for measurement of physiological parameters in the immediate environment surrounding the valve 140. Furthermore, such a configuration may help, for example in cases when one or more sensors 380 are coupled to the outflow end portion 142, to avoid interference between the sensors 380 and the leaflets 152, that could otherwise contact the lumenal surface of the frame 146 during a phase of the cardiac cycle, such as systole.

According to some embodiments, threshold values for either flow or pressure measurements are pre-set, such that measured signals exceeding the pre-set threshold values, either above a pre-set maximal value or below a pre-set minimal value, may produce a visual or an auditory warning to the clinician. Alternatively, exceeding the predetermined threshold value may automatically halt the transplantation procedure.

According to some embodiments, the plurality of sensors 380 according to any of the embodiments described and illustrated in conjunction with Figs. 31A-31E may be releasably coupled to corresponding transmission lines 368. In such embodiments, any of the sensors 380 may be retained within a corresponding sensor housing 382, and any one of the transmission lines 368 may extend through a transmission line shaft 376, wherein the sensor housing 382 may be attached to the prosthetic valve 140 according to any of the configurations described and illustrated in conjunction with Figs. 31A-31E, and the transmission line shaft 376 may be releasably coupled to the sensor housing 382 in a manner similar to that described and illustrated in conjunction with Figs. 23A-23C. Alternatively, or additionally, the plurality of sensors 380 according to any one of the embodiments described and illustrated in conjunction with Figs. 31A-31E may be post-procedural sensors 380.

According to some embodiments, the magnitude of the measured parameters, such as maximal or average flow or pressure, can be indicative of certain clinically relevant assessments. For example, post-procedural measurement signals acquired by post-procedural sensors 380 can be indicative of the cardiac output (CO), which may be of high interest for patients having cardiac-resynchronization-therapy (CRT) devices, in order to monitor improvement or deterioration of the CO. Such inputs can assist in decision making regarding the need to readjust synchronization parameters of the CRT device.

Prosthetic valve related hemodynamic disturbances are not confined only to flow disturbances occurring during valve implantation procedures, for example due to sub-optimal valve orientation, expansion and/or positioning, but may also develop over time, post implantation, for example due to inflammatory and other biological processes that may result from valve-tissue or valve-blood-flow interactions.

One such complication is associated with flow stagnation within the small anatomic space confined between the leaflets 152 and the frame 146 of a prosthetic valve 140, which may promote leaflet thrombosis. Even sub-clinical leaflet thrombosis may be associated with reduced leaflet motility, thereby deteriorating prosthetic valve performance. It is desirable, therefore, to provide means of measuring the flow patterns in such regions of interest, in order to detect whether the flow field around the leaflets 152 is disrupted.

According to some embodiments, there is provided a method of using a delivery assembly 100 carrying a prosthetic valve 140 (e.g., a prosthetic aortic valve) and comprising an ultrasonic measurement catheter 394 extending through the delivery shaft 106. The method includes the steps of deploying the prosthetic valve at the region of interest, such as the aortic annulus 42, advancing the ultrasonic measurement catheter 394 to the region of the deployed prosthetic valve 140, and subsequently acquiring measurements of the flow at the anatomic spaces confined between the leaflets 152 and frame 146.

According to some embodiments, the ultrasonic measurement catheter 394 comprises a Doppler sensor 180a, configured to provide flow pattern measurements that can be compared to absolute threshold values, for example to detect long-residence time that may exceed a pre-set threshold value.

The ultrasonic measurement catheter 394 may be rotated to direct the Doppler sensor 180a toward a selected region of interest, or toward several regions of interest. According to some embodiments, measurements acquired by the Doppler sensor 180a from different regions, such as the anatomic spaces confined between each of the leaflets 152 and the frame 146, can be compared with each other. Such comparison can be useful for detection of susceptible regions exhibiting flow which is disturbed relative to other regions.

According to some embodiments, the ultrasonic measurement catheter 394 is configured to provide real-time measurement signals during prosthetic valve deployment. According to some embodiments, the ultrasonic measurement catheter 394 is retracted once the valve deployment procedure is completed.

In some cases, thrombus may be formed in regions subjected to low flow or blood stasis, such as the regions bound between leaflets 152 and the frame 146. According to some embodiments, there is provided a method of detecting leaflet thrombosis or leaflet calcifications using an ultrasound echocardiography catheter 594. The ultrasound echocardiography catheter 594 comprises a sensing head 596, which comprises an ultrasound echocardiography sensor 180a. The ultrasound echocardiography sensor 180a may be utilized for imaging the leaflets 152, for example to detect leaflet thrombosis, leaflet calcification or any other deposits formed thereon.

Leaflet thrombosis usually occurs in the course of several days post-implantation. Leaflet stenosis is usually a result of an even longer process. Thus, leaflet thrombosis or leaflet calcification detection is a post-procedural process.

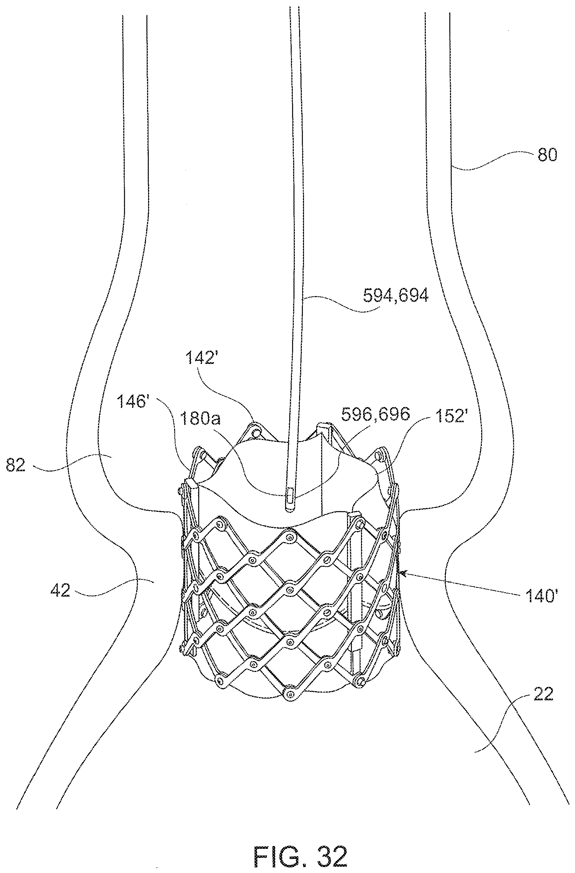

Reference is now made to Fig. 32, illustrating an embodiment of an ultrasound echocardiography catheter 594 advanced toward a prosthetic valve 140' implanted in the aortic annulus 42. According to some embodiments, a method of identifying leaflet thrombosis within a pre-mounted prosthetic valve 140, using the ultrasound echocardiography catheter 594, comprises the step of introducing the ultrasound echocardiography catheter 594 into a patient's body during a follow-up visit and not during valve deployment, wherein the sensing head 596 is advanced into the lumen of the prosthetic valve 140' through the opening defined by the outflow end portion 142', to a position adjacent an imaging region of interest. For example, the ultrasound echocardiography sensor 180a may be directed toward at least one of the leaflets 152', and utilized to acquire an image of the anatomic space confined between leaflet 152' and the frame 146'.

According to some embodiments, the ultrasound echocardiography sensor 180a comprises at least one ultrasound transducer configured to provide imaging across a specific lateral region projecting radially outward therefrom. The ultrasound echocardiography catheter 594 may be rotated around its longitudinal axis to orient the ultrasound transducer toward any of the desired regions. For example, the ultrasound echocardiography sensor 180a may be directed toward one leaflet 152', utilized to acquire an image of the anatomic space confined between this leaflet 152' and the frame 146', the sensing head 596 can then be rotated to direct the ultrasound echocardiography sensor 180a toward one other, different leaflet 152', utilized to acquire an image of the anatomic space confined between the at least one other leaflet 152' and the frame 146', and so on.

According to some embodiments, the sensing head 596 comprises an array of ultrasound transducers spanning it circumferentially, configured to provide lateral imaging spanning 360 degrees across the longitudinal axis of the sensing head 596.

According to some embodiments, there is provided a method for measuring changes in blood viscosity comprising the use of an acoustic viscosity catheter 694 comprising a sensing head 696, which comprises an acoustic viscosity sensor 180a. The method may be applied, for example, in the anatomic spaces confined between the leaflets 152 and the frame 146.

Blood viscosity may change prior to, or in the early stages of, thrombosis. For example, blood viscosity can be altered due to change in blood composition, which may include particulates such as fibrinogen. Fig. 32 may be similarly illustrative of an embodiment of an acoustic viscosity catheter 694 advanced toward a prosthetic valve 140' implanted in the aortic annulus 42. According to some embodiments, a method of using the acoustic viscosity catheter 694 includes a step of introducing the acoustic viscosity catheter 694 into a patient's body during a follow-up visit and not during valve deployment, wherein the sensing head 696 is advanced into the lumen of the prosthetic valve 140' through the opening defined by the outflow end portion 142', to a position adjacent a measurement region of interest. For example, the acoustic viscosity sensor 180a may be directed toward at least one of the leaflets 152', and utilized to measure blood viscosity in the anatomic space confined between leaflet 152' and the frame 146'.

According to some embodiments, the acoustic viscosity sensor 180a comprises an acoustic wave transducer and a piezoelectric transducer, configured to measure modifications in the acoustic field quantities of the acoustic wave transducer. The acoustic viscosity catheter 694 may be rotated around its longitudinal axis to orient the acoustic viscosity sensor 180a toward any of the desired circumferential regions. For example, the acoustic viscosity sensor 180a may be directed toward one leaflet 152', utilized to measure modifications in the acoustic field quantities in the anatomic space confined between this leaflet 152' and the frame 146', the sensing head 696 can then be rotated to direct the acoustic viscosity sensor 180a toward one other, different leaflet 152', and utilized to measure modifications in the acoustic field quantities in the anatomic space confined between the at least one other leaflet 152' and the frame 146', and so on.

Although shown in Fig. 32 in conjunction with a mechanically expandable aortic valve 140', either the ultrasound echocardiography catheter 594 or the acoustic viscosity catheter 694 can be similarly utilized in conjunction with any other type of a prosthetic valve 140, positioned within the aortic annulus 42 or at other locations of the heart, such as within the mitral annulus 32, the pulmonary valve annulus, and the tricuspid valve annulus.

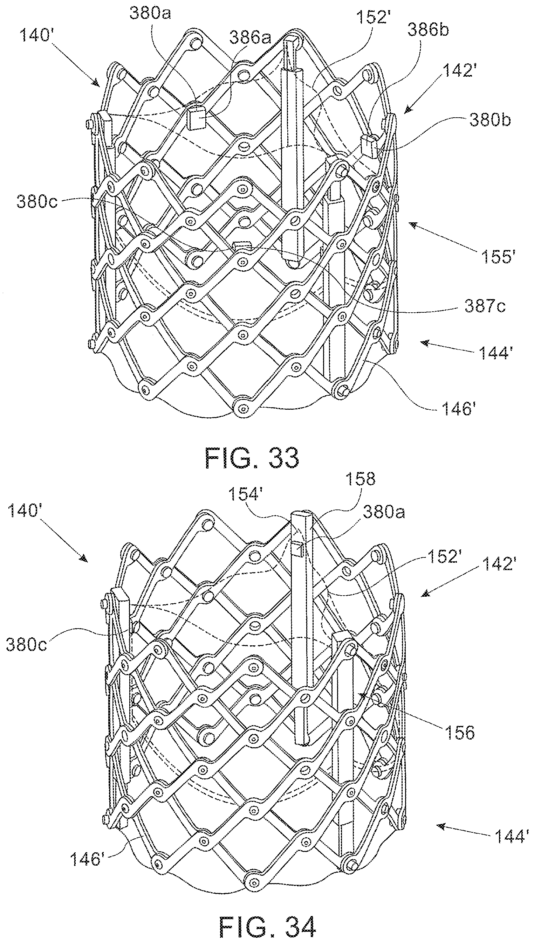

Reference is now made to Figs. 33-36, illustrating various configurations of a prosthetic valve 140, such as a mechanically expandable valve 140', comprising a plurality of sensors 380 attached thereto.

According to some embodiments, the prosthetic valve 140 comprises a plurality of sensors 380 circumferentially distanced from each other and attached to a mid-portion 155 of the prosthetic valve 140. The mid-portion 155 is defined as the region between the inflow end portion 144 and the outflow end portion 142.

Fig. 33 shows an embodiment of a prosthetic valve 140' equipped with three circumferentially distanced sensors 380a, 380b and 380c, attached to the mid-portion 155' across the same horizontal plane. The axial position of the sensors 380a, 380b and 380c may be chosen to be adjacent the small anatomic spaces confined between the leaflets 152' and the frame 146'.

According to some embodiments, the amount of sensors 380 is equal to the amount of leaflets 152'. According to some embodiments, each sensor 380 is positioned in the vicinity of one of the leaflets 152', and configured the measure hemodynamic parameters, such as blood flow or pressure, within anatomic spaces confined between the leaflet 152' and the frame 146'. According to some embodiments, each sensor 380 is oriented radially inward, facing a corresponding leaflet 152'. Each sensor can be attached to the prosthetic valve 140 such that its passive face 387 is directed at the frame 146', while its active face 386 is directed at the leaflet 152'.

According to some embodiments, the at least one sensor 380 is a flow sensor, configured to provide flow measurement signals that can be compared to absolute threshold values, for example to detect long-residence time that may exceed the pre-set threshold value.

According to some embodiments, the at least one sensor 380 is a pressure sensor, configured to provide pressure measurement signals that can be associated with flow values, and can be compared to absolute threshold values, for example to detect long-residence time that may exceed the pre-set threshold value. Pressure sensors 380 can sense pressure variations associated with the change in flow velocity. Without being bound by any theory or mechanism of action, such measurement may be based on Bernoulli's principle, namely, an increase in the speed of a fluid can occur simultaneously with a decrease in pressure.

According to some embodiments, readings from different sensors 380 can be compared with each other to detect regions in which the flow or pressure is disturbed relative to other regions or to detect regions susceptible to such disturbances.

According to some embodiments, the at least one sensor 380, and preferably a plurality of sensors such as the sensors 380a, 380b and 380c shown in Fig. 33, are fiber optic sensors, oriented toward corresponding leaflets 152' and configured to obtain light data within the region confined between the leaflets 152' and the frame 146'.

Advantageously, optic sensors such as sensors 380a, 380b and 380c, oriented toward the anatomic spaces confined between the leaflets 152' and the frame 146', can be configured to obtain light data along the surface of the leaflets 152', which can be used to provide an indication regarding thrombus formation, calcification or other particulate matter accumulated thereon.

According to some embodiments, the at least one sensor 380, and preferably a plurality of sensors such as the sensors 380a, 380b and 380c shown in Fig. 33, are impedance sensors, oriented towards the corresponding leaflets 152' and configured to obtain electric conductivity data within the region confined between the leaflets 152' and the frame 146'.

The conductivity of blood may be affected by flow induced changes, which in turn may affect, for example, the orientation of red blood cells and other particulates. According to some embodiments, the sensors 380 are configured to detect changes in impedance induced by changes in blood flow in the regions confined between the leaflets 152' and the frame 146'.

According to some embodiments, the measurement signals acquired by the sensors 380 are compared to absolute threshold values, for example to detect abnormal impedance values that may be indicative of flow disturbance.

According to some embodiments, the impedance sensors 380 are post-procedural sensors 380, configured to wirelessly transmit impedance measurement signals acquired by the sensors 380, thereby enabling post-procedural monitoring to detect valve performance deterioration over time.

The conductivity of blood may be also affected by its composition or viscosity, for example due to particulates such as fibrinogen affecting the composition and viscosity of the blood. Thus, changes in impedance can be analyzed in order to detect changes in the blood composition or viscosity in the regions confined between the leaflets 152' and the frame 146'.

According to some embodiments, each impedance sensor 380 is positioned substantially in front of the distal region of a respective leaflet 152', in close proximity to its attachment to the frame 146'. The impedance of blood differs from the impedance of the material from which the prosthetic leaflet 152' is made (e.g., pericardial tissue), and may depend on the morphological properties of the material. As such, changes in impedance can indicate the presence of a thrombus formed in the regions confined between the leaflet 152' and the frame 146', as well as the presence of calcified debris and/or other large deposits.

Advantageously, detection of post-procedural leaflet thickening, acquired for example by post-procedural impedance sensors 380, can be followed by appropriate therapy, such as oral anti-coagulation therapy. It is preferable to avoid uniform antiplatelet therapy for all patients, irrespective of their individual needs, as such therapies may also result in undesirable bleeding-risks. Thus, it is important to determine the appropriate anticoagulation therapy on a case-by-case basis.

Advantageously, post-procedural measurements obtained from post-procedural impedance sensors 380 may provide data regarding patients who develop leaflet thrombosis. Thus, the methods and systems disclose herein enable to design custom-made anticoagulation therapy to patients in need thereof. Moreover, it is possible to follow up and acquire impedance measurements during anticoagulation therapy, to determine treatment regimen and effectiveness.

According to some embodiments, the prosthetic valve 140 comprises at least one sensor 380 coupled thereto at the region of at least one commissure 154. Fig. 34 shows a prosthetic valve 140' comprising three sensors 380a, 380b and 380c (sensor 380b is hidden from view), attached to three corresponding commissures 154. According to some embodiments, the sensors 380 can be attached to commissure posts, such as outer members 158 of actuator assemblies 156, and more specifically, to an inner surface of such posts.

The sensors 380 attached to the commissures 154 can be implemented as any of the flow sensors, pressure sensors, optic sensors and/or impedance sensors, described above in conjunction with Fig. 33. Moreover, the sensors 380 attached to the commissures 154 may be implemented as post-procedural sensors. Positioning a sensor 380 at the commissure 154, between two adjacent leaflet 152, may provide data regarding various parameters, such as deposit accumulation, pannus and the like, focused in the regions of the commissures 154 or adjacent to such regions.

A prosthetic aortic valve 140 may be deployed within an aortic annulus 42, such that the outflow end portion 142 extends proximally beyond the native aortic leaflets 44. In such cases, a gap may be formed between the outflow end portion 142 and the surrounding anatomy, enabling placement of sensors 380 oriented radially outward from its frame 146, without risking the sensors 380 being pressed against the blood vessel's wall.

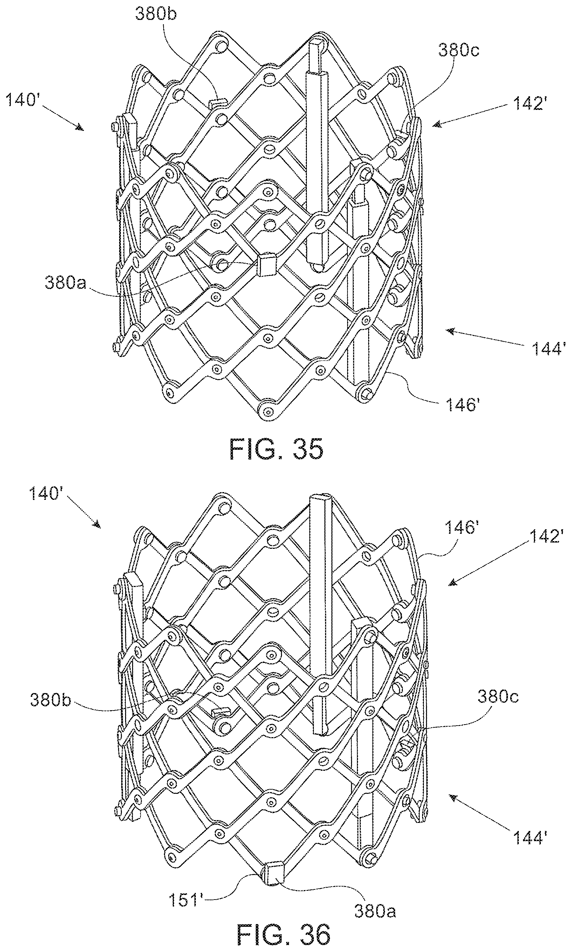

Fig. 35 shows an exemplary embodiment of three sensors 380a, 380b and 380c attached to the outflow end portion 142'. According to some embodiments, each sensor 380 is oriented radially outward from the frame 146', configured to measure a hemodynamic parameter, such as flow or pressure, in a region surrounding the prosthetic valve 140. For example, the sensors 380a, 380b and 380c shown in Fig. 35 can measure hemodynamic parameters in a region confined between the outflow end portion 142' and the surrounding anatomy (not shown in Fig. 35). Measurement of flow or pressure in that region may be desirable, for example, for detection of flow patterns of interest in the vicinity of the coronary ostia.

According to some embodiments, at least one flow or pressure sensor 380, and preferably a plurality of flow or pressure sensors 380, are attached to the prosthetic valve 140 and configured to detect central leak of the prosthetic valve 140. For example, in the case of a prosthetic aortic valve 140, aortic insufficiency may be detected by the sensors 380 either during prosthetic aortic valve deployment, or as a post-procedural ongoing monitoring process utilizing ongoing measurements derived from post-procedural sensors 380.

According to some embodiments, at least one sensor 380, and preferably a plurality of sensors, such as sensors 380a, 380b and 380c shown in Fig. 35, are temperature sensors, oriented radially outward from the frame 146', and configured to contact the surrounding tissue in order to measure tissue temperature. An inflamed region may be identified by detecting temperature above the normal body temperature. Elevated temperature typically indicates metabolic activity of inflammatory cells within the tissue. Specifically, activated inflammatory cells have a heat signature which is slightly higher than that of connective tissue cells. The sensitivity of the temperature sensors 380 is configured to match the expected temperature variations, in order to adequately detect inflammation.

Utilization of temperature sensors 380 is feasible as long as the sensors 380 are attached to the outer surface of the prosthetic valve 140 in such a manner that they contact the surrounding tissue once the prosthetic aortic valve 360 is positioned at the implantation site. Alternatively, the temperature sensors can be in close vicinity to the surrounding tissue, rather than in full contact therewith. In such configuration, blood temperature near the tissue of interest is measured (rather than tissue temperature).. According to some embodiments, temperature sensors 380 are post-procedural temperature sensors 380intended for detecting post-procedural inflammation.

Fig. 36 shows an exemplary embodiment of three sensors 380a, 380b and 380c attached to the inflow end portion 144'. According to some embodiments, each sensor 380 is oriented radially outward from the frame 146', configured to measure physiological parameters in regions surrounding the prosthetic valve 140'. For example, the sensors 380a, 380b and 380c may be configured to contact the aortic wall tissue or the aortic annulus 42.

According to some embodiments, at least one sensor 380, and preferably a plurality of sensors, such as sensors 380a, 380b and 380c shown in Fig. 36, are temperature sensors, attached to the inflow end portion 144' and oriented radially outward from the frame 146', configured to contact the aortic annulus 42 or any other annulus or native tissue, in order to measure tissue temperature.

According to some embodiments, a plurality of temperature sensors 380 are circumferentially distanced from each other, as shown in Figs. 35-36. According to some embodiments, at least two temperature sensors 380 are axially spaced from each other, for example, similar to the configurations shown in Figs. 23A-23B.

According to some embodiments, temperature measurements from different temperature sensors 380 disposed around different regions of the prosthetic valve 140 are compared with each other, to generate a temperature-map of the surrounding tissues, and detect whether an inflamed area is confined to a specific region, or whether it surrounds the entire prosthetic valve 140.

According to some embodiments, temperature may be measured periodically to detect potential rise in measured temperature values over time, in order to monitor inflammation development.

Advantageously, post-procedural readings from the post-procedural temperature sensors 380 may assist a clinician to determine type of recommended anti-inflammatory therapy. Moreover, it is possible to follow up and obtain temperature readings during the anti-inflammatory therapy, to observe treatment effectiveness and/or determine a desired treatment regimen.

According to some embodiments, at least one sensor 380, and preferably a plurality of sensors, such as sensors 380a, 380b and 380c shown in Fig. 36, are sensing electrodes, oriented radially outward from inflow end portion 144', and configured to contact the surrounding tissue, such as the aortic annulus 42, in order to measure the intrinsic electrical activity of the tissue. The sensing electrodes 380 exploit the intrinsic electrical heart activity, to detect regions of reduced activity which can be attributed to scarred inflamed tissue.

The sensing electrodes 380 can be located at any position along the prosthetic valve 140, as long as they may directly contact the surrounding tissue once the prosthetic valve 140 is deployed. An example for a preferred location for sensing electrodes 380 attachment, is along the inflow end portion 144', for example along the inflow apices 151, where the proximity of the natural rapid conduction paths may be valuable. It will be clear that the number and locations of sensing electrodes 380 may vary, and in some embodiments, may comprise a single sensing electrode 380.

According to some embodiments, the sensing electrodes 380 are attached to the frame 146 or any other component of the prosthetic valve 140, oriented radially outward therefrom so as to directly contact the surrounding tissue. According to some embodiments, the sensing electrodes 280 are electrically isolated from the frame 146.

According to some embodiments, the sensing electrodes 380 are attached to positions of a prosthetic valve 140 (e.g., a prosthetic aortic valve), which enable contact between the sensing electrodes 380 and a proximal portion of the coronary sinus when the prosthetic valve 140 is implanted within the patient's body.

According to some embodiments, the sensing electrodes 380 are operable for sensing purposes only, and not for providing electric pacing signals.

In some instances, a time-dependent decay in tissue electric activity is expected due to foreign body implantation, which results in an expected loss of cells depolarization in the vicinity of device implantation. The signals acquired by the sensing electrodes 380 can be compared with the expected decay or pre-determined threshold values, in order to detect unexpected decay rates, indicative of inflammatory regions that may warrant appropriate treatment protocols.

Mitigation of such inflammatory response can be achieved by using suitable drugs, such as steroids. Alternatively, or additionally, the surface of the sensing electrodes 380 may be coated with anti-inflammatory drugs, as a preventive measure. According to some embodiments, the outer surfaces of the sensing electrodes 380 are coated by a nano-level rough material, configured to prevent relative motion that may cause constant irritation.

According to some embodiments, the sensing electrodes 380 are coated with materials configured to diminish electrode polarization. According to some embodiments, the sensing electrodes 380 are fractal coated, with coating materials such as, but not limited to, Irox (Iridium Oxide) or TiN (Titanium nitride). Surfaces of fractal coatings are constructed by repeated application of a mathematical operation which doubles the electrochemically active surface area. Repeating the doubling steps ten times, for example, may give rise to a ratio between the electrochemically active and the geometric electrode surface area of about 1,000. Advantageously, fractal coated electrodes 380 feature very low, nearly constant impedance in the range from 0.1 Hz to 200 Hz, which is the relevant range within which the important spectral components of cardiac signals are situated.

According to some embodiments, the sensing electrodes 380 are implemented as post-procedural sensors. Advantageously, post-procedural measurements from the post-procedural sensing electrodes 380 may assist a clinician to determine the type of anti-inflammatory therapy. Moreover, it is possible to follow up and obtain electrical activity readings to determine proper therapy duration, as well as detect further deterioration in signal activity.

According to some embodiments, the sensing electrodes 380 are similar in structure and function to pacemaker electrodes, and may be simultaneously utilized for providing pacemaker signals when required, thereby acting as both sensing electrodes and signal delivering electrodes. Similarly, the sensing and signal delivering electrodes 380 can be utilized to deliver other types of signals when needed, such as defibrillation signals, cardiac contractility modulation and the like.

Alternatively, in case the electrodes 380 cannot be used for delivering pacing signals, the signals sensed by electrodes 380 may provide useful information assisting in deciding whether a pacemaker/ICD/CCM device should be implanted, and/or whether anti-arrhythmic drug therapy should be administered.

Although shown in Figs. 33-36 in relation to a mechanically expandable valve 140', the one or more sensors 380 can be similarly coupled to any other type of a prosthetic valve 140 configured for implantation at any location of the heart, such as within the aortic annulus 42, within the mitral annulus 32, within the annulus of the native pulmonary valve, and/or within the annulus of the native tricuspid valve.

According to some embodiments, a plurality of sensors 380, comprising more than one type of sensor from the sensor types described herein above, are attached to a prosthetic valve 140.

According to some embodiments, any of the first sensor 180a, 280a and/or the second sensor 180b, 280b, attached to a component of the delivery apparatus 102, as well as any sensor 380 attached to the prosthetic valve 140, may transmit the measured signals to a control unit, which can be either the internal control unit 1110 connected to or housed within a component of the delivery apparatus 102, such as the handle 110, or an external control unit (not shown) provided separately from the delivery assembly 100.

The control unit can be operatively coupled to a communication component, such as the proximal communication component 1130 operatively coupled to the internal control unit 1110. The communication component can include a receiver, operable to receive measurement signals from any of the first sensor 180a, 280a and/or the second sensor 180b, 280b, attached to a component of the delivery apparatus 102, as well as any sensor 380 attached to the prosthetic valve 140.

According to some embodiments, the external control unit is operatively connected to any of the post-procedural sensors 380 via wireless communication. As mentioned above, the post-procedural sensor 380 can comprise, or be coupled to, a transmitter for remote communication, for example with the external control unit. According to some embodiments, the transmitter is a radiofrequency transmitter. In one variant of the embodiment, every post-procedural sensor 380 comprises a transmitter. In another variant of the embodiment, a plurality of post-procedural sensors 380 are coupled to a single transmitter.

According to some embodiments, the post-procedural sensor 380 comprises an internal control circuitry (not shown), electrically connected to or embedded within the post-procedural sensor 380 or the prosthetic valve 140. In one variant of the embodiment, every post-procedural sensor 380 comprises an internal control circuitry. In another variant of the embodiment, a plurality of post-procedural sensors 380 are connected to a single internal control circuitry.

According to some embodiments, the post-procedural sensor 380 comprises, or is coupled to, an internal memory (not shown), electrically connected to, or embedded within, the post-procedural sensor 380 or the prosthetic valve 140. In one variant of the embodiment, every post-procedural sensor 380 comprises an internal memory. In another variant of the embodiment, a plurality of post-procedural sensors 380 are connected to a single internal memory.

According to some embodiments, the post-procedural sensor 380 may be powered remotely. According to some embodiments, the post-procedural sensor 380 comprises an induction capacitor circuit or any other energy harvesting circuitry (not shown), which may be powered using radiofrequency (RF) by a transmitting/receiving antenna. In one variant of the embodiment, post-procedural sensor 380 comprises an energy harvesting circuitry. In another variant of the embodiment, a plurality of post-procedural sensors 380 are connected to a single energy harvesting circuitry.

According to some embodiments, the post-procedural sensor 380 may be coupled to an RFID reader unit (not shown), configured to allow power to be provided and/or information to be read from, and/or transmitted to, the post-procedural sensor 380. In one variant of the embodiment, every post-procedural sensor 380 comprises an RFID reader unit. In another variant of the embodiment, a plurality of post-procedural sensors 380 are connected to a single RFID reader unit.

The energy harvesting circuitry may be structured to receive RF energy from the RFID reader unit and harvest energy therefrom by converting the RF energy into DC energy, e.g., a DC voltage. The DC energy may be used to power the post-procedural sensors 380 and any other energy consuming components attached to the post-procedural sensors 380, such as the internal control circuitry, the internal memory member, and/or the transmitter.

Alternatively, or additionally, a prosthetic valve 140 may be provided with a local power source, such as a battery, attached thereto (not shown), for powering the at least one sensor 380. In such embodiments, the battery may provide sufficient electric power to enable sensor operability during the implantation procedure, and may be depleted afterwards leaving both battery and sensors inoperably attached to the implanted valve 140, without requiring incorporation of additional complex detachment mechanisms of the sensors 280 from the valve 140 once the implantation procedure is completed. Alternatively, the battery may provide sufficient electric power to enable sensor operability during a limited post-procedural time period.

According to some embodiments, the control unit, such as the internal control unit 1110, comprises a processor for processing and interpreting measurement data received from any of the first sensor 180a, 280a and/or the second sensor 180b, 280b, attached to a component of the delivery apparatus 102, as well as any sensor 380 attached to the prosthetic valve 140. The control unit may include software for interpreting and/or displaying data. A wide variety of algorithms can be used to provide warnings, for example to the clinician, associated with sensed signals interpretations. In addition, the control unit may provide for multiple measurements to be averaged over several cycles, and/or may provide for cycle-to-cycle variations to be visualized. Thus, an operator of the delivery assembly 100 according to any of the embodiments of the current disclosure, can quickly and easily obtain real-time measurements that may be displayed in the form of transvalvular pressure gradients, flow patterns across different regions around the prosthetic valve 140, and any other parameters measured by the sensors of the current disclosure.

According to some embodiments, the control unit, further comprises a memory member (not shown), such as an internal memory within the internal control unit 1110, configured to store the signals received from any of the first sensor 180a, 280a and/or the second sensor 180b, 280b, attached to a component of the delivery apparatus 102, as well as any sensor 380 attached to the prosthetic valve 140, and/or store interpreted data by the processor. A memory member may include a suitable memory chip or storage medium such as, for example, a PROM, EPROM, EEPROM, ROM, flash memory, solid state memory, or the like. A memory member can be integral with the control unit or may be removably coupled to the control unit.

According to some embodiments, measurement signals may be stored in a memory member and compared to historical values, in order to detect improvement or deterioration of the measured parameters.

According to some embodiments, the measurement signals may be mathematically manipulated or processed by the control unit on the measurement signals, in order to derive known relationships and indices that may be of clinical relevance or may be indicative of relevant clinical outcomes.

According to some embodiments, the internal control unit 1110 is configured to transmit, for example via the proximal communication component 1130, raw or interpreted data, including stored data, to an external control unit or any other external device, via either wired or wireless communication protocols.

Advantageously, measurement of physiological parameters (e.g., pressure gradients, blood flow, temperature indicative of inflammation, visual deposit detecting, and/or native electric activity), acquired by sensors according to any of the embodiments of the current disclosure, may provide real-time accurate quantitative data related to functional performance of prosthetic valves 140, during and/or after the implantation procedure.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. No feature described in the context of an embodiment is to be considered an essential feature of that embodiment, unless explicitly specified as such.

Although the invention is described in conjunction with specific embodiments thereof, it is evident that numerous alternatives, modifications and variations that are apparent to those skilled in the art may exist. It is to be understood that the invention is not necessarily limited in its application to the details of construction and the arrangement of the components and/or methods set forth herein. Other embodiments may be practiced, and an embodiment may be carried out in various ways. Accordingly, the invention embraces all such alternatives, modifications and variations that fall within the scope of the appended claims.

## Claims

1. A delivery assembly (100), comprising:
a prosthetic valve (140) movable between a radially compressed configuration and a radially expanded configuration;
a delivery apparatus (102) comprising:
a handle (110);
a delivery shaft (106) extending distally from the handle (110);
a nosecone shaft (118; 218; 418; 518; 1118) extending through the delivery shaft (106), and comprising:
a nosecone shaft outer surface (125; 1125);
a nosecone shaft guidewire lumen (122; 222); and
a nosecone shaft distal portion (120; 220; 420; 520; 620; 720);
a nosecone (126; 226; 326; 426; 526; 626; 1126; 1326) attached to the nosecone shaft distal portion (120; 220; 420; 520; 620; 720), and comprising a nosecone guidewire lumen (134; 234; 434; 534; 634; 1334) and a nosecone outer surface (127; 227; 327; 427; 527); wherein the nosecone (126; 226; 326; 426; 526; 626; 1126; 1326) further comprises a nosecone lateral port (236; 436; 536; 636; 1236) terminating at a nosecone port opening (237; 537; 637),;
a first sensor (180a, 380a) retained within the nosecone (126; 226; 326; 426; 526; 626; 1126; 1326); wherein the first sensor (180a; 380a) is positioned within the nosecone lateral port (236; 436; 536; 636; 1236) in alignment with the nosecone port opening (237; 537; 637); and
a first transmission line (168a; 368) coupled to the first sensor (180a; 380a), and extending proximally therefrom, toward the handle (110).

2. The delivery assembly (100) according to claim 1, wherein the first sensor (180a, 380a) is a first pressure sensor (180a, 380a); in particular wherein the first transmission line (168a; 368) is a first optic fiber, and wherein the first pressure sensor (180a, 380a) is a first optic pressure sensor (180a, 380a).

3. The delivery assembly (100) according to claim 1, wherein the nosecone port opening (237; 537; 637) is formed at the nosecone outer surface (127; 227; 327; 427; 527); or
wherein the nosecone port opening (237; 537; 637) is formed between the nosecone lateral port (236; 436; 536; 636; 1236) and the nosecone guidewire lumen (134; 234; 434; 534; 634; 1334).

4. The delivery assembly (100) according to claim 2 or 3, wherein the first pressure sensor (180a, 380a) is attached to the nosecone shaft outer surface (125; 1125).

5. The delivery assembly (100) according to claim 2 or 3, wherein the nosecone shaft (118; 218; 418; 518; 1118) further comprises a nosecone shaft sensor lumen and a nosecone shaft side opening, wherein the first pressure sensor (180a, 380a) is positioned in alignment with the nosecone shaft side opening, and wherein the first transmission line (168a; 368) extends through the nosecone shaft sensor lumen.

6. The delivery assembly (100) according to claim 2 or 3, wherein the delivery apparatus (102) further comprises a first sensor shaft extending through the delivery shaft (106), and comprising
a first sensor shaft lumen;
a first sensor shaft distal portion; and
a first sensor shaft side opening at the first sensor shaft distal portion; wherein the nosecone (126; 226; 326; 426; 526; 626; 1126; 1326) is attached to the first sensor shaft distal portion;
wherein the first pressure sensor (180a, 380a) is positioned in alignment with the first sensor shaft side opening; and
wherein the first transmission line (168a; 368) extends through the first sensor shaft lumen.

7. The delivery assembly (100) according to any one of claims 2 to 4, wherein the delivery apparatus (102) further comprises a second pressure sensor (180b; 380b) positioned proximal to the prosthetic valve (140), and a second transmission line (168b; 368b) coupled to the second pressure sensor (180b; 380b) and extending proximally therefrom, toward the handle (110); in particular
wherein the second transmission line (168b; 368b) is a second optic fiber, and wherein the second pressure sensor (180b; 380b) is a second optic pressure sensor.

8. The delivery assembly (100) according to claim 7, wherein the second pressure sensor (180b; 380b) is attached to the nosecone shaft outer surface (125; 1125); or
wherein the nosecone shaft (118; 218; 418; 518; 1118) further comprises a first nosecone shaft sensor lumen having a first nosecone shaft side opening, and a second nosecone shaft sensor lumen having a second nosecone shaft side opening, wherein the first pressure sensor (180a, 380a) is positioned in alignment with the first nosecone shaft side opening, and wherein the second pressure sensor (180b; 380b) is positioned in alignment with the second nosecone shaft side opening.

9. The delivery assembly (100) according to claim 7, wherein the nosecone shaft (118; 218; 418; 518; 1118) further comprises a nosecone shaft sensor lumen comprising a first nosecone shaft side opening and a second nosecone shaft side opening, wherein the first pressure sensor (180a, 380a) is positioned in alignment with the first nosecone shaft side opening, wherein the second pressure sensor (180b; 380b) is positioned in alignment with the second nosecone shaft side opening, and wherein both the first transmission line (168a; 368) and the second transmission line (168b; 368b) extend through the nosecone shaft sensor lumen; or
wherein the delivery apparatus (102) further comprises a plurality of actuator arm assemblies, extending through the delivery shaft (106) and releasably coupled to the prosthetic valve (140), and wherein the second pressure sensor (180b; 380b) is attached to at least one actuation assembly.

10. The delivery assembly (100) according to claim 4, wherein the delivery apparatus (102) further comprises a re-compression mechanism configured to compress a mechanically expandable prosthetic valve (140), the re-compression mechanism comprising:
a re-compression shaft extending through the delivery shaft (106), and comprising a re-compression shaft main lumen; and
a re-compression member extending through the re-compression shaft main lumen, and comprising a distal loop;
wherein the second pressure sensor (180b; 380b) is attached to the recompression shaft; in particular wherein
the second pressure sensor (180b; 380b) is attached to an outer surface of the re-compression shaft outer surface; or
the re-compression shaft further comprises a re-compression shaft sensor lumen and a re-compression shaft side opening, wherein the second pressure sensor (180b; 380b) is positioned in alignment with the re-compression shaft side opening, and wherein the second transmission line (168b; 368b) extends through the re-compression shaft sensor lumen.

11. The delivery assembly (100) according to claim 4, wherein the second pressure sensor (180b; 380b) is attached to the delivery shaft (106); in particular wherein
the second pressure sensor (180b; 380b) is attached to an outer surface of the delivery shaft (106); or
the delivery shaft (106) further comprises a delivery shaft sensor lumen and a delivery shaft side opening, wherein the second pressure sensor (180b; 380b) is positioned in alignment with the delivery shaft side opening, and wherein the second transmission line (168b; 368b) extends through the delivery shaft sensor lumen.

12. The delivery assembly (100) according to any one of claims 2 to 6, wherein the delivery apparatus (102) further comprises a sensing catheter comprising a sensing head, wherein the sensing catheter is axially movable relative to the delivery shaft (106), and wherein the sensing head comprises a second pressure sensor (180b; 380b); or
wherein the handle (110) further comprises an internal control unit connected to the first transmission line (168a; 368), and configured to receive signals from, and/or transmit signals to, the first pressure sensor (180a, 380a), via the first transmission line (168a; 368); in particular wherein the handle (110) further comprises a proximal communication component operatively coupled to the internal control unit, and configured to receive signals from, and/or transmit signals to, components and/or devices external to the delivery assembly (100).

13. The delivery assembly (100) according to claim 12, wherein the handle (110) further comprises a display operatively coupled to the internal control unit; in particular wherein the display comprises a digital screen or LED lights.

14. The delivery assembly (100) according to any one of claims 7 to 13, wherein the handle (110) further comprises an internal control unit connected to the first transmission line (168a; 368) and to the second transmission line (168b; 368b), and configured to receive signals from, and/or transmit signals to, the first pressure sensor (180a, 380a) via the first transmission line (168a; 368), and the second pressure sensor (180b; 380b) via the second transmission line (168b; 368b); in particular wherein the handle (110) further comprises a proximal communication component operatively coupled to the internal control unit, and configured to receive signals from, and/or transmit signals to, components and/or devices external to the delivery assembly (100).

15. A system comprising the delivery assembly (100) according to any one of claims 2 to 6, and a sensing catheter comprising a sensing head, wherein the sensing head comprises a second pressure sensor (180b; 380b); in particular wherein the sensing catheter is a pigtail catheter.

## Patentansprüche

1. Zuführungsanordnung (100), umfassend:
eine Klappenprothese (140), die zwischen einer radial komprimierten Konfiguration und einer radial expandierten Konfiguration beweglich ist;
eine Zuführungsvorrichtung (102), umfassend:
einen Griff (110);
einen Zuführungsschaft (106), der sich distal vom Griff (110) erstreckt;
einen Nasenkonusschaft (118; 218; 418; 518; 1118), der sich durch den Zuführungsschaft (106) erstreckt und umfasst:
eine Nasenkonusschaft-Außenfläche (125; 1125);
ein Nasenkonusschaft-Führungsdrahtlumen (122; 222); und
einen Nasenkonusschaft-Distalabschnitt (120; 220; 420; 520; 620; 720);
einen Nasenkonus (126; 226; 326; 426; 526; 626; 1126; 1326), der an dem Nasenkonusschaft-Distalabschnitt (120; 220; 420; 520; 620; 720) angebracht ist und ein Nasenkonus-Führungsdrahtlumen (134; 234; 434; 534; 634; 1334) und eine Nasenkonus-Außenfläche (127; 227; 327; 427; 527) umfasst; wobei der Nasenkonus (126; 226; 326; 426; 526; 626; 1126; 1326) ferner einen lateralen Nasenkonusanschluss (236; 436; 536; 636; 1236) umfasst, der an einer Nasenkonus-Anschlussöffnung (237; 537; 637) endet;
einen ersten Sensor (180a; 380a), der in dem Nasenkonus (126; 226; 326; 426; 526; 626; 1126; 1326) gehalten wird, wobei der erste Sensor (180a; 380a) innerhalb des lateralen Nasenkonusanschlusses (236; 436; 536; 636; 1236) in Ausrichtung auf die Nasenkonus-Anschlussöffnung (237; 537; 637) angeordnet ist; und
eine erste Übertragungsleitung (168a; 368), die mit dem ersten Sensor (180a; 380a) gekoppelt ist und sich von diesem proximal in Richtung zu dem Griff (110) erstreckt.

2. Zuführungsanordnung (100) nach Anspruch 1, wobei der erste Sensor (180a, 380a) ein erster Drucksensor (180a, 380a) ist; insbesondere wobei die erste Übertragungsleitung (168a; 368) eine erste optische Faser ist, und wobei der erste Drucksensor (180a, 380a) ein erster optischer Drucksensor (180a, 380a) ist.

3. Zuführungsanordnung (100) nach Anspruch 1, wobei die Nasenkonus-Anschlussöffnung (237; 537; 637) an der Nasenkonus-Außenfläche (127; 227; 327; 427; 527) ausgebildet ist; oder
wobei die Nasenkonus-Anschlussöffnung (237; 537; 637) zwischen dem lateralen Nasenkonusanschluss (236; 436; 536; 636; 1236) und dem Nasenkonus-Führungsdrahtlumen (134; 234; 434; 534; 634; 1334) ausgebildet ist.

4. Zuführungsanordnung (100) nach Anspruch 2 oder 3, wobei der erste Drucksensor (180a, 380a) an der Nasenkonusschaft-Außenfläche (125; 1125) angebracht ist.

5. Zuführungsanordnung (100) nach Anspruch 2 oder 3, wobei der Nasenkonusschaft (118; 218; 418; 518; 1118) ferner ein Nasenkonusschaft-Sensorlumen und eine Nasenkonusschaft-Seitenöffnung umfasst, wobei der erste Drucksensor (180a, 380a) in Ausrichtung auf die Nasenkonusschaft-Seitenöffnung positioniert ist, und wobei sich die erste Übertragungsleitung (168a; 368) durch das Nasenkonusschaft-Sensorlumen erstreckt.

6. Zuführungsanordnung (100) nach Anspruch 2 oder 3, wobei die Zuführungsvorrichtung (102) ferner einen ersten Sensorschaft umfasst, der sich durch den Zuführungsschaft (106) erstreckt, und umfassend:
ein erstes Sensorschaft-Lumen;
einen ersten Sensorschaft-Distalabschnitt; und
eine erste Sensorschaft-Seitenöffnung an dem ersten Sensorschaft-Distalabschnitt;
wobei der Nasenkonus (126; 226; 326; 426; 526; 626; 1126; 1326) an dem ersten Sensorschaft-Distalabschnitt angebracht ist;
wobei der erste Drucksensor (180a, 380a) in Ausrichtung auf die erste Sensorschaft-Seitenöffnung positioniert ist; und
wobei sich die erste Übertragungsleitung (168a; 368) durch das erste Sensorschaft-Lumen erstreckt.

7. Zuführungsanordnung (100) nach einem der Ansprüche 2 bis 4, wobei die Zuführungsvorrichtung (102) ferner einen zweiten Drucksensor (180b; 380b), der proximal zur Klappenprothese (140) angeordnet ist, und eine zweite Übertragungsleitung (168b; 368b) umfasst, die mit dem zweiten Drucksensor (180b; 380b) gekoppelt ist und sich von diesem proximal in Richtung zu dem Griff (110) erstreckt; insbesondere
wobei die zweite Übertragungsleitung (168b; 368b) eine zweite optische Faser ist, und wobei der zweite Drucksensor (180b; 380b) ein zweiter optischer Drucksensor ist.

8. Zuführungsanordnung (100) nach Anspruch 7, wobei der zweite Drucksensor (180b; 380b) an der Nasenkonusschaft-Außenfläche (125; 1125) angebracht ist; oder
wobei der Nasenkonusschaft (118; 218; 418; 518; 1118) ferner ein erstes Nasenkonusschaft-Sensorlumen mit einer ersten Nasenkonusschaft-Seitenöffnung und ein zweites Nasenkonusschaft-Sensorlumen mit einer zweiten Nasenkonusschaft-Seitenöffnung aufweist, wobei der erste Drucksensor (180a, 380a) in Ausrichtung auf die erste Nasenkonusschaft-Seitenöffnung angeordnet ist, und wobei der zweite Drucksensor (180b; 380b) in Ausrichtung auf die zweite Nasenkonusschaft-Seitenöffnung angeordnet ist.

9. Zuführungsanordnung (100) nach Anspruch 7, wobei der Nasenkonusschaft (118; 218; 418; 518; 1118) ferner ein Nasenkonusschaft-Sensorlumen umfasst, das eine erste Nasenkonusschaft-Seitenöffnung und eine zweite Nasenkonusschaft-Seitenöffnung umfasst, wobei der erste Drucksensor (180a, 380a) in Ausrichtung mit der ersten Nasenkonusschaft-Seitenöffnung angeordnet ist, wobei der zweite Drucksensor (180b; 380b) in Ausrichtung mit der zweiten Nasenkonusschaft-Seitenöffnung positioniert ist, und wobei sich sowohl die erste Übertragungsleitung (168a; 368) als auch die zweite Übertragungsleitung (168b; 368b) durch das Nasenkonusschaft-Sensorlumen erstrecken; oder
wobei die Zuführungsvorrichtung (102) ferner mehrere Betätigungsarm-Anordnungen umfasst, die sich durch den Zuführungsschaft (106) erstrecken und lösbar mit der Klappenprothese (140) gekoppelt sind, und wobei der zweite Drucksensor (180b; 380b) an wenigstens einer Betätigungsanordnung angebracht ist.

10. Zuführungsanordnung (100) nach Anspruch 4, wobei die Zuführungsvorrichtung (102) ferner eine Rekompressionseinrichtung umfasst, die dazu konfiguriert ist, eine mechanisch expandierbare Klappenprothese (140) zu komprimieren, wobei die Rekompressionseinrichtung umfasst:
einen Rekompressionsschaft, der sich durch den Zuführungsschaft (106) erstreckt und ein Rekompressionsschaft-Hauptlumen umfasst; und
ein Rekompressionselement, das sich durch das Rekompressionsschaft-Hauptlumen erstreckt und eine distale Schleife umfasst;
wobei der zweite Drucksensor (180b; 380b) an dem Rekompressionsschaft angebracht ist; insbesondere wobei
der zweite Drucksensor (180b; 380b) an einer Außenfläche des Rekompressionsschafts angebracht ist; oder
der Rekompressionsschaft ferner ein Rekompressionsschaft-Sensorlumen und eine Rekompressionsschaft-Seitenöffnung umfasst, wobei der zweite Drucksensor (180b; 380b) in Ausrichtung auf die Rekompressionsschaft-Seitenöffnung angeordnet ist, und wobei sich die zweite Übertragungsleitung (168b; 368b) durch das Rekompressionsschaft-Sensorlumen erstreckt.

11. Zuführungsanordnung (100) nach Anspruch 4, wobei der zweite Drucksensor (180b; 380b) an dem Zuführungsschaft (106) angebracht ist; insbesondere wobei
der zweite Drucksensor (180b; 380b) an einer Außenfläche des Zuführungsschafts (106) angebracht ist; oder
der Zuführungsschaft (106) ferner ein Zuführungsschaft-Sensorlumen und eine Zuführungsschaft-Seitenöffnung umfasst, wobei der zweite Drucksensor (180b; 380b) in Ausrichtung auf die Zuführungsschaft-Seitenöffnung angeordnet ist, und wobei sich die zweite Übertragungsleitung (168b; 368b) durch das Zuführungsschaft-Sensorlumen erstreckt.

12. Zuführungsanordnung (100) nach einem der Ansprüche 2 bis 6, wobei die Zuführungsvorrichtung (102) ferner einen Sensorkatheter umfasst, der einen Sensorkopf umfasst, wobei der Sensorkatheter relativ zu dem Zuführungsschaft (106) axial beweglich ist, und wobei der Sensorkopf einen zweiten Drucksensor (180b; 380b) umfasst; oder
wobei der Griff (110) ferner eine interne Steuereinheit umfasst, die mit der ersten Übertragungsleitung (168a; 368) verbunden ist und dazu konfiguriert ist, über die erste Übertragungsleitung (168a; 368) Signale von dem ersten Drucksensor (180a, 380a) zu empfangen und/oder Signale an diesen zu senden; insbesondere wobei der Griff (110) ferner eine proximale Kommunikationskomponente umfasst, die operativ mit der internen Steuereinheit gekoppelt ist und dazu konfiguriert ist, Signale von Komponenten und/oder Vorrichtungen außerhalb der Zuführungsanordnung (100) zu empfangen und/oder Signale an diese zu senden.

13. Zuführungsanordnung (100) nach Anspruch 12, wobei der Griff (110) ferner eine operativ mit der internen Steuereinheit gekoppelte Anzeigeeinrichtung umfasst, insbesondere wobei die Anzeigeeinrichtung einen digitalen Bildschirm oder LED-Leuchten umfasst.

14. Zuführungsanordnung (100) nach einem der Ansprüche 7 bis 13, wobei der Griff (110) ferner eine interne Steuereinheit umfasst, die mit der ersten Übertragungsleitung (168a; 368) und mit der zweiten Übertragungsleitung (168b; 368b) verbunden ist und dazu konfiguriert ist, über die erste Übertragungsleitung (168a; 368b) Signale von dem ersten Drucksensor (180a, 380a) zu empfangen und/oder Signale an diesen zu senden und über die zweite Übertragungsleitung (168b; 368b) Signale von dem zweiten Drucksensor (180b; 380b) zu empfangen und/oder Signale an diesen zu senden; insbesondere wobei der Griff (110) ferner eine proximale Kommunikationskomponente umfasst, die operativ mit der internen Steuereinheit gekoppelt ist und dazu konfiguriert ist, Signale von Komponenten und/oder Vorrichtungen außerhalb der Zuführungsanordnung (100) zu empfangen und/oder Signale an diese zu senden.

15. System, das die Zuführungsanordnung (100) nach einem der Ansprüche 2 bis 6 und einen Sensorkatheter umfasst, der einen Sensorkopf umfasst, wobei der Sensorkopf einen zweiten Drucksensor (180b; 380b) umfasst; insbesondere wobei der Sensorkatheter ein Pigtail-Katheter ist.

## Revendications

1. Ensemble de pose (100), comprenant :
une valvule prothétique (140) mobile entre une configuration radialement comprimée et une configuration radialement déployée ;
un appareil de pose (102) comprenant :
une poignée (110) ;
une tige de pose (106) s'étendant distalement à partir de la poignée (110) ;
une tige (118 ; 218 ; 418 ; 518 ; 1118) de pointe avant s'étendant à travers la tige de pose (106) et comprenant :
une surface externe (125 ; 1125) de tige de pointe avant ;
une lumière (122 ; 222) à fil-guide de tige de pointe avant ; et
une partie distale (120 ; 220 ; 420 ; 520 ; 620 ; 720) de tige de pointe avant ;
une pointe avant (126 ; 226 ; 326 ; 426 ; 526 ; 626 ; 1126 ; 1326) fixée à la partie distale (120 ; 220 ; 420 ; 520 ; 620 ; 720) de tige de pointe avant et comprenant une lumière (134 ; 234 ; 434 ; 534 ; 634 ; 1334) à fil-guide de pointe avant et une surface externe (127 ; 227 ; 327 ; 427 ; 527) de pointe avant ; la pointe avant (126 ; 226 ; 326 ; 426 ; 526 ; 626 ; 1126 ; 1326) comprenant en outre un orifice latéral (236 ; 436 ; 536 ; 636 ; 1236) de pointe avant se terminant au niveau d'une ouverture (237 ; 537 ; 637) d'orifice de pointe avant ;
un premier capteur (180a, 380a) retenu à l'intérieur de la pointe avant (126 ; 226 ; 326 ; 426 ; 526 ; 626 ; 1126 ; 1326) ; le premier capteur (180a ; 380a) étant positionné à l'intérieur de l'orifice latéral (236 ; 436 ; 536 ; 636 ; 1236) de pointe avant en alignement avec l'ouverture (237 ; 537 ; 637) d'orifice de pointe avant ; et
une première ligne de transmission (168a ; 368) accouplée au premier capteur (180a ; 380a) et s'étendant proximalement à partir de celui-ci, vers la poignée (110).

2. Ensemble de pose (100) selon la revendication 1, le premier capteur (180a, 380a) étant un premier capteur de pression (180a, 380a) ; en particulier la première ligne de transmission (168a ; 368) étant une première fibre optique et le premier capteur de pression (180a, 380a) étant un premier capteur de pression (180a, 380a) optique.

3. Ensemble de pose (100) selon la revendication 1, l'ouverture (237 ; 537 ; 637) d'orifice de pointe avant étant formée au niveau de la surface externe (127 ; 227 ; 327 ; 427 ; 527) de pointe avant ; ou
l'ouverture (237 ; 537 ; 637) d'orifice de pointe avant étant formée entre l'orifice latéral (236 ; 436 ; 536 ; 636 ; 1236) de pointe avant et la lumière (134 ; 234 ; 434 ; 534 ; 634 ; 1334) à fil-guide de pointe avant.

4. Ensemble de pose (100) selon la revendication 2 ou 3, le premier capteur de pression (180a, 380a) étant fixé à la surface externe (125 ; 1125) de tige de pointe avant.

5. Ensemble de pose (100) selon la revendication 2 ou 3, la tige (118 ; 218 ; 418 ; 518 ; 1118) de pointe avant comprenant en outre une lumière à capteur de tige de pointe avant et une ouverture latérale de tige de pointe avant, le premier capteur de pression (180a, 380a) étant positionné en alignement avec l'ouverture latérale de tige de pointe avant et la première ligne de transmission (168a ; 368) s'étendant à travers la lumière à capteur de tige de pointe avant.

6. Ensemble de pose (100) selon la revendication 2 ou 3, l'appareil de pose (102) comprenant en outre une tige de premier capteur s'étendant à travers la tige de pose (106) et comprenant
une lumière de tige de premier capteur ;
une partie distale de tige de premier capteur ; et
une ouverture latérale de tige de premier capteur au niveau de la partie distale de tige de premier capteur ;
la pointe avant (126 ; 226 ; 326 ; 426 ; 526 ; 626 ; 1126 ; 1326) étant fixée à la partie distale de tige de premier capteur ;
le premier capteur de pression (180a, 380a) étant positionné en alignement avec l'ouverture latérale de tige de premier capteur ; et
la première ligne de transmission (168a ; 368) s'étendant à travers la lumière de tige de premier capteur.

7. Ensemble de pose (100) selon l'une quelconque des revendications 2 à 4, l'appareil de pose (102) comprenant en outre un second capteur de pression (180b ; 380b) positionné proximalement par rapport à la valvule prothétique (140) et une seconde ligne de transmission (168b ; 368b) accouplée au second capteur de pression (180b ; 380b) et s'étendant proximalement à partir de celui-ci, vers la poignée (110) ; en particulier
la seconde ligne de transmission (168b ; 368b) étant une seconde fibre optique et le second capteur de pression (180b ; 380b) étant un second capteur de pression optique.

8. Ensemble de pose (100) selon la revendication 7, le second capteur de pression (180b ; 380b) étant fixé à la surface externe (125 ; 1125) de tige de pointe avant ; ou
la tige (118 ; 218 ; 418 ; 518 ; 1118) de pointe avant comprenant en outre une lumière à premier capteur de tige de pointe avant présentant une première ouverture latérale de tige de pointe avant et une lumière à second capteur de tige de pointe avant présentant une seconde ouverture latérale de tige de pointe avant, le premier capteur de pression (180a, 380a) étant positionné en alignement avec la première ouverture latérale de tige de pointe avant et le second capteur de pression (180b ; 380b) étant positionné en alignement avec la seconde ouverture latérale de tige de pointe avant.

9. Ensemble de pose (100) selon la revendication 7, la tige (118 ; 218 ; 418 ; 518 ; 1118) de pointe avant comprenant en outre une lumière à capteur de tige de pointe avant comprenant une première ouverture latérale de tige de pointe avant et une seconde ouverture latérale de tige de pointe avant, le premier capteur de pression (180a, 380a) étant positionné en alignement avec la première ouverture latérale de tige de pointe avant, le second capteur de pression (180b ; 380b) étant positionné en alignement avec la seconde ouverture latérale de tige de pointe avant et à la fois la première ligne de transmission (168a ; 368) et la seconde ligne de transmission (168b ; 368b) s'étendant à travers la lumière à capteur de tige de pointe avant ; ou
l'appareil de pose (102) comprenant en outre une pluralité d'ensembles bras d'actionneur, s'étendant à travers la tige de pose (106) et accouplés de manière détachable à la valvule prothétique (140) et le second capteur de pression (180b ; 380b) étant fixé à au moins un ensemble d'actionnement.

10. Ensemble de pose (100) selon la revendication 4, l'appareil de pose (102) comprenant en outre un mécanisme de re-compression conçu pour comprimer une valvule prothétique (140) mécaniquement déployable, le mécanisme de re-compression comprenant :
une tige de re-compression s'étendant à travers la tige de pose (106) et comprenant une lumière principale de tige de re-compression ; et
un élément de re-compression s'étendant à travers la lumière principale de tige de re-compression et comprenant une boucle distale ;
le second capteur de pression (180b ; 380b) étant fixé à la tige de re-compression ; en particulier
le second capteur de pression (180b ; 380b) étant fixé à une surface externe de la surface externe de tige de re-compression ; ou
la tige de re-compression comprenant en outre une lumière à capteur de tige de re-compression et une ouverture latérale de tige de re-compression, le second capteur de pression (180b ; 380b) étant positionné en alignement avec l'ouverture latérale de tige de re-compression et la seconde ligne de transmission (168b ; 368b) s'étendant à travers la lumière à capteur de tige de re-compression.

11. Ensemble de pose (100) selon la revendication 4, le second capteur de pression (180b ; 380b) étant fixé à la tige de pose (106) ; en particulier
le second capteur de pression (180b ; 380b) étant fixé à une surface externe de la tige de pose (106) ; ou
la tige de pose (106) comprenant en outre une lumière à capteur de tige de pose et une ouverture latérale de tige de pose, le second capteur de pression (180b ; 380b) étant positionné en alignement avec l'ouverture latérale de tige de pose et la seconde ligne de transmission (168b ; 368b) s'étendant à travers la lumière à capteur de tige de pose.

12. Ensemble de pose (100) selon l'une quelconque des revendications 2 à 6, l'appareil de pose (102) comprenant en outre un cathéter de détection comprenant une tête de détection, le cathéter de détection étant mobile axialement par rapport à la tige de pose (106) et la tête de détection comprenant un second capteur de pression (180b ; 380b) ; ou
la poignée (110) comprenant en outre une unité de commande interne connectée à la première ligne de transmission (168a ; 368) et conçue pour recevoir des signaux provenant du premier capteur de pression (180a, 380a) et/ou transmettre des signaux à celui-ci, par l'intermédiaire de la première ligne de transmission (168a ; 368) ; en particulier la poignée (110) comprenant en outre un composant de communication proximal couplé fonctionnellement à l'unité de commande interne et conçu pour recevoir des signaux provenant de composants et/ou dispositifs externes à l'ensemble de pose (100) et/ou transmettre des signaux à ceux-ci.

13. Ensemble de pose (100) selon la revendication 12, la poignée (110) comprenant en outre un dispositif d'affichage couplé fonctionnellement à l'unité de commande interne ; en particulier le dispositif d'affichage comprenant un écran numérique ou des lumières à DEL.

14. Ensemble de pose (100) selon l'une quelconque des revendications 7 à 13, la poignée (110) comprenant en outre une unité de commande interne connectée à la première ligne de transmission (168a ; 368) et à la seconde ligne de transmission (168b ; 368b) et conçue pour recevoir des signaux provenant du premier capteur de pression (180a, 380a) et/ou transmettre des signaux à celui-ci par l'intermédiaire de la première ligne de transmission (168a ; 368) et pour recevoir des signaux provenant du second capteur de pression (180b ; 380b) et/ou transmettre des signaux à celui-ci par l'intermédiaire de la seconde ligne de transmission (168b ; 368b) ; en particulier la poignée (110) comprenant en outre un composant de communication proximal couplé fonctionnellement à l'unité de commande interne et conçu pour recevoir des signaux provenant de composants et/ou de dispositifs externes à l'ensemble de pose (100) et/ou transmettre des signaux à ceux-ci.

15. Système comprenant l'ensemble de pose (100) selon l'une quelconque des revendications 2 à 6 et un cathéter de détection comprenant une tête de détection, la tête de détection comprenant un second capteur de pression (180b ; 380b) ; en particulier le cathéter de détection étant un cathéter queue de cochon.
